# EUROPEAN PATENT SPECIFICATION

(11) **EP 4 288 033 B1**
(45) Date of publication and mention of the grant of the patent: **25.06.2025**
(21) Application number: 22705744.5
(22) Date of filing: 02.02.2022
(51) Int. Cl.: A61K 9/127, A61K 39/39, A61P 37/04, A61K 39/00, A61K 39/12, A61P 31/14, A61P 31/16

(54) **POLYMERSOME ASSOCIATED ADJUVANT FOR USE IN STIMULATING AN IMMUNE RESPONSE**
MIT POLYMERSOMEN ASSOZIERTE ADJUVANTIEN ZUR VERWENDUNG BEI DER STIMULIERUNG EINER IMMUNANTWORT
POLYMÉROSOME ASSOCIÉ À UN ADJUVANT POUR UTILISATION DANS LA STIMULATION D'UNE RÉPONSE IMMUNITAIRE

(30) Priority: 02.02.2021 EP 21154745
(43) Date of publication of application: 13.12.2023
(73) Proprietor: ACM Biolabs Pte Ltd, 638075 Singapore (SG)
(72) Inventor: NALLANI, Madhavan, 638075 Singapore (SG); LAM, Jian Hang, 638075 Singapore (SG); CORNELL, Thomas Andrew, 638075 Singapore (SG); KHAN, Amit Kumar, 638075 Singapore (SG); MARTIN, Liam Thomas, 638075 Singapore (SG)
(74) Representative: Schiweck Weinzierl Koch Patentanwälte Partnerschaft mbB
(86) International application number: PCT/EP2022/052508
(87) International publication number: WO 2022/167497

(56) References cited:
- WO-A2-2019/145475
- WO-A2-2021/019102
- US-A1- 2015 297 711
- JIAN HANG LAM ET AL: "Next generation vaccine platform: polymersome as stable nanocarriers for a highly immunogenic and durable SARS-CoV-2 spike protein subunit vaccine", INTERNET CITATION, 25 January 2021 (2021-01-25), XP002804320, Retrieved from the Internet <URL:https://www.biorxiv.org/content/10.1101/2021.01.24.427729v1.full> [retrieved on 20210928]

## Description

### Cross-reference to related applications

The present application claims the right of priority of European patent application 21154745 filed with the European Patent Office on 2 February 2021.

### Sequence Listing

This application contains a Sequence Listing.

### Technical Field

The present invention relates to eliciting an immune response in a subject by sole administration of an adjuvant, wherein the adjuvant is associated with one or more populations of polymersomes.

### Background of the Invention

Although immunization is a well-established process, there are differences in the response level elicited between different immunogens or antigens. For example, membrane proteins form a class of antigens that produce a low response level, which in turn means that large amounts of membrane proteins are required to generate or elicit an immune response to the desired level. Membrane proteins are notoriously difficult to synthesize and are insoluble in water without the presence of a detergent. This makes it expensive and difficult to obtain membrane proteins in sufficient quantity for immunization. Furthermore, membrane proteins require proper folding to function correctly. The immunogenicity of correctly folded native membrane proteins is typically much better than that of their solubilized forms, which may not be folded in a physiologically relevant manner. Thus, even though adjuvants may be used to boost the immunogenicity of such solubilized antigens, it is an inefficient method that does not provide too much of an advantage (e.g., WO2014/077781A1).

Although transfected cells and lipid-based systems have been used to present membrane protein antigens to increase the chances of isolating antibodies that may be efficient *in vivo,* these systems are often unstable (e.g., oxidation sensitive), tedious and costly. Moreover, the current state of the art for such membrane protein antigens is to use inactive virus-like particles for immunization.

On the other hand, vaccines are the most efficient way to prevent diseases, mainly infectious diseases [e.g., Liu et al., 2016]. As of today, most of the licensed vaccines are made of either live or killed viruses. Despite their effectiveness in generating a humoral response (an antibody mediated response) to prevent viral propagation and entry into cells, safety of such vaccines remains a concern. In the past few decades, scientific advances have helped to overcome such issues by engineering vaccine vectors that are non-replicating recombinant viruses. In parallel, protein based antigens or sub-unit antigens are explored as safer alternatives. However, such protein based vaccines typically illicit poor immune (both humoral and cellular response). To improve immunogenic properties of antigens, several approaches have been used. For example, microencapsulation of antigens into polymers have been investigated extensively, although it did enhance the immunogenicity, aggregation and denaturing of antigens remain unsolved [e.g., Hilbert et al., 1999]. Furthermore, adjuvants (e.g., oil in water emulsions or polymer emulsions) [e.g., US9636397B2, US2015/0044242 A1] are used together with antigens to elicit a more pronounced humoral and cellular response. Despite these advances, they are less efficient in uptake and cross-presentation. To promote cross-presentation, based on the available information of the immune system during infection by viruses, viral like particles that mimics such properties have been exploited. Synthetic architectures such as liposomes with encapsulated antigens are particularly attractive. Liposomes are unilamellar self-assembling structures made of lipids and, cationic liposomes are more attractive and promising as delivery vehicles because of their efficient uptake by Antigen Presenting Cells (APCs) [e.g., Maji et al., 2016]. Furthermore, it allows to integrate immunomodulators such as Monophosphoryl Lipid A (MPL), CpG oligodeoxynucleotide, that are toll-like receptor (TLR) agonists which stimulate immune cells through receptors. Despite these opportunities of such delivery vehicles, one of the limiting factors is stability of liposomes in the presence of serum components. By PEGylations, loading with high melting temperature lipids, stability issues of liposomes are somewhat reduced with and one such well characterized example being interbilayered-crosslinked multilamellar vesicles (ICMVs), formed by stabilizing multilamellar vesicles with short covalent crosslinks linking lipids [e.g., Moon et al., 2011]. Other nanoparticle architectures have led to successful immunisations using nanodiscs [e.g., Kuai et al., 2017] or pH sensitive particles [e.g., Luo et al., 2017]. But such strategies either still requires adjuvants or are not as efficient outside the prototypical Ovalbumin (OVA) models.

In addition, polymersomes, offer as a stable alternative for liposomes and they have been used to integrate membrane proteins to elicit immune response [e.g., Quer et al., 2011, WO2014/077781A1]. Protein antigens were also encapsulated in a chemically altered membrane of the polymersome (however oxidation-sensitive membranes) to release antigens and the adjuvants to dendritic cells [e.g., Stano et al., 2013].

Despite this progress made by the use of polymers, there remains a need to provide alternative methods of eliciting an immune response (e.g., in the absence of antigen), in particular for treatment and/or prevention of infectious diseases, cancers and autoimmune diseases.

### Summary of the Invention

The present invention relates to an adjuvant associated with one or more populations of polymersomes for use in a method of eliciting an immune response in a subject by sole administration of said adjuvant, wherein said sole administration is an administration characterized in that no antigen is administered to said subject in combination (e.g., co-administered) with said adjuvant (e.g., either simultaneously or at a different time). The present invention further relates to an adjuvant for use in eliciting an immune response in a subject, wherein the use comprising sole administration of said adjuvant, wherein said adjuvant is associated with one or more populations of polymersomes.

Therefore, the present application satisfies the demand by provision of two separate populations of polymersomes that, when administered, improve the immunogenic properties of antigens, methods for production of such two populations of polymersomes and compositions comprising such two populations of polymersomes, described herein below, characterized in the claims and illustrated by the appended Examples and Figures.

### Overview of the Sequence Listing

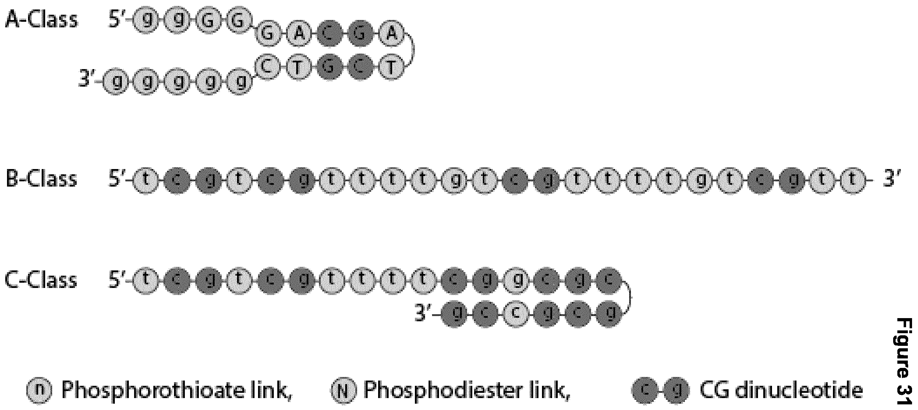

As described herein references are made to UniProtKB Accession Numbers (http://www.uniprot.org/ e.g., as available in UniProtKB Release 2017_12, unless indicated otherwise or otherwise inherent).
SEQ ID NO: 1 is the amino acid sequence of the tumor neoantigen polypeptide Reps1 P45A derived from the colon cancer MC-38 mouse model.
SEQ ID NO: 2 is the amino acid sequence of the tumor neoantigen peptide Adpgk R304M derived from the colon cancer MC-38 mouse model.
SEQ ID NO: 3 is the amino acid sequence of the tumor neoantigen peptide Dpagt1 V213L derived from the colon cancer MC-38 mouse model.
SEQ ID NO: 4 is the amino acid sequence of the chicken Ovalbumin (OVA), UniProtKB Accession Number: P01012.
SEQ ID NO: 5 is the amino acid sequence of the influenza A virus (A/New York/38/2016(H1N1)) hemagglutinin, UniProtKB Accession Number: A0A192ZYK0.
SEQ ID NO: 6 is the amino acid sequence of the influenza A virus (A/swine/4/Mexico/2009(H1N1)) hemagglutinin, UniProtKB Accession Number: D2CE65.
SEQ ID NO: 7 is the amino acid sequence of the influenza A virus (A/Puerto rico/8/1934(H1N1)) hemagglutinin.
SEQ ID NO: 8 is the amino acid sequence of the influenza A virus (A/California/07/2009(H1N1)) hemagglutinin.
SEQ ID NO: 9 is the amino acid sequence of the tumor neoantigen polypeptide CD8 Trp2 173-196 derived from the melanoma B16-F10 mouse model.
SEQ ID NO: 10 is the amino acid sequence of the tumor neoantigen polypeptide CD4 M30 Kif18b K739N derived from the melanoma B16-F10 mouse model.
SEQ ID NO: 11 is the amino acid sequence of the tumor neoantigen polypeptide CD4 M44 Cpsf3I D314N derived from the melanoma B16-F10 mouse model.
SEQ ID NO: 12 is the amino acid sequence of the soluble portion (amino acid residues 19 to 1327) of the Porcine Epidemic Diarrhea virus (PEDv) Spike protein (S Protein) (UniProtKB Accession number: V5TA78)
SEQ ID NO: 13 is the amino acid sequence of the S1 region (amino acid residues 19 to 739) of the PEDv Spike protein (S Protein) and
SEQ ID NO: 14 is the amino acid sequence of the S2 region (amino acid residues 739 to 1327) of the PEDv Spike protein (S Protein)
SEQ ID NO: 15 is the amino sequence of the enhanced Green Fluorescent Protein (eGFP).
SEQ ID NO: 16: is the sequence of a CD8 T cell peptide epitope (SIINFEKL).
SEQ ID NO: 17 is the sequence of a CD8 T cell peptide epitope (SVYDFFVWL).
SEQ ID NO: 18: is the sequence of the class B CpG oligodeoxynucleotide CpG ODN1826 (5'-tccatgacgttcctgacgtt-3') that is available from InvivoGen.
SEQ ID NO: 19: is the amino acid sequence of the SARS-CoV-2 Spike protein according to UniProtKB accession no. P0DTC2.
SEQ ID NO: 20: is the amino acid sequence of the SARS-CoV-2 Spike protein according to GenBank accession no. QII57278.1.
SEQ ID NO: 21: is the amino acid sequence of the SARS-CoV-2 Spike protein according to GenBank accession no. YP_009724390.1.
SEQ ID NO: 22: is the amino acid sequence of the SARS-CoV-2 Spike protein according to GenBank accession no. QIO04367.1.
SEQ ID NO: 23: is the amino acid sequence of the SARS-CoV-2 Spike protein according to GenBank accession no. QHU79173.2.
SEQ ID NO: 24: is the amino acid sequence of the SARS-CoV-2 Spike protein according to GenBank accession no. QII87830.1.
SEQ ID NO: 25: is the amino acid sequence of the SARS-CoV-2 Spike protein according to GenBank accession no. QIA98583.1.
SEQ ID NO: 26: is the amino acid sequence of the SARS-CoV-2 Spike protein according to GenBank accession no. QIA20044.1.
SEQ ID NO: 27: is the amino acid sequence of the SARS-CoV-2 Spike protein according to GenBank accession no. QIK50427.1.
SEQ ID NO: 28: is the amino acid sequence of the SARS-CoV-2 Spike protein according to GenBank accession no. QHR84449.1.
SEQ ID NO: 29: is the amino acid sequence of the SARS-CoV-2 Spike protein according to GenBank accession no. QIQ08810.1.
SEQ ID NO: 30: is the amino acid sequence of the SARS-CoV-2 Spike protein according to GenBank accession no. QIJ96493.1.
SEQ ID NO: 31: is the amino acid sequence of the SARS-CoV-2 Spike protein according to GenBank accession no. QIC53204.1.
SEQ ID NO: 32: is the amino acid sequence of the SARS-CoV-2 Spike protein according to GenBank accession no. QHZ00379.1.
SEQ ID NO: 33: is the amino acid sequence of the SARS-CoV-2 Spike protein according to GenBank accession no. QHS34546.1.
SEQ ID NO: 34: is the amino acid sequence of a soluble fragment of the SARS-CoV-2 Spike protein corresponding to positions 16-1213 of UniProtKB accession no. P0DTC2.
SEQ ID NO: 35: is the amino acid sequence of a soluble fragment of the SARS-CoV-2 Spike protein corresponding to positions 14-1204 of UniProtKB accession no. P0DTC2.
SEQ ID NO: 36: is the amino acid sequence of a soluble fragment of the SARS-CoV-2 Spike protein.
SEQ ID NO: 37: is the amino acid sequence of a soluble fragment of the SARS-CoV-2 Spike protein corresponding to positions 16-685 of UniProtKB accession no. P0DTC2.
SEQ ID NO: 38: is the amino acid sequence of a soluble fragment of the SARS-CoV-2 Spike protein corresponding to positions 686-1213 of UniProtKB accession no. P0DTC2.
SEQ ID NO: 39: is the amino acid sequence of a soluble fragment of the SARS-CoV-2 Spike protein corresponding to positions 646-1204 of UniProtKB accession no. P0DTC2.
SEQ ID NO: 40: is the amino acid sequence of a soluble fragment of the SARS-CoV-2 Spike protein.
SEQ ID NO: 41: is the amino acid sequence of a soluble fragment of the SARS-CoV-2 Spike protein corresponding to positions 318-524 of UniProtKB accession no. P0DTC2.
SEQ ID NO: 42: is the amino acid sequence of the MERS-CoV Spike protein according to UniProtKB accession no. K0BRG7.
SEQ ID NO: 43: is the amino acid sequence of a soluble fragment of the MERS-CoV Spike protein corresponding to positions 1-1297 of UniProtKB accession no. K0BRG7.
SEQ ID NO: 44: is the amino acid sequence of a soluble fragment of the MERS-CoV Spike protein corresponding to positions 18-725 of UniProtKB accession no. K0BRG7.
SEQ ID NO: 45: is the amino acid sequence of a soluble fragment of the MERS-CoV Spike protein corresponding to positions 726-1296 of UniProtKB accession no. K0BRG7.
SEQ ID NO: 46: is the amino acid sequence of a soluble fragment of the MERS-CoV Spike protein corresponding to positions 377-588 of UniProtKB accession no. K0BRG7.
SEQ ID NO: 47: is the amino acid sequence of the SARS-CoV-1 Spike protein according to UniProtKB accession no. P59594.
SEQ ID NO: 48: is the amino acid sequence of a soluble fragment of the SARS-CoV-1 Spike protein corresponding to positions 14-1195 of UniProtKB accession no. P59594.
SEQ ID NO: 49: is the amino acid sequence of a soluble fragment of the SARS-CoV-1 Spike protein corresponding to positions 14-667 of UniProtKB accession no. P59594.
SEQ ID NO: 50: is the amino acid sequence of a soluble fragment of the SARS-CoV-1 Spike protein corresponding to positions 668-1195 of UniProtKB accession no. P59594.
SEQ ID NO: 51: is the amino acid sequence of a soluble fragment of the SARS-CoV-1 Spike protein corresponding to positions 306-527 of UniProtKB accession no. P59594.
SEQ ID NO: 52: is the amino acid sequence of a furin cleavage site of the SARS-CoV-2 Spike protein.
SEQ ID NO: 53: is the amino acid sequence of a mutated furin cleavage site of the SARS-CoV-2 Spike protein.
SEQ ID NO: 54: is the amino acid sequence of a foldon domain.
SEQ ID NO: 55: is the amino acid sequence of a GCN4 domain.
SEQ ID NO: 56: is the amino acid sequence of an immunosilenced GCN4 domain
SEQ ID NO: 57: is the amino acid sequence of a honey bee melittin leader sequence.
SEQ ID NO: 58: is the amino acid sequence of a furin cleavage site of the MERS-CoV Spike protein.
SEQ ID NO: 59: is the amino acid sequence of a mutated furin cleavage site of the MERS-CoV Spike protein.
SEQ ID NO: 60: is the amino acid sequence of a furin cleavage site of the SARS-CoV-1 Spike protein.
SEQ ID NO: 61: is the amino acid sequence of a mutated furin cleavage site of the SARS-CoV-1 Spike protein.
SEQ ID NO: 62 is the nucleotide sequence of the CpG oligonucleotide ODN 2006.
SEQ ID NO: 63 is the nucleotide sequence of the CpG oligonucleotide ODN 2007.
SEQ ID NO: 64 is the nucleotide sequence of the CpG oligonucleotide ODN 2216.
SEQ ID NO: 65 is the amino acid sequence of a soluble fragment of the SARS-CoV-2 Spike protein corresponding to positions 19-1204 of UniProtKB accession no. P0DTC2 but with a mutated furin cleavage site.
SEQ ID NO: 66 is the amino acid sequence of the SARS-CoV-2 Spike protein corresponding to positions 19-1273 of UniProtKB accession no. P0DTC2 but with a mutated furin cleavage site.
SEQ ID NO: 67 is the nucleotide sequence of the CpG oligonucleotide ODN 2135.
SEQ ID NO: 68 is the nucleotide sequence of an exemplary CpG oligonucleotide ODN.
SEQ ID NO: 69 is the nucleotide sequence of an exemplary CpG oligonucleotide ODN.
SEQ ID NO: 70 is the nucleotide sequence of an exemplary CpG oligonucleotide ODN.
SEQ ID NO: 71 is an exemplary nucleotide sequence of the CpG oligonucleotide ODN class A (derived from https://www.invivogen.com/cpg-odns-classes and depicted in Figure 31).
SEQ ID NO: 72 is an exemplary nucleotide sequence of the CpG oligonucleotide ODN class B (derived from https://www.invivogen.com/cpg-odns-classes and depicted in Figure 31).
SEQ ID NO: 73 is an exemplary nucleotide sequence of the CpG oligonucleotide ODN class C (derived from https://www.invivogen.com/cpg-odns-classes and depicted in Figure 31).
SEQ ID NO: 74 an exemplary nucleotide sequence of the CpG oligonucleotide ODN class B (ODN 2007).
SEQ ID NO: 75 an exemplary nucleotide sequence of the CpG oligonucleotide ODN class B (ODN 2006).
SEQ ID NO: 76 an exemplary nucleotide sequence of the CpG oligonucleotide ODN (ODN 2014).
SEQ ID NO: 77 an exemplary nucleotide sequence of the CpG oligonucleotide ODN (ODN 2135).

### Brief Description of the Drawings

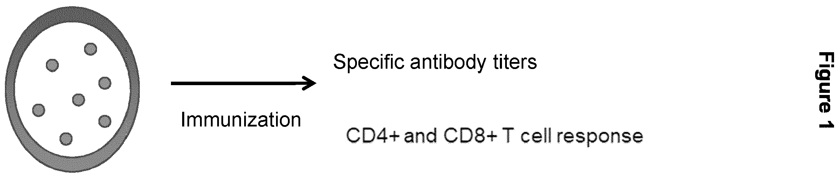

**Figure 1** shows a schematic view of the immunization with a polymersome of the present invention encapsulating antigens and measuring the humoral and cellular responses.

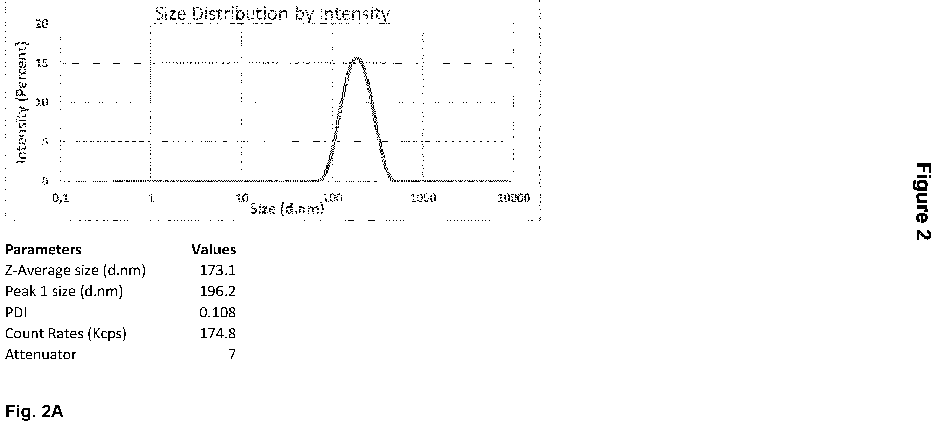

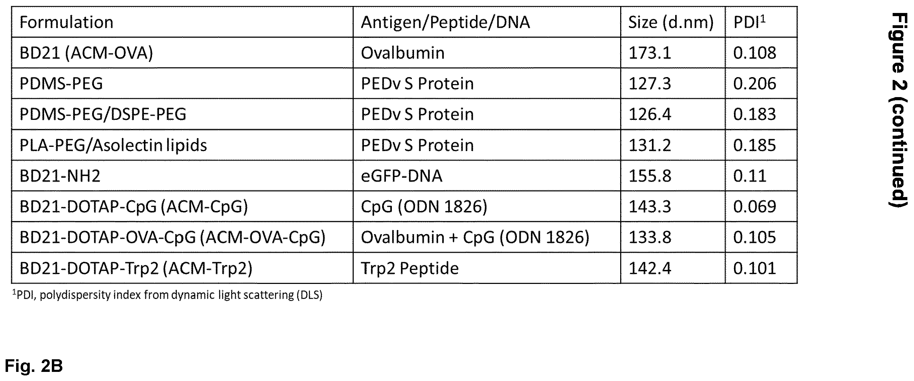

**Figure 2** shows the results of dynamic light scattering results for polymersome of the invention. **Fig. 2A** shows dynamic light scattering plot of OVA encapsulating polymersomes with a monodisperse population of 173.1 nm (diameter). **Fig. 2B** shows a table of mean diameter (Z average) measured by DLS for different polymersomes encapsulated with different antigens. The names of the formulations e.g. "ACM-OVA", "ACM-CpG", "ACM-OVA-CpG" and "ACM-Trp2" shown in brackets are used elsewhere in the present application.

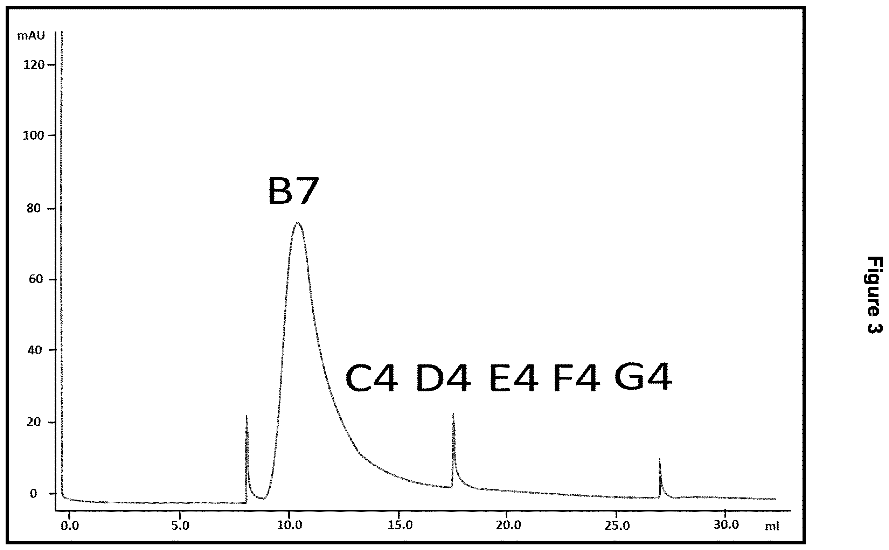

**Figure 3** shows an elution profile of OVA encapsulating polymersome in a size exclusion chromatography.

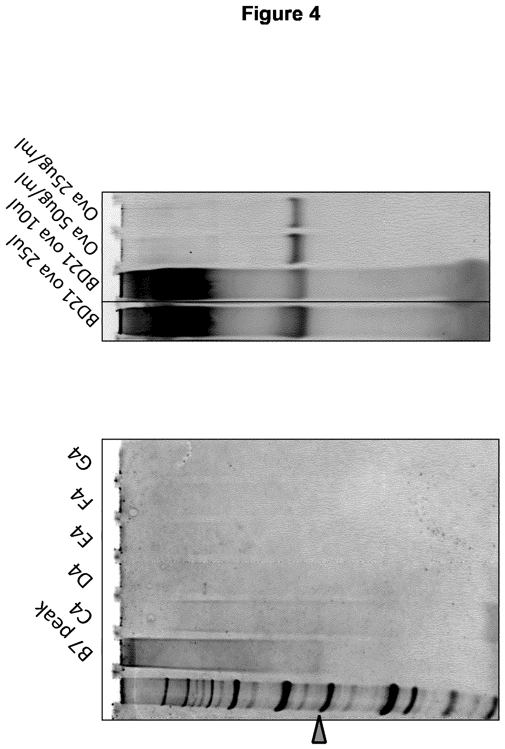

**Figure 4** shows sodium dodecyl sulphate-polyacrylamide gel electrophoresis (SDS-PAGE) of OVA encapsulating polymersomes.

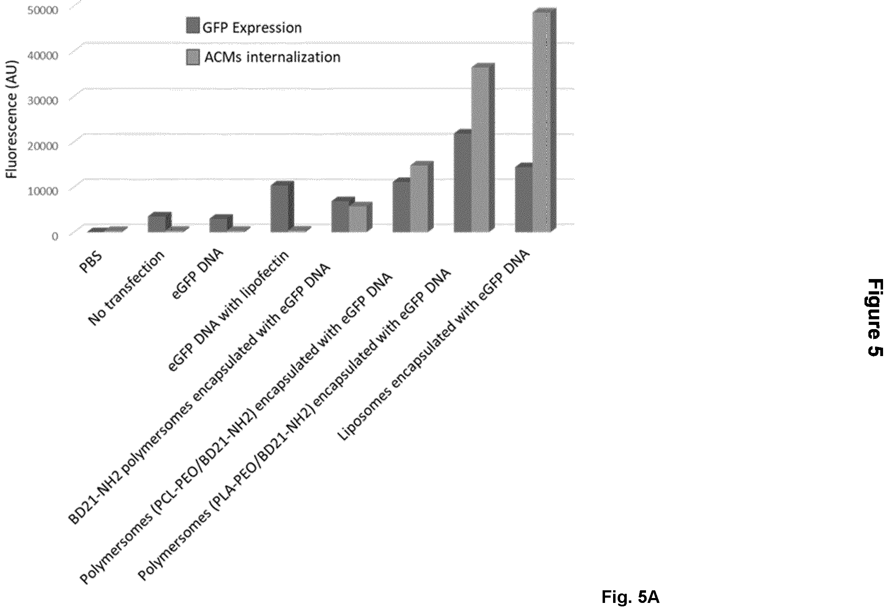

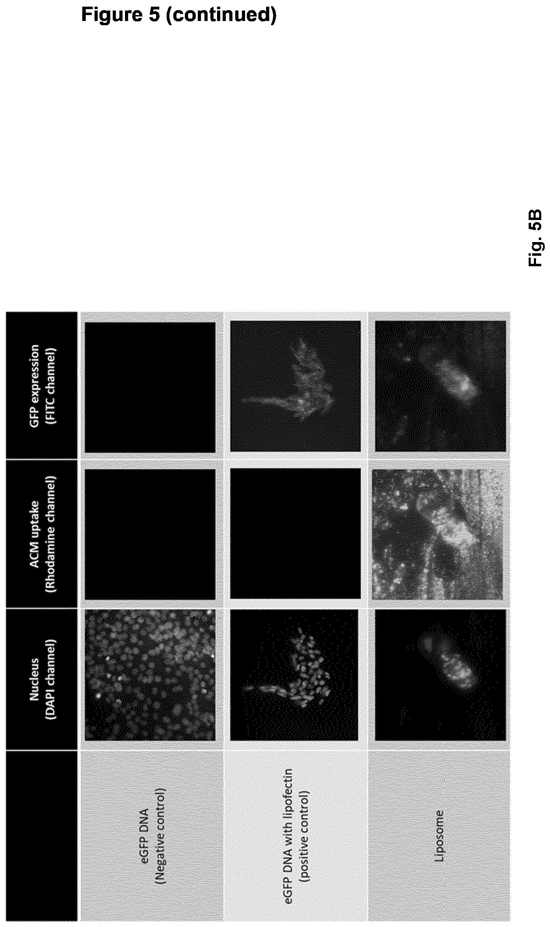

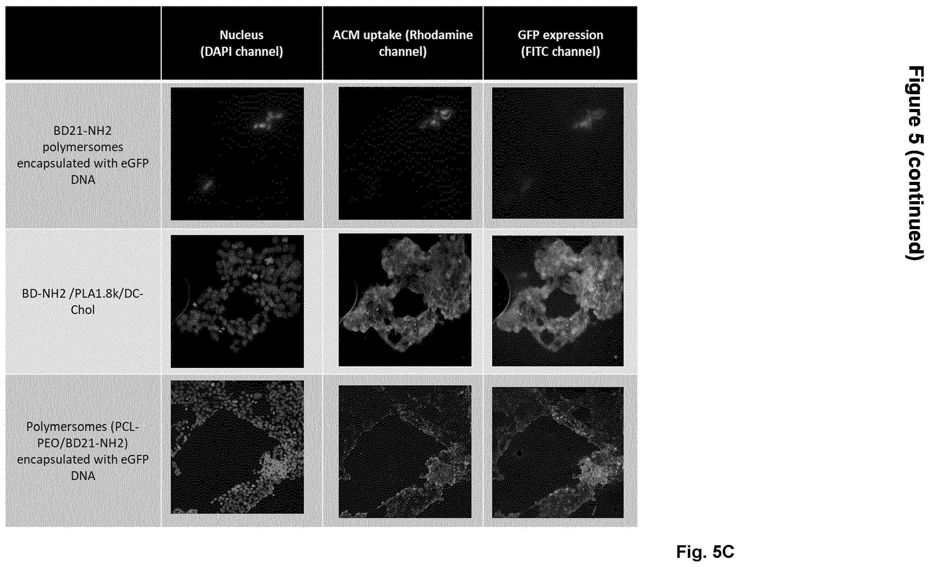

**Figure 5** shows the results of encapsulation of a nucleic acid (here the coding gene of enhanced Green Flourescent Protein (eGFP) in polymersomes of the invention and uptake of the polymers with the encapsulated nucleic acid in cells. **Fig.5A** shows fluorescence intensity uptake of different polymersomes inside the cells and eGFP expression based on the DNA encapsulated in the polymersomes, while **Fig. 5B** and **Fig.5C** show fluorescence images of cells that are transfected with DNA encapsulated polymersomes.

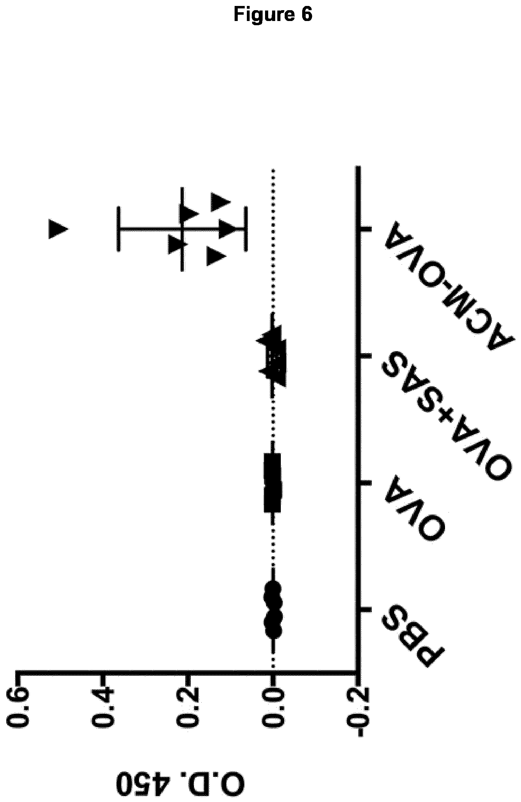

**Figure 6** shows antibody titers from the mice sera that were immunized with PBS, OVA alone, OVA with SAS adjuvant, OVA encapsulating polymersomes without adjuvants. Only ACM encapsulated OVA (herein after "ACM" refers to a polymersome of the present invention) was able to induce an IgG titer.

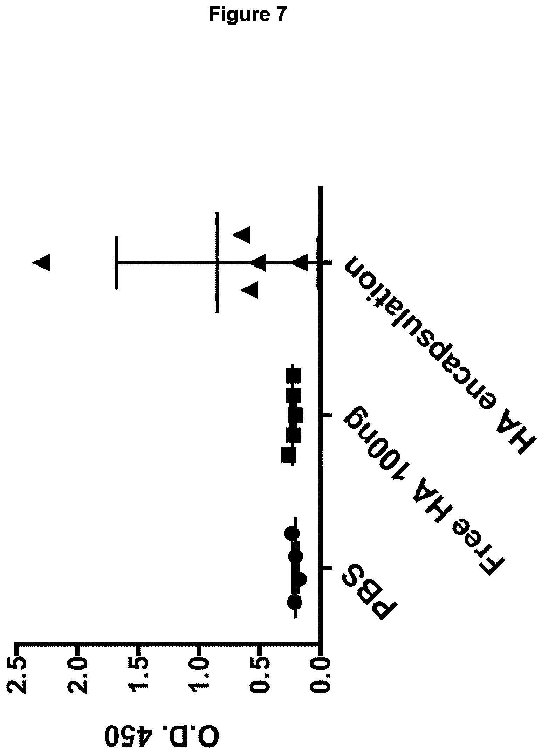

**Figure 7** shows antibody titers from the mice sera that were immunized with PBS, HA alone and HA encapsulating polymersomes without adjuvants. Only ACM encapsulated HA (polymersome of the present invention) was able to induce an IgG titer.

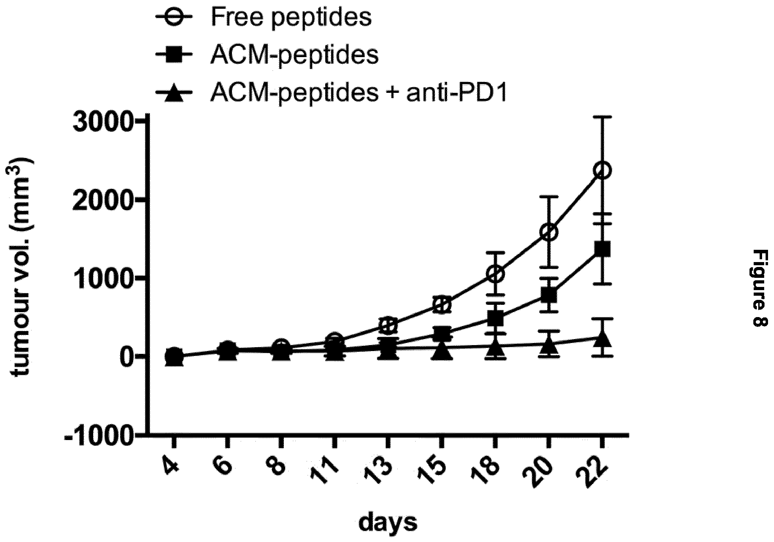

**Figure 8** shows results for a MC-38 mouse tumor model. Tumor volume was monitored in mice immunized with free peptides (open circle), ACM encapsulated peptides (closed square, polymersomes of the present invention) or with ACM encapsulated peptides together with an anti-PD1 antibody treatment (closed triangle). Tumor development was altered by ACM encapsulated peptides (polymersomes of the present invention) over free peptides, which is further potentiated by addition of the anti-PD1 antibody. No adjuvant was added in any of the groups.

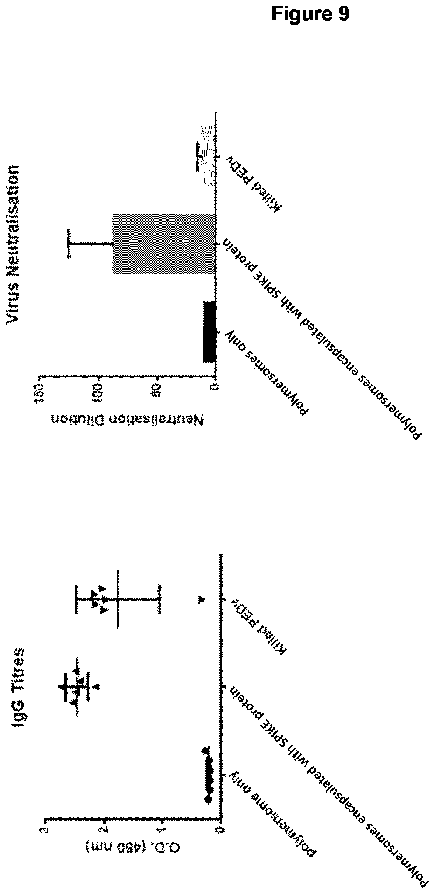

**Figure 9** shows IgG Antibody titres and virus neutralisation (against the strain PEDv USA/Colorado/2013 (CO/13)) from mice sera that were immunised with PBS and with a soluble fragment of the PEDv S Protein that has been encapsulated in a polymersome used as herein ("Polymersomes encapsulated with SPIKE protein") and in comparison, with killed PED virus ("Killed PEDv") and ACM polymersomes only (i.e., without any antigen, "polymersomes only"). From the IgG Titre of Fig. 9, it is evident that both the ACM encapsulated fragment of the PEDv S Protein and the killed virus induce IgG titres. The virus neutralisation data shows that only the ACM encapsulated PEDv S protein results in a significant neutralising titre while the negative control (ACM Polymersomes without any antigen) and killed PED virus show negligible neutralisation.

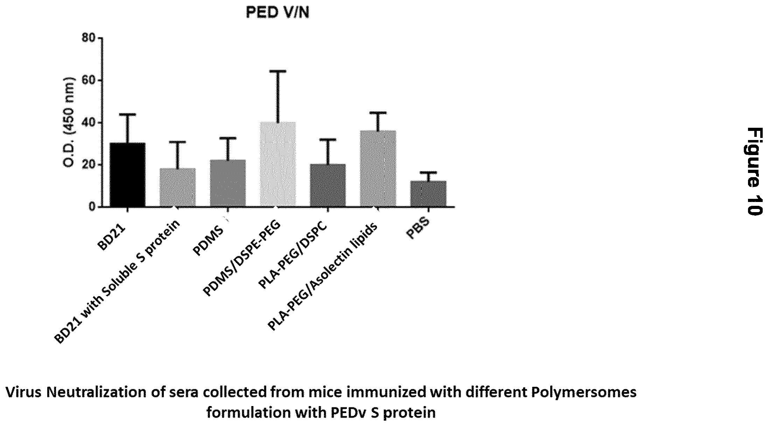

**Figure 10** shows virus neutralization data (against the strain PEDv USA/Colorado/2013 (CO/13)) from sera generated from mice after immunization with PBS and different polymersomes (e.g., BD21 (as defined later), PDMS₄₆-PEO₃₇ (marked in the figure just as "PDMS"), PDMS₄₆-PEO₃₇ with DSPE-PEG (distearoylphosphatidylethanolamine [DSPE] polyethylene glycol) as added lipid, polyethylene glycol-polylactic acid (PLA-PEG) with added Asolectin lipids (commercially available phospholipids from soybean) encapsulating either full length soluble PED spike protein (in the case of "BD21 with soluble S protein") or a S1 or S2 fragment thereof (in all other cases). From **Fig. 10****,** it is evident that the groups of mice immunized with PBS sample do not show any virus neutralization, whereas all polymersome formulations show varying degree of virus neutralization regardless of whether they encapsulate the full length protein or a fragment thereof.

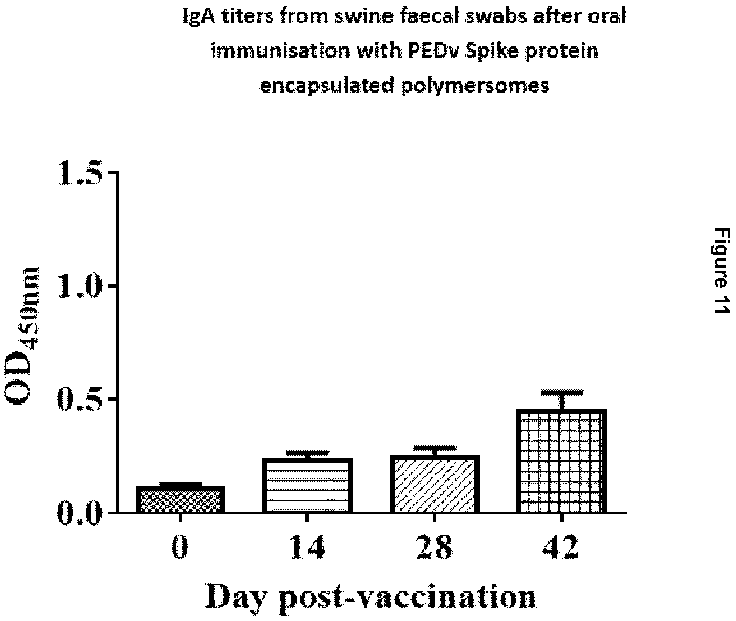

**Figure 11** shows IgA Antibody titers from swine immunised orally with ACM encapsulated PEDv S protein without the use of adjuvants. Titres are from faecal swabs. As seen in Fig. 11 the titres raises over time, showing that the orally administered polymersomes of the invention with PEDv S protein encapsulated therein, are able to elicit an immune response in the swine.

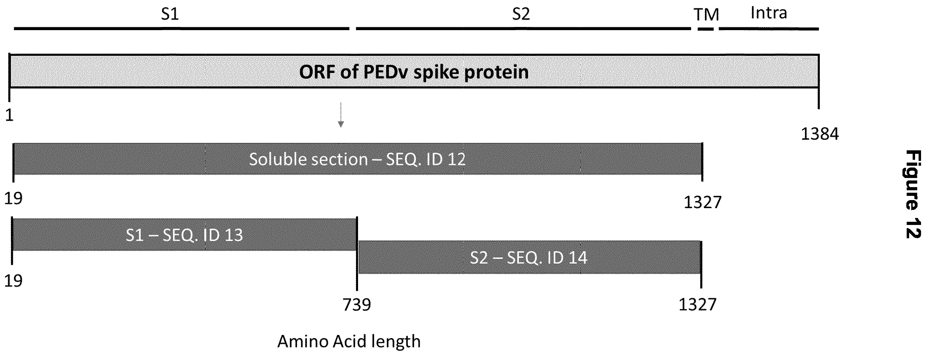

**Figure 12** shows a schematic representation of the Porcine Epidemic Diarrhea virus (PEDv) Spike protein (S Protein) (UniProtKB Accession number: V5TA78) and the soluble fragments of SEQ ID NO: 12 (amino acid residues 19 to 1327), SEQ ID NO: 13 (amino acid residues 19 to 739) and SEQ ID NO: 14 (amino acid residues 739 to 1327) that have been used for the encapsulation of soluble S Protein in polymersomes and subsequent immunization/vaccination of mice and pigs as described herein.

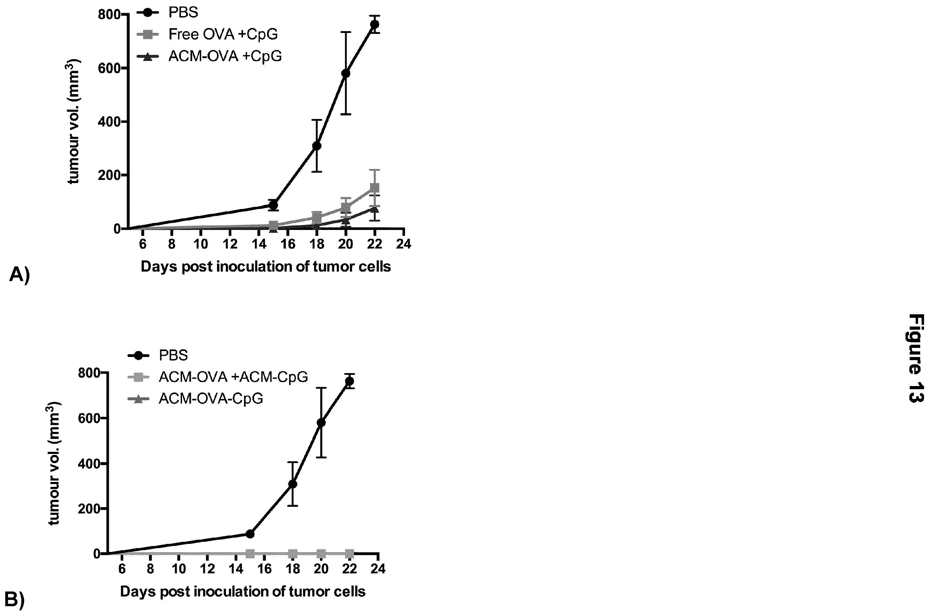

**Figure 13** shows the tumor growth curves after prophylactic vaccination of ACM OVA formulations. Tumor growth curves for mice administered with different OVA formulations with subsequent inoculation of 10⁵ B16-OVA cells. **Fig.13** A shows the PBS group, free OVA with CpG administered group and ACM encapsulated OVA with free CpG co-administered group, **Fig. 13B** shows the PBS group, ACM encapsulated OVA and ACM encapsulated CpG co-administered and ACM encapsulated with OVA and CpG together.

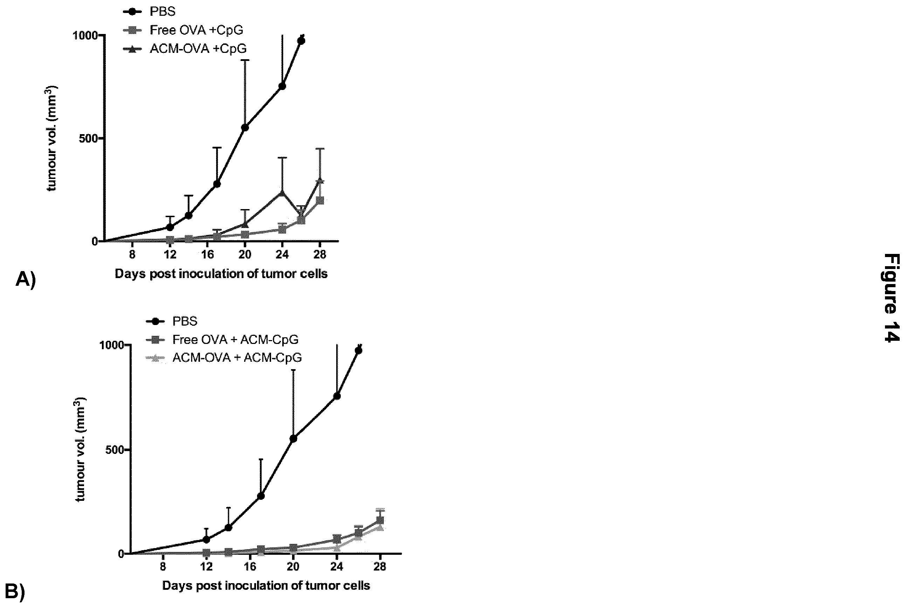

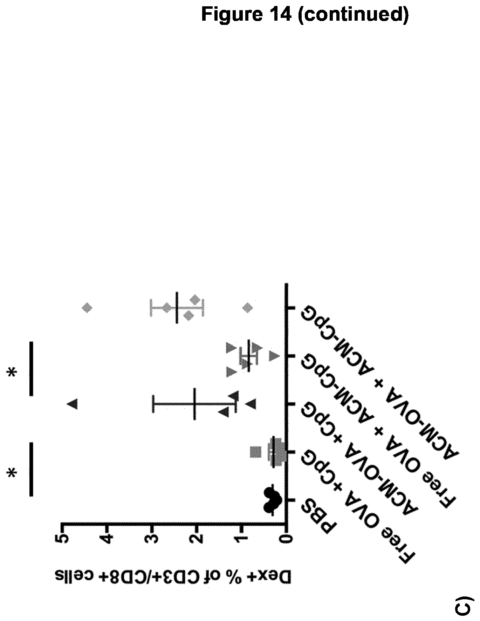

**Figure 14** shows the tumor growth curves after therapeutic vaccination of ACM OVA formulations. Tumor growth curves for mice vaccinated with different ACM OVA formulations and subsequent inoculation of 10⁵ B16-OVA cells. **Fig. 14A**) PBS group, free OVA with CpG administered group and ACM encapsulated OVA with free CpG co-administered group, B) PBS group, free OVA with CpG encapsulated ACM and ACM encapsulated OVA and ACM encapsulated with CpG co-administered C) OVA specific CD8 T cells quantified using dextramer specific for the CD8 T cell SIINFEKL (SEQ ID NO: 16) peptide epitope.

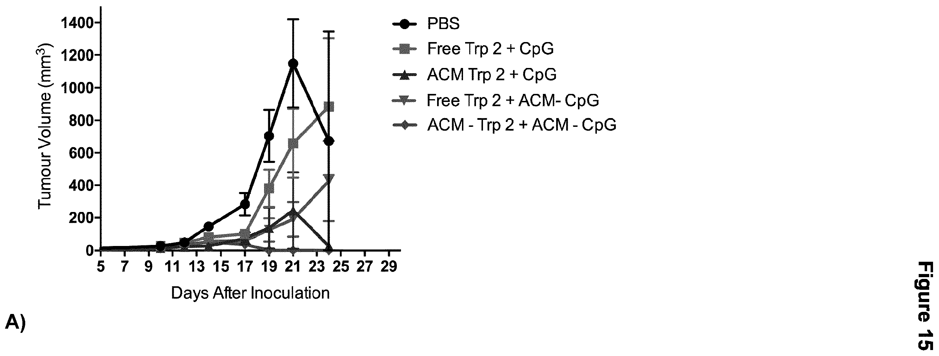

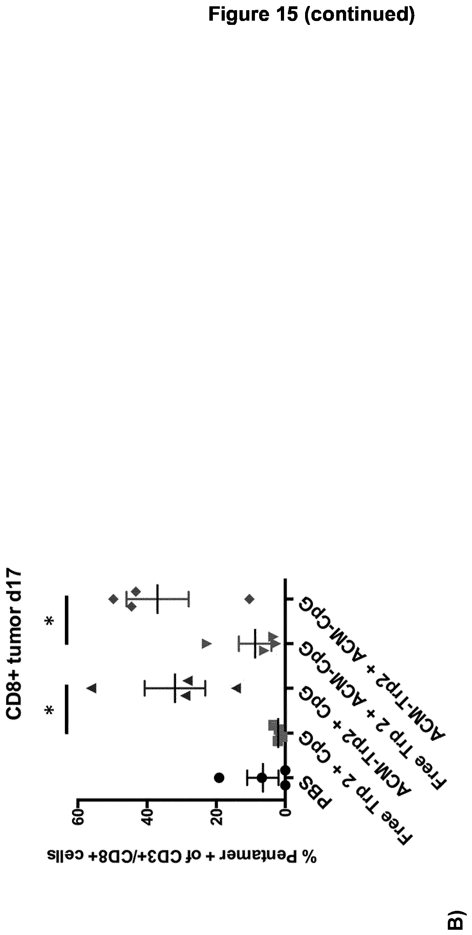

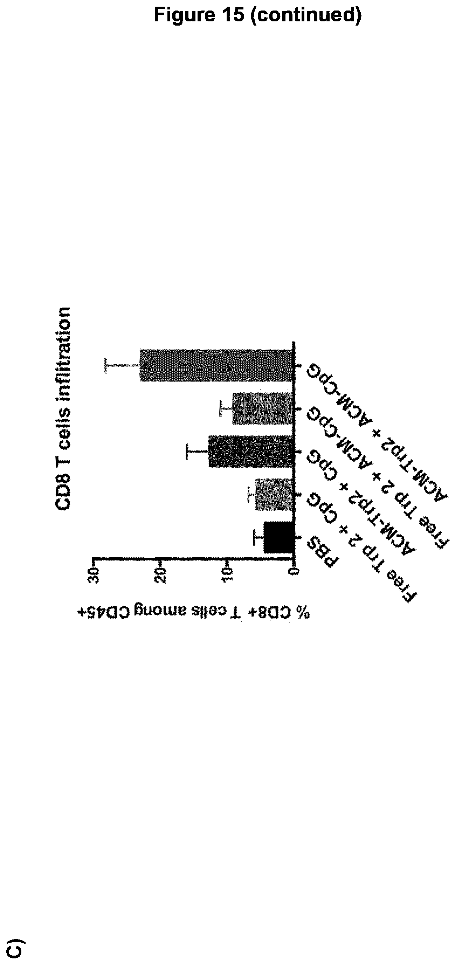

**Figure 15** shows the tumor growth curves of therapeutic vaccination of ACM melanoma B16F10 formulations of mice inoculated with 10⁵ B16F10 cells. **Fig. 15** A shows PBS group, free Trp2 (SEQ ID NO:9) and CpG co-administered, ACM encapsulated with Trp2 and CpG co-administered, free Trp2 and ACM encapsulated CpG co-administered and ACM encapsulated Trp2 and ACM encapsulated CpG co-administered together, **Fig. 15B** shows Trp2 specific CD8 specific T cells quantified in blood using pentamer specific for the CD8 T cell SVYDFFVWL (SEQ ID NO: 17) peptide epitope, and **FIG15****.C** shows CD8 T cell infiltration in tumors.

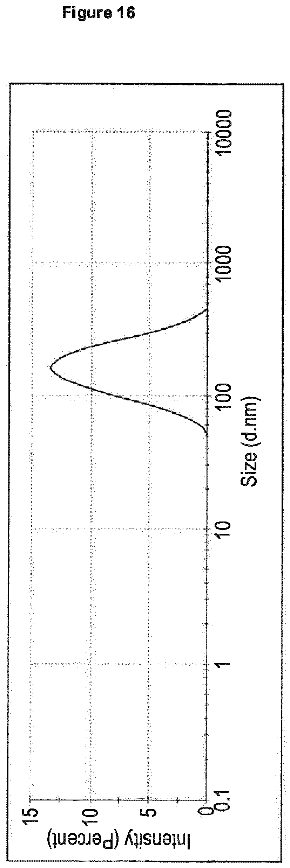

**Figure 16** shows Dynamic Light Scattering (DLS) spectra of OVA conjugated ACMs.

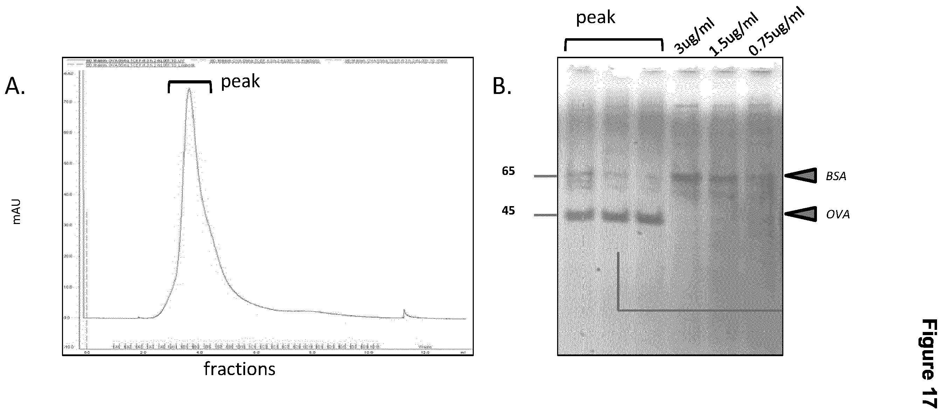

**Figure 17** shows characterization of OVA conjugated ACMs with **Fig.17A** showing a size exclusion chromatography (SEC) profile of OVA conjugated ACMs and **Fig.17B** shows an SDS-PAGE loaded with samples from SEC peak and stained using silver staining.

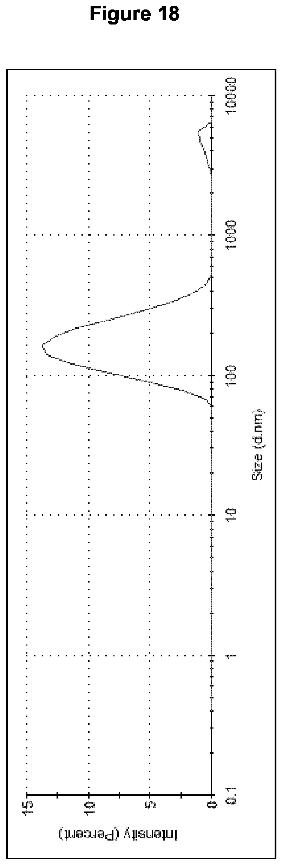

**Figure 18** shows DLS spectra of HA conjugated ACMs.

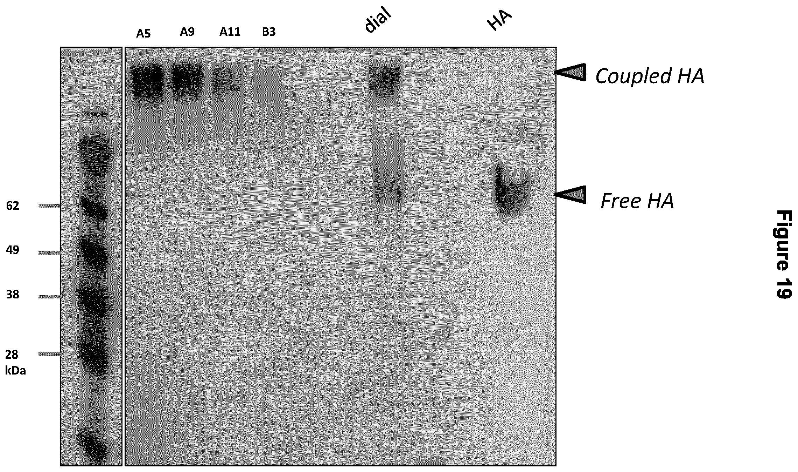

**Figure 19** shows an immunoblot of ACM conjugated HA samples. Coupled and free HA migrate differently.

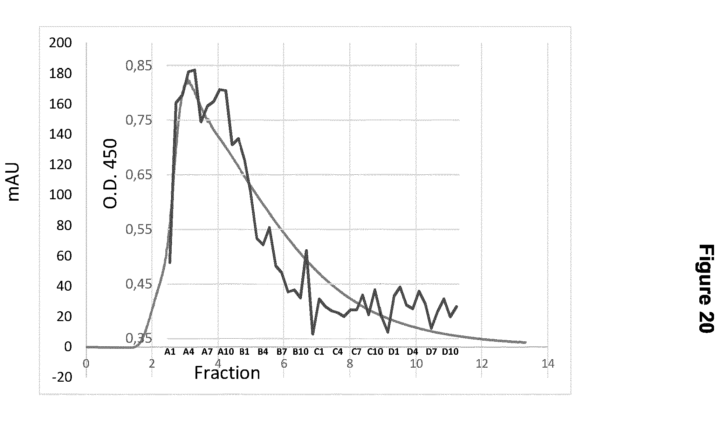

**Figure 20** shows SEC profile of HA conjugated ACMs (mAU, light gray trace) superimposed with ELISA signals performed on all collected fractions (O.D. 450, black trace).

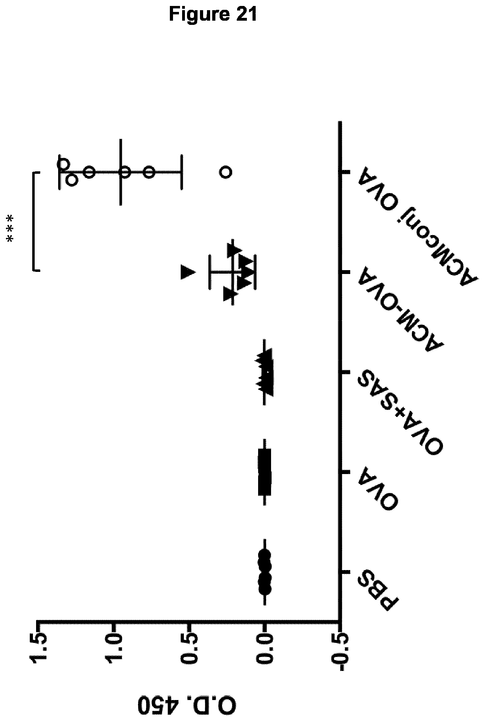

**Figure 21** shows antibody titers from sera of immunized C57BI/6 mice with PBS, free OVA, free OVA with SAS, BD21 encapsulated OVA and BD21 conjugated OVA, *p<0.01.*

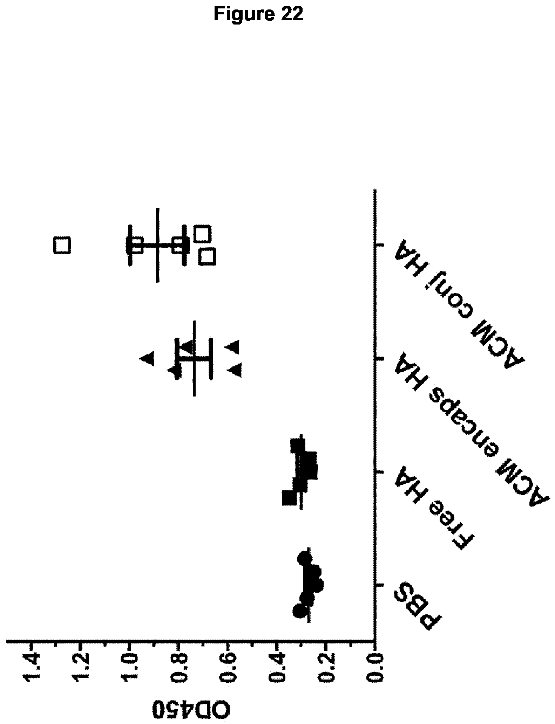

**Figure 22** shows antibody titers from sera of immunized Balb/c mice with PBS, free HA, BD21 encapsulated HA and BD21 conjugated HA.

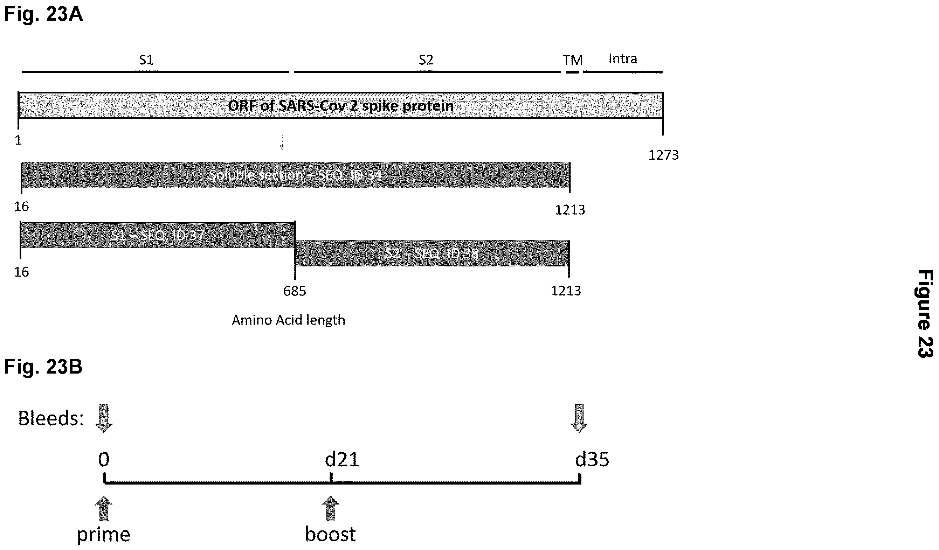

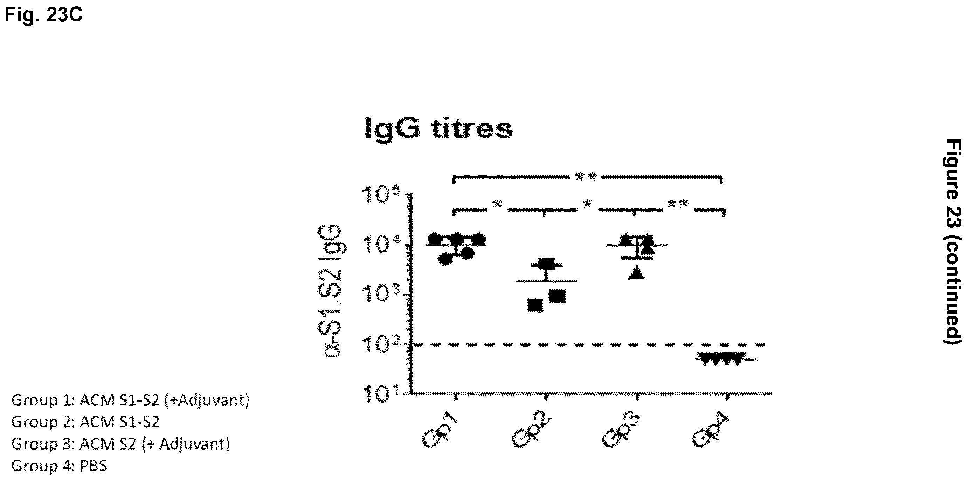

**Figure 23A** shows a schematic representation of the severe acute respiratory syndrome coronavirus 2 (SARS-CoV-2) Spike protein (S Protein) (UniProtKB Accession number: P0DTC2) and the soluble fragments of SEQ ID NO: 34 (amino acid residues 16 to 1213), SEQ ID NO: 37 (amino acid residues 16 to 685) and SEQ ID NO: 38 (amino acid residues 685 to 1213). According to UniProtKB, the amino acids 1214 to 1234 form the transmembrane region and positions 1235 to 1273 form the intraviral region. The endpoints of S1 and S2 segments, the transmembrane region, and/or intraviral region may vary depending on the prediction software. **Fig. 23B** shows the protocol for immunization of mice with ACMs having encapsulated SARS-CoV-2 spike protein. **Fig. 23C** shows the IgG titres measured in Balb/c mice at day 35 that were immunized with the following formulations: BD21 encapsulated soluble S1 and S2 segments co-administered with adjuvant (Group 1), BD21 encapsulated soluble S1 and S2 segments (Group 2), BD21 encapsulated soluble S2 segment co-administered with encapsulated adjuvant (Group 3), and PBS as negative control (Group 4)

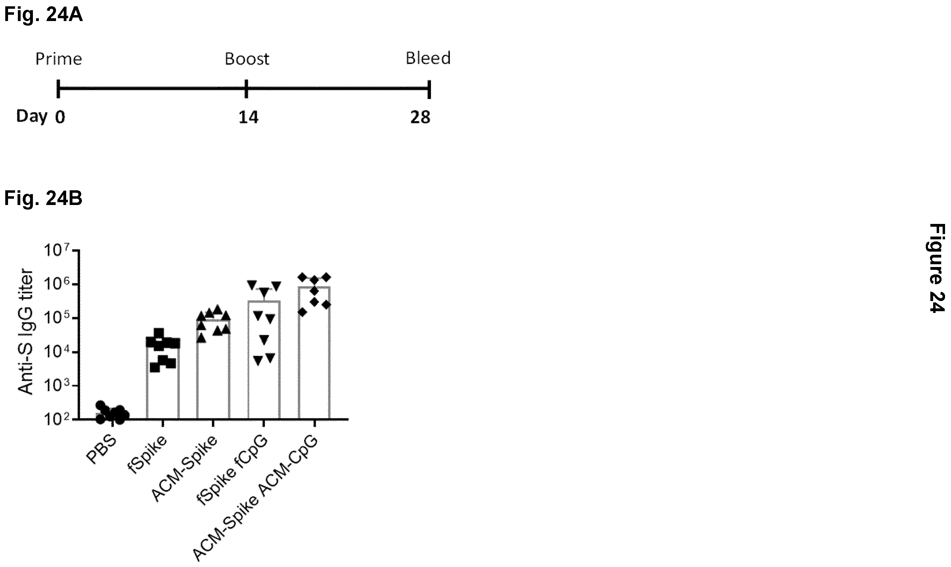

**Figure 24** shows the protocol and results of mice that were immunized with ACM encapsulated full length soluble encapsulated SARS-CoV-2 spike protein. **Fig. 24A** shows the immunization protocol. **Fig. 24B** shows the titers of IgG antibodies against SARS-CoV-2 spike protein 28 days after the first immunization for four groups. following formulations were prepared: i) free recombinant spike protein "fSpike"); ii) BD21 polymersome-encapsulated spike protein ("ACM-Spike"); iii) a mixture of free spike protein and free CpG adjuvant ("fSpike fCpG"); iv) a mixture of BD21 polymersome-encapsulated spike protein and BD21 polymersome-encapsulated CpG ("ACM-Spike ACM-CpG").

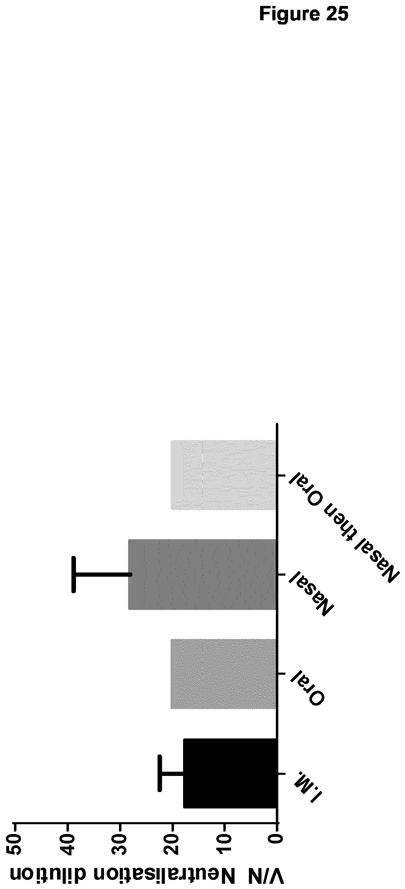

**Figure 25** shows the result of a virus neutralization assay (PEDv) after immunization of guinea pigs with ACMs having encapsulated PEDv S2 spike protein mixed with ACMs having encapsulated CpG that were administered by different routes.

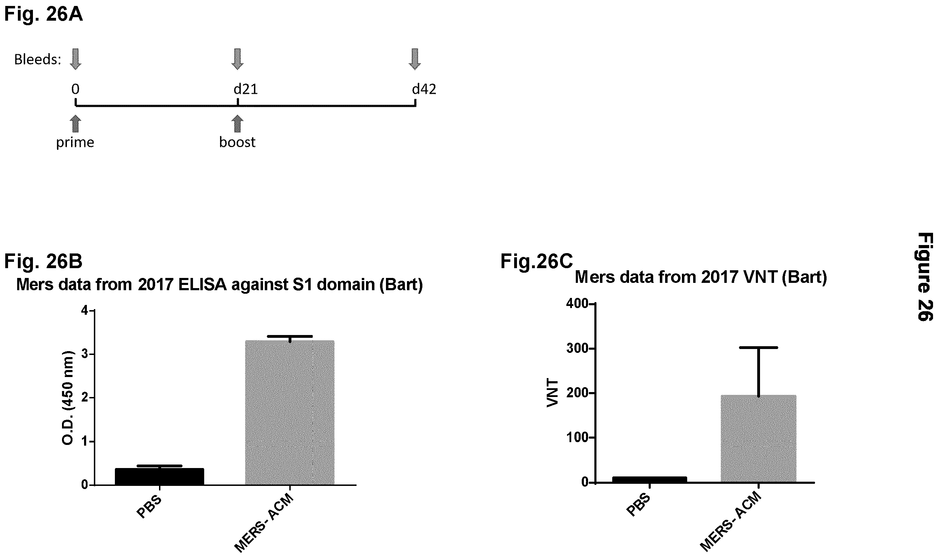

**Figure 26** shows protocol and results of mice that were immunized with ACMs having encapsulated MERS spike protein. **Fig. 26A** shows the immunization protocol, **Fig. 26B** shows the result of an ELISA against MERS-CoV spike protein S1 domain. **Fig. 26C** shows the results of the virus neutralization assay (MERS-CoV).

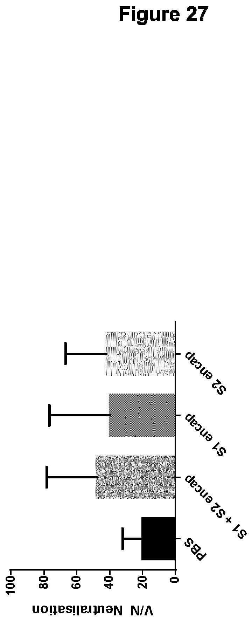

**Figure 27** shows results of a virus neutralisation assay (PEDv) for mice that were immunized with ACM having encapsulated either the S1 domain or the S2 domain or a mixture of S1 and S2 domains of the PEDv Spike protein.

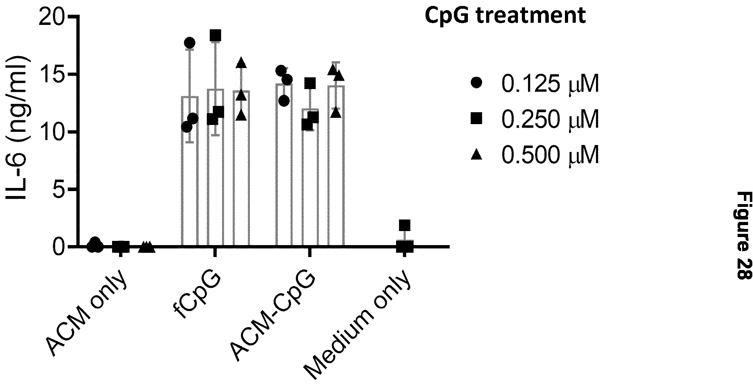

**Figure 28** shows results of the CpG treatment carried out demonstrating successful *in vitro* induction of cytokines by ACM-CpG adjuvants due to innate immune response.

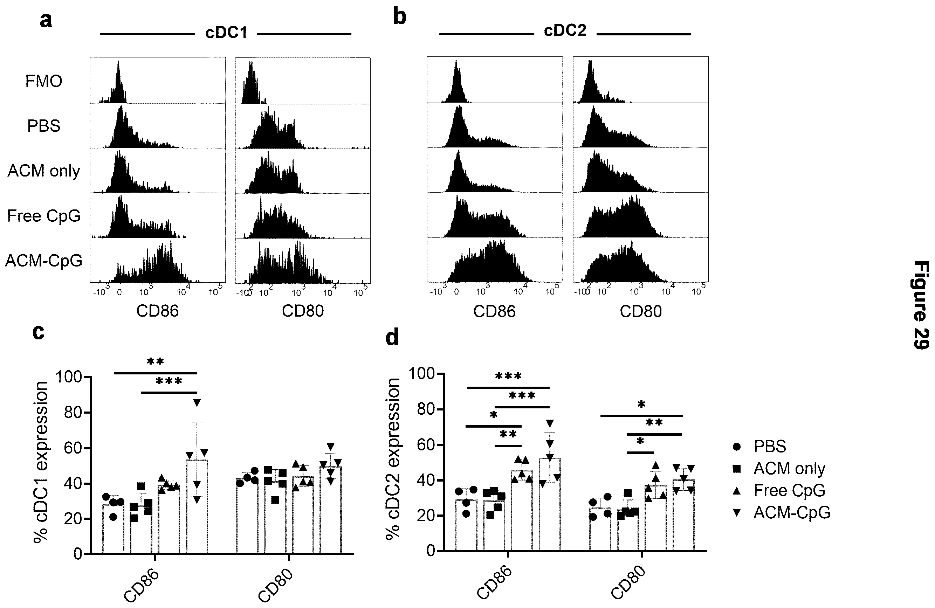

**Figure 29****: Activation of cDCs by free or ACM-CpG.** a, b. Representative histograms showing expression of CD86 and CD80 activation markers on cDC1 and cDC2. Mice were SC injected PBS, empty ACM, free CpG or ACM-CpG and cDCs from inguinal lymph nodes were examined two days after. c, d. Comparison of CD86⁺ and CD80⁺ cDC1 and cDC2 among treatment groups.

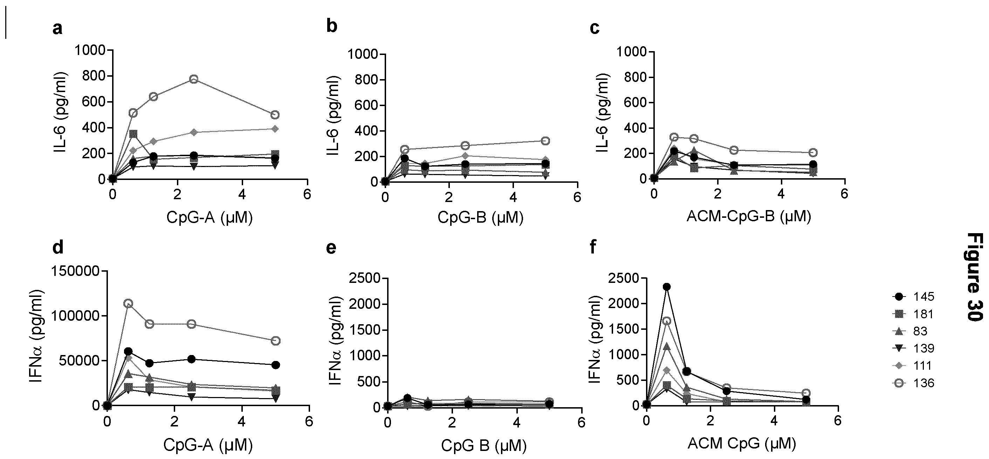

**Figure 30****: Cytokine profile of free or ACM-encapsulated CpG-B.** a-c. IL-6 production by human PBMCs after incubating with free CpG-A, free CpG-B or ACM-CpG-B. d-f. IFNα production. Individual dose-response curves are shown. Numerical identities of healthy donors are indicated at the bottom right.
**Figure 31****: Exemplary CpG ODN classes derived and modified from** https://www.invivogen.com/cpg-odns-classes. Exemplary CpG-A ODNs are characterized in that they comprise a PO central CpG-containing palindromic motif and a PS-modified 3' poly-G string. Exemplary CpG-B ODNs are characterized in that they comprise a full PS backbone with one or more CpG dinucleotides. Exemplary CpG-C ODNs are characterized in that they combine features of both classes A and B.

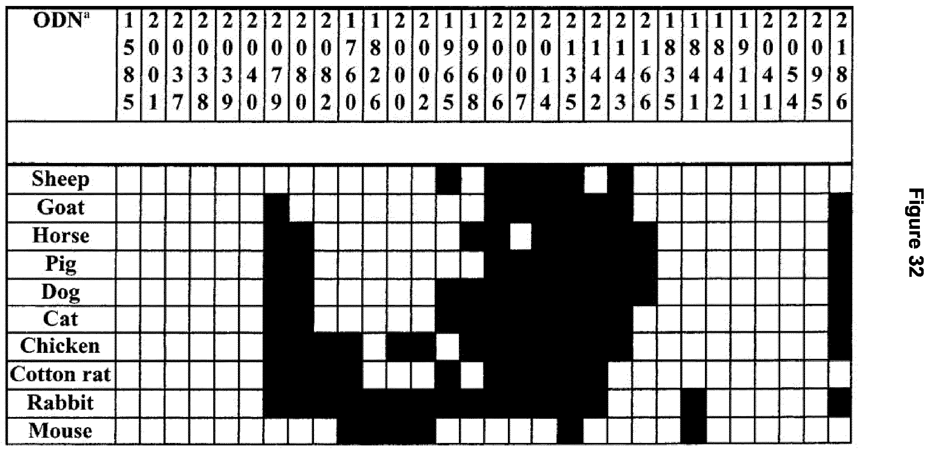

**Figure 32****: Reactivity patterns of individual species to each CpG** ODN (adopted from Rankin et al., 2001). ^{a}Compartments are shaded if the mean proliferative response of a particular ODN and species was at least 50% of the value for the maximally stimulatory ODN for that species.

### Detailed Description of the Invention

The present invention relates to compositions for use in a method of eliciting an immune response (e.g., innate immune response) in a subject by sole administration of one or more adjuvants, wherein said one or more adjuvants are associated with one or more populations of polymersomes. This essentially means that one or more adjuvants of the present invention can be, for example, individually encapsulated within one or more populations of polymersomes of the present invention and the polymersomes can be administered to a subject, thereby eliciting an immune response (e.g., innate immune response). In the context of the present invention, sole administration of one or more adjuvants may be characterized in that no antigen (e.g., capable of eliciting an immune response, e.g., antibody-mediated immune response) is administered in combination (e.g., co-administered) with the one or more adjuvants associated with one or more populations of polymersomes. Sole administration can thus be characterized in that no antigen is administered to the subject either simultaneously (at the same time) or at a different time. In preferred embodiments one or more adjuvants of the present invention are encapsulated in polymersomes of the present invention. In another preferred embodiment the adjuvant of the present invention is selected from the group consisting of: CpG oligodeoxynucleotides (or CpG ODN, e.g., any suitable CpG ODN described herein), or components derived from bacterial and mycobacterial cell wall and proteins.

The present invention is based on the surprising finding that two separate populations of polymersomes, wherein the first population of polymersomes is associated with only antigen and the second population of polymersomes is associated with only adjuvant, when administered together, improve the immune response to the antigen, thereby providing either immunization or a curative effect, for example, to an infectious disease or cancer (cf. Examples 7 to 9 or Example 19 of the present application, with Example 8 showing that administration of a first polymersome population having encapsulated antigen together with a separate second polymersome population having encapsulated CpG (adjuvant) produce an immune response in mice for which both the tumor load and T-cell infiltration correlates, with Example 9 showing that administration of an immunogenic tumor neoantigen Trp2 peptide encapsulated in a first population of polymersomes together with a CpG oligonucleotide (adjuvant) encapsulated in a second (separate) population showed a much stronger anti-tumor response compared to, for example, free Trp2 peptide and with Example 19 showing the highest immune response against the spike protein of the Sars-CoV-2 virus when the spike protein of Sars-CoV-2 is encapsulated in a first population of polymersomes and a CpG oligonucleotide (adjuvant) is encapsulated in a second (separate) population. The finding that such two separate populations of polymersomes result in an improved immune response has the added advantage that is allows to produce the two populations of polymersomes separately/independently from each other. This in turn simplifies, for example, GMP production of a respective vaccine or therapeutic composition, since the first population of polymersomes, which for example, comprises an antigen encapsulated in the polymersomes or conjugated to the surface of the polymersomes, can be produced under standardized GMP conditions, while the second population of polymersomes, which, for example, comprises an adjuvant encapsulated in the polymersomes or conjugated to the surface of the polymersomes, can also be produced under standardized conditions. These two populations can then be combined either in the manufacturing process (to yield a composition that combines both populations of polymersomes for co-administration) or can be administered to a subject separately. Such a drug/vaccine manufacturing process is much easier to control than to, for example, encapsulate both antigen and adjuvant in the same polymersome population.

The antigen can be associated with the first population of polymersomes by any possible interaction of the antigen with the first population of polymersomes. For example, the antigen may be encapsulated within the first population of polymersomes as described in co-pending PCT application PCT/EP2019/051853, filed 25 January 2019. Alternatively, the antigen may be integrated into the circumferential membrane of the polymersomes of the first population of polymersomes as described in International Application WO2014/077781. It is also possible that the antigen is conjugated to the exterior surface of the polymersomes of the first polymersome population via a covalent bond as described in co-pending European patent application 18193946.3, filed 12 September 2018.

It is further possible to conjugate the antigen to the exterior surface of the polymersomes of the first polymersome population via a non-covalent bond. Examples of such non-covalent bonds include electrostatic interactions such as salt-bridges between positively and negatively charged residues that are present on surface of the polymersome or the surface of the antigen. For example, a salt bridge can be formed between a positively charged amino group (NH₂ group) and a negatively charged carboxylate group (COOH). A further illustrative example of such a non-covalent interaction between the first polymersome population and the antigen are binding pair between streptavidin and biotin, avidin and biotin, streptavidin and a streptavidin binding peptide, or avidin and an avidin binding peptide. For example, polymersomes with biotin groups located on their surface can be prepared as described in Broz et al "Cell targeting by a generic receptor-targeted polymer nanocontainer platform" Journal of Controlled Release. 2005;102(2):475-488 and can be reacted with an antigen that is conjugated to streptavidin or avidin. Non-covalent biotin-streptavidin conjugates of polymersomes with antigens can also prepared as described by Egli et al, "Functionalization of Block Copolymer Vesicle Surfaces Polymers" 2011, 3(1), 252-280. In this context, the term "an antigen associated with a first population of polymersomes" as used herein does not mean that only one particular antigen is associated with the first population of polymersomes but also includes that more than one, for example, two or more antigens can be associated with the first population of polymersomes. As an illustrative example, for example, two or more immunogenic peptides can be associates with a first population of polymersomes of the present invention. It is also possible that one or more immunogenic peptides and respective nucleic acid molecules encoding these peptides are associated with a first population of polymersomes as used herein. The term "an antigen associated with a first population of polymersomes" as used herein also means that two or more first populations of polymersomes, each of which carries a different antigen can be used in the present invention. For example, it is possible to use two different antigenic peptides and associate each of them with a separate first polymersome population of the invention.

The adjuvant can be associated with the second population of polymersomes by also any possible interaction, in the same manner as the association of the antigen with the first population of polymersomes can occur. This means, the adjuvant may be encapsulated within the first population of polymersomes as described in co-pending PCT application PCT/EP2019/051853, filed 25 January 2019. Alternatively, the adjuvant may be integrated into the circumferential membrane of the polymersomes of the first population of polymersomes as described in International Application WO2014/077781. Illustrative examples of adjuvants that can be incorporated/integrated into the circumferential membrane of polymersomes (of the second polymersome population) include synthetic monophosphoryl lipid A (cf. in this respect Cluff "Monophosphoryl Lipid A (MPL) as an Adjuvant for Anti-Cancer Vaccines: Clinical Results" in Lipid A in Cancer Therapy, edited by Jean-François Jeannin, 2009 Landes Bioscience and Springer), polysorbate 80, Alpha-DL-Tocopherol, dioleoyl-3-trimethylammonium propane (DOTAP), the cationic lipid 1-[2-(oleoyloxy)ethyl]-2-oleyl-3-(2-hydroxyethyl)imidazolinium chloride (DOTIM) (see Bernstein et al "The Adjuvant CLDC Increases Protection of a Herpes Simplex Type 2 Glycoprotein D Vaccine in Guinea Pig" Vaccine. 2010 May 7; 28(21): 3748-3753, or the synthetic amphiphile dimethyldioctadecylammonium (DDA) (see Smith Korsholm et al "The adjuvant mechanism of cationic dimethyldioctadecylammonium liposomes" Immunology,121,216-226) to name only a few. It is evident in this context, the one or more adjuvants can be present in the polymersomes of the second polymersome population used herein. For example, the second polymersome population may comprise an encapsulated adjuvant such as a CpG oligonucleotide and an adjuvant that is integrated into the circumferential membrane of the polymersomes such as monophosphoryl lipid A or DOTAP (in accordance with the above disclosure the second polymersome population is however free of antigen, meaning it does not contain any antigen).

In line with the above, it is of course also possible that the adjuvant is conjugated to the exterior surface of the polymersomes of the first polymersome population via a covalent bond as described in co-pending European patent application 18193946.3 filed 12 September 2018. Alternatively, the conjugation of the adjuvant to the exterior surface of the polymersome may also tale place via a non-covalent bond such as a biotin-streptavidin interaction. It is noted here that CpG oligonucleotides such as the class B CpG oligodeoxynucleotide CpG ODN1826 (5'-tccatgacgttcctgacgtt-3', SEQ ID NO: 18) is available in biotinylated form and can thus be readily reacted with a biotinylated polymersome that is "decorated" with streptavidin as described in Broz et al "Journal of Controlled Release. 2005; supra*.* Also, from this example it is evident that the second polymersome population may carry more than one (kind of) adjuvants, for example, a CpG oligonucleotide covalently or non-covalently conjugated to the exterior surface of the polymersomes and a further adjuvant such as monophosphoryl lipid A or DOTAP integrated into the circumferential membrane of the polymersomes. It is further evident that the same adjuvant may be associated with the second polymersome population in different ways, for example, a CpG oligonucleotide can be encapsulated into the polymersomes and at the same time covalently or non-covalently conjugated to the exterior surface of the polymersome. By so doing, a higher amount of adjuvant can be provided for administration, if desired.

In line with the above disclosure, any kind of first polymersome population can be used for administration with any kind of second polymersome population, regardless of how the antigen and the adjuvant is associated with the first and second polymersome population. For example, the first population of polymersomes may have the antigen encapsulated within the polymersomes and also the second population of polymersomes may have the adjuvant encapsulated within the polymersomes. Alternatively, the first population of polymersomes may have the antigen conjugated to the exterior surface of the polymersomes by a covalent or a non-covalent bond while also the second population of polymersomes has the adjuvant conjugated to the exterior surface of the polymersomes by a covalent or a non-covalent bond. As a further purely illustrative example, the first population of polymersomes may have the antigen integrated into the circumferential membrane of the polymersomes and the second population of polymersomes may also have the adjuvants integrated into the circumferential membrane of the polymers. As further illustrative examples, the first population of polymersomes may have the antigen encapsulated within the polymersomes while the second population of polymersomes may have a) the adjuvant conjugated to the exterior surface of the polymersomes by a covalent or non-covalent bond or b) may also have the adjuvant integrated into the circumferential membrane of the polymersome. As yet a further illustrative example, the first population of polymersomes may have the antigen conjugated to the exterior surface of the polymersomes by a covalent bond and the second population of polymersomes may have the adjuvant encapsulated within the polymersomes.

Addressing now the administration of the two polymersome populations of the invention in more detail: the first population of polymersomes and the second population of polymersomes can be administered to a subject either simultaneously (i.e. at the same time) or at a different time. In case the two populations are simultaneously administered, the two populations of polymersomes may be administered together (i.e. by co-administration). In that case, the two populations of polymersomes are combined or mixed together prior to administration and are thus present in the same composition, for example, a pharmaceutically acceptable carrier (such as a physiological buffer or a solid formulation suitable for oral administration). In case of administration at the same time, it is however also possible to administer each of the two populations of polymersomes individually. In that case, the two populations of polymersomes are of course not combined with each other prior to administration, and for example may be administered via two or more separate injections.

The two populations of polymersomes can be administered to a chosen subject in any way that is known for eliciting an immune response in a subject and that is suitable for administering the polymersome population to the given subject. In case fish or farm animals such as chicken, pigs or sheep are to be immunized, it may be advantageous to use oral administration, for example, and formulate a composition containing the two polymersome populations of the invention as food additive. Alternatively, intradermal administration by means of an injection gun or jet injector may be used for farm animals. For humans, both invasive and non-invasive administration can be used. Suitable administration routes for both human and non-human animals include but are not limited to oral administration, intranasal administration, administration to a mucosal surface, inhalation, intradermal administration, intraperitoneal administration, subcutaneous administration, intravenous administration or intramuscular administration.

Turning to conjugation of the antigen and/or the adjuvants to exterior surface of polymersomes of either the first or second polymersome population in more detail, the covalent bond can be any suitable covalent bond capable of conjugating an antigen (e.g., the antigen of the present invention) or an adjuvant to the exterior surface of the polymersome of the present invention. Conjugating reactions producing covalent bonds of the present invention are well known in the art (e.g., NHS-EDC conjugations, reductive amination conjugations, sulfhydryl conjugations, "click" and "photo-click" conjugations, pyrazoline conjugations etc.). Non-limiting examples of such covalent bonds and methods of producing thereof are listed below herein. Thus, in some aspects, the covalent bond via which the antigen or adjuvant of the present invention is conjugated to the exterior surface of the polymersome of the present invention comprises: i) an amide moiety (e.g., as described in the Examples section herein); and/or ii) a secondary amine moiety (e.g., as described in the Examples section herein); and/or iii) a 1,2,3-triazole moiety (e.g., as described in van Dongen et al., 2008, Macromol. Rapid Communications, 2008, 29, pages 321-325), preferably said 1,2,3-triazole moiety is a 1,4-disubstituted[1,2,3]triazole moiety or a 1,5-disubstituted[1,2,3]triazole moiety (e.g., as described in Boren et al., 2008); and/or iv) pyrazoline moiety (e.g., as described in de Hoog et al., Polym. Chem., 2012,3, 302-306) and/or an ether moiety. It is noted in this context that it might be necessary to modify both the polymersome and the antigen, for example a protein, for the conjugation/formation of the covalent bond between the exterior surface of the polymersome and the antigen. In addition to classical chemical conjugation chemistry (reaction) as described above, it is also possible to form the covalent bond between the exterior surface of the polymersome and the antigen by enzymatic reaction.

In some aspects, the present invention relates to NHS-EDC conjugation (i.e., conjugation based on N-hydroxysuccinimide (NHS), and 1-Ethyl-3-(3-dimethylaminopropyl)-carbodiimide (EDC)) is one of the exemplary alternative ways of conjugating antigens to polymersomes of the present invention. In this method, carboxylic acid groups react with EDC producing an intermediate O-acylisourea that is then reacts with primary amines to form an amide moiety with said carboxyl group.

In some aspects, the present invention relates to a reductive amination conjugation, which is another exemplary alternative way of conjugating antigens or adjuvants to polymersomes of the present invention. In this method an aldehyde-containing compound is conjugated to amine-containing compound to form a Schiff-base intermediate that in turn undergoes reduction to form a stable secondary amine moiety.

In some aspects, the present invention relates to a sulfhydryl conjugation, which is another exemplary alternative way of conjugating an antigen or adjuvant to polymersomes of the present invention. In this method sulfhydryl (-SH) containing compound (e.g., present in side chains of cysteine) is conjugated to sulfhydryl-reactive chemical group (e.g., maleimide) via alkylation or disulfide exchange to form a thioether bond or disulfide bond respectively.

In some aspects, the present invention relates to a so-called "click" reaction (also known as "azide-alkyne cycloaddition") on polymersome surface (e.g., described by van Dongen et al., 2008, supra), which is another exemplary alternative way of conjugating antigens to polymersomes of the present invention. According to this method a 1,2,3-triazole moiety is produced in that an aqueous solution of azido-functionalised antigens (e.g., a polypeptide) is added to a dispersion of polymersomes, followed by an addition of a premixed aqueous solutions of Cu(II)SO₄·5H₂O with sodium ascorbate and bathophenanthroline ligand to the resulting dispersion of polymersomes and then left at 4°C for 60 hours, followed by filtering of said dispersion with a 100 nm cutoff and centrifuging to dryness. In this context it is further noted that copper-catalysed reaction of azide-alkyne cycloaddition" (also known as CuAAC) allows for synthesis of the 1,4-disubstituted regioisomers specifically, whereas a ruthenium-catalysed reaction of azide-alkyne cycloaddition (also known as RuAAC) (e.g., using Cp*RuCl(PPh₃)₂ as catalysator) allows for the production of 1,5-disubstituted triazoles (cf. R. Johansson, Johan & Beke-Somfai, Tamás & Said Stålsmeden, Anna & Kann, Nina. (2016). Ruthenium-Catalyzed Azide Alkyne Cycloaddition Reaction: Scope, Mechanism, and Applications. Chemical Reviews. 116. 10.1021/acs.chemrev.6b00466.).

In some aspects, the present invention relates to a photo-induced generation of the nitrile imine intermediate (e.g., generated from bisaryl-tetrazoles) and its cycloaddition to alkenes (a so-called photo-induced cycloaddition or "photo-click" reaction, e.g., described by de Hoog et al., 2011, supra), which is another exemplary alternative way of conjugating antigens to polymersomes of the present invention. According to this method, ABA block copolymer is methacrylate (MA) terminated or hydroxyl terminated with tetrazole by the photo-induced generation of the nitrile imine intermediate producing ABA polymersomes containing MA-ABA and hydroxyl terminated ABA copolymer, followed by reacting said polymersomes with tetrazole-containing antigen (HRP) under UV-irradiation to produce a pyrazoline moiety.

The covalent bond that conjugates the antigen or the adjuvant to the exterior surface of the polymersome can either be formed between an atom/group of a molecule such an amphiphilic polymer that is part of (present in) of the circumferential membrane of the polymersome. Alternatively, the covalent bond between the antigen or the antigen and the exterior surface of the polymer is formed via a linker moiety that is attached to a molecule that that is part of (present in) of the circumferential membrane of the polymersome. The linker may have any suitable length and can have a length of one main chain atom (for example, if the linker is a simple carbonyl group (C=O) that yields an amide or an ester moiety forming the covalent linkage). An illustrative example for such "one atom/linker moiety with a length of one main atom is the modification of the amphiphilic polymer BD21 by Dess-Martin periodinane carried out in the Example Section to yield BD₂₁-CHO (i.e. a terminal aldehyde group) which is then used to form an amine bond with the selected antigen (hemagglutinin is used as a purely illustrative example antigen in the Experimental Section. Alternatively, the linker moiety may have a length of several hundreds or even more main chain atoms, for example, if a moiety such as polyethylenglycol (PEG) that is commonly used for conjugation (covalent coupling) of polypeptides with a molecule of interest. As a purely illustrative example see distearoylphosphatidylethanolamine [DSPE] polyethylene glycol (DSPE-PEG) conjugates discussed below and used in the Example Section of the present application. The DSPE-PEG(3000) linker moiety used in the Example section has about 65 ethylene oxide (CH₂-CH₂-O)-subunit and thus about 325 main chain atom in the PEG part alone and a total length of about 408 main chain atoms. In line with the above, illustrative embodiments, the linker moiety may comprise 1 to about 550 main chain atoms, 1 to about 500 main chain atoms, 1 to about 450 main chain atoms, 1 to about 350 main chain atoms, 1 to about 300 main chain atoms, 1 to about 250 main chain atoms, 1 to about 200 main chain atoms, 1 to about 150 main chain atoms, 1 to about 100 main chain atoms, 1 to about 50 main chain atoms, 1 to about 30 main chain atoms, 1 to about 20 main chain atoms, 1 to about 15 main chain atoms, or 1 to about 12 main chain atoms, or 1 to about 10 main chain atoms, wherein the main chain atoms are carbon atoms that are optionally replaced by one or more heteroatoms selected from the group consisting of N, O, P and S.

Also in accordance with the above disclosure, the linker moiety may be a peptidic linker or a straight or branched hydrocarbon-based linker. The linker moiety may also be or a co polymer with a different block length. The linker moiety used in the present invention may comprise a membrane anchoring domain which integrates the linker moiety into the membrane of the polymersome. Such a membrane anchoring domain may comprise a lipid such as a phospholipid or a glycolipid. The glycolipid used in membrane anchoring domain may comprise glycophosphatidylinositol (GPI) which has been widely used a membrane anchoring domain (see, for example, International Patent Applications WO 2009/127537 and WO 2014/057128). The phospholipid used in the linker of the present invention may be phosphosphingolipid or a glycerophospholipid. In illustrative examples of such a linker, the phosphosphingolipid may comprise as a membrane anchoring domain distearoylphosphatidylethanolamine [DSPE] conjugate to polyethylene glycol (PEG) (DSPE-PEG). In such conjugates, the DSPE-PEG may comprise any suitable number of ethylene oxide, for example, from 2 to about 500 ethylene oxide units. Illustrative examples include DSPE-PEG(1000), DSPE-PEG(2000) or DSPE-PEG(3000) to name only a few. Alternatively, the phospholipid (phosphosphingolipid or a glycerophospholipid) may comprise cholesterol as membrane anchoring domain. Cholesterol-based membrane anchoring domains are, for instance, described in Achalkumar et al, "Cholesterol-based anchors and tethers for phospholipid bilayers and for model biological membranes", Soft Matter, 2010, 6, 6036-6051. In illustrative embodiments the linker moiety of such a membrane anchoring domain comprises 1 to about 550 main chain atoms, 1 to about 500 main chain atoms, 1 to about 450 main chain atoms, 1 to about 350 main chain atoms, 1 to about 300 main chain atoms, 1 to about 250 main chain atoms, 1 to about 200 main chain atoms, 1 to about 150 main chain atoms, 1 to about 100 main chain atoms, 1 to about 50 main chain atoms, 1 to about 30 main chain atoms, 1 to about 20 main chain atoms, 1 to about 15 main chain atoms, or 1 to about 12 main chain atoms, or 1 to about 10 main chain atoms, wherein the main chain atoms are carbon atoms that are optionally replaced by one or more heteroatoms selected from the group consisting of N, O, P and S.

Any kind of polymersome can be used in the present invention, as long as the polymersome is able to function as a carrier for the associated antigen or adjuvant. The polymersome can for example, be an oxidation-sensitive polymersome as described by Stano et al. "Tunable T cell immunity towards a protein antigen using polymersomes vs. solid-core nanoparticles, Biomaterials 34 (2013): 4339-4346" or in US patent 8,323,696 of Hubbel. Alternatively, the polymersomes may also be insensitive to oxidation. Irrespective of chemical stability (including their possible sensitivity or insensitivity to oxidation), in the present invention, polymersomes are vesicles with a polymeric membrane, which are typically, but not necessarily, formed from the self-assembly of dilute solutions of one or more amphiphilic block copolymers, which can be of different types such as diblock and triblock (A-B-A or A-B-C). Polymersomes of the present invention may also be formed of tetra-block or penta-block copolymers. For tri-block copolymers, the central block is often shielded from the environment by its flanking blocks, while di-block copolymers self-assemble into bilayers, placing two hydrophobic blocks tail-to-tail, much to the same effect. In most cases, the vesicular membrane has an insoluble middle layer and soluble outer layers. The driving force for polymersome formation by self-assembly is considered to be the microphase separation of the insoluble blocks, which tend to associate in order to shield themselves from contact with water. Polymersomes of the present invention possess remarkable properties due to the large molecular weight of the constituent copolymers. Vesicle formation is favored upon an increase in total molecular weight of the block copolymers. As a consequence, diffusion of the (polymeric) amphiphiles in these vesicles is very low compared to vesicles formed by lipids and surfactants. Owing to this less mobility of polymer chains aggregated in vesicle structure, it is possible to obtain stable polymersome morphologies. Unless expressly stated otherwise, the term "polymersome" and "vesicle", as used herein, are taken to be analogous and may be used interchangeably. Importantly, a polymersome of the invention can be formed from either one kind pf block copolymers or from two or more kinds of block copolymers, meaning a polymersome can also be formed from a mixtures of polymersomes and thus can contain two or more block copolymers. In some aspects, the polymersome of the present invention is oxidation-stable.

In some aspects, the present invention relates to a method for eliciting an immune response to a soluble (e.g., solubilized) encapsulated antigen in a subject. The method is suitable for injecting the subject with a composition comprising a polymersome (e.g., carrier or vehicle) having a membrane (e.g., circumferential membrane) of an amphiphilic polymer. The composition comprises a soluble (e.g., solubilized) antigen encapsulated by the membrane (e.g., circumferential membrane) of the amphiphilic polymer of the polymersome of the present invention. The antigen may be one or more of the following: i) a polypeptide; ii) a carbohydrate; iii) a polynucleotide (e.g., said polynucleotide is not an antisense oligonucleotide, preferably said polynucleotide is a DNA or messenger RNA (mRNA) molecule) or a combination of i) and/or ii) and/or iii).

In some further aspects, the present invention relates to polymersomes capable of eliciting a CD8(+) T cell-mediated immune response.

In some aspects, the present invention relates to polymersomes capable of targeting of lymph node-resident macrophages and/or B cells. Exemplary non-limiting targeting mechanisms envisaged by the present invention include: i) delivery of encapsulated antigens (e.g., polypeptides, etc.) to dendritic cells (DCs) for T cell activation (CD4 and/or CD8). Another one is: ii) delivery of whole folded antigens (e.g., proteins, etc.) that will be route to DC and will also trigger a titer (B cells).

In some aspects, the present invention relates to polymersomes encapsulating an antigen selected from a group consisting of: i) a self-antigen, ii) a non-self antigen, iii) a non-self immunogen and iv) a self-immunogen. Accordingly, the products and methods of the present invention are suitable for uses in settings (e.g., clinical settings) of induced tolerance, e.g., when targeting an autoimmune disease.

In some aspects, the present invention relates to polymersomes of the present invention comprising a lipid polymer.

The polymersomes of the present invention can also have co-encapsulated (i.e. encapsulated in addition to the antigen) one or more adjuvants. Examples of adjuvants include synthetic oligodeoxynucleotides (ODNs) containing unmethylated CpG motifs which can trigger cells that express Toll-like receptor 9 (including human plasmacytoid dendritic cells and B cells) to mount an innate immune response characterized by the production of Th1 and proinflammatory cytokines, cytokines such as Interleukin-1, Interleukin-2 or Interleukin-12, keyhole limpet hemocyanin (KLH), serum albumin, bovine thyroglobulin, or soybean trypsin inhibitor, too name only a few illustrative examples.

The polymersomes of the present invention can be of any size as long as the polymersomes are able to elicit an immune response. For example, the polymersomes may have a diameter of greater than 70nm. The diameter of the polymersomes may range from about 100nm to about 1µm, or from about 100nm to about 750nm, or from about 100nm to about 500nm. The diameter of the polymersome may further range from about 125 nm to about 175 nm or, from about 125nm to about 250 nm, from about 140 nm to about 240 nm, from about 150 nm to about 235 nm, from about 170nm to about 230nm, or from about 220nm to about 180nm, or from about 190nm to about 210nm. The diameter of the polymersomes may, for example, about 200nm; about 205 nm or about 210nm. When used as a (first and second) population to elicit an immune response, the population of polymersomes is typically a monodisperse population. The mean diameter of the used population of polymersomes is typically above 70nm, or above 120 nm, or above 125nm, or above 130nm, or above 140 nm, or above 150 nm, or above 160nm, or for above 170 nm, or above 180 nm, or above 190 nm (cf. also Fig. 2 in this respect). The mean diameter of the population of polymersomes may, for example, also in range of the individual polymersomes mentioned above, meaning the mean diameter of the population of polymersomes may be in the range of 100nm to about 1µm, or in the range of about 100nm to about 750nm, or in the range of about 100nm to about 500nm, or in the range from about 125 nm to about 250 nm, from about 140 nm to about 240 nm, from about 150 nm to about 235 nm, from about 170nm to about 230nm, or from about 220nm to about 180nm, or from about 190nm to about 210nm. The mean diameter of the population of polymersomes may, for example, also be about 200nm; about 205 nm or about 210nm. The diameter can, for example, be determined by a dynamic light scattering (DLS) instrument using Z-average (d, nm), a preferred DLS parameter. Z-average size is the intensity weighted harmonic mean particle diameter (cf. Examples 1 and 2). In this context, it is noted that according to US Patent 8,323,696 of Hubbel et al, a collection/population of polymersomes should have a mean diameter of less than 70 nm to be able to elicit immune response. Similarly, Stano et al , supra, 2013, while wanting to use smaller polymersome, used, due to technical constraints, polymersomes having a diameter of 125nm +/- 15nm to elicit an immune response. Thus, it is surprising that a population/collection of polymersomes of the present invention with a mean diameter of, for example, than more 150nm are able to induce both a cellular and a humoral immune response (cf. Example section). Such a population of polymersomes may be in a form suitable for eliciting an immune response, for example, by injection or oral administration.

In some aspects, the present invention relates to compositions of the present invention suitable for intradermal, intraperitoneal, subcutaneous, intravenous, or intramuscular injection, or non-invasive administration of an antigen of the present invention, for example, oral administration or inhaled administration or nasal administration. The composition may include a polymersome (e.g., carrier) of the present invention having a membrane (e.g., circumferential membrane) of an amphiphilic polymer. The composition further includes a soluble (e.g., solubilized) antigen encapsulated by the membrane of the amphiphilic polymer of the polymersome. The compositions of the present invention may be used for therapeutic purposes (for example, treatment of a subject suffering from a disease or for preventing from suffering from a disease, for example, by means of vaccination) or be used in antibody discovery, vaccine discovery, or targeted delivery.

In some aspects, polymersomes of the present invention have hydroxyl groups on their surface. In some further aspects, polymersomes of the present invention do not have hydroxyl groups on their surface.

In the present context, the term "encapsulated" means enclosed by a membrane (e.g., membrane of the polymersome of the present invention, e.g., embodied inside the lumen of said polymersome). With reference to an antigen the term "encapsulated" further means that said antigen is neither integrated into- nor covalently bound to- nor conjugated to said membrane (e.g., of a polymersome of the present invention). With reference to compartmentalization of the vesicular structure of polymersome as described herein the term "encapsulated" means that the inner vesicle is completely contained inside the outer vesicle and is surrounded by the vesicular membrane of the outer vesicle. The confined space surrounded by the vesicular membrane of the outer vesicle forms one compartment. The confined space surrounded by the vesicular membrane of the inner vesicle forms another compartment.

In the present context, the term "sole administration" may refer to a kind of administration characterized in that no antigen is administered in combination (e.g., co-administered) with one or more adjuvants of the present invention (e.g., either simultaneously or at a different time point (e.g., prior and/or post). In preferred embodiments sole administration of one or more adjuvants of the present invention elicits an innate immune response, e.g., comprising production and/or secretion of interleukin-6 (IL-6).

In the present context, the term "adjuvant" as used herein may refer to a compound or substance capable of inducing, enhancing or improving an immune response with or without an antigen. Adjuvants of the present invention further include immunostimulants capable of inducing the immune response (e.g., innate immune response) without an antigen. Exemplary adjuvants of the present invention include but are not limited to compound or substance capable of inducing, enhancing or improving an immune response in the presence of an antigen.

In the present context, the term "innate immune response" as used herein may refer to an immune response characterized by the production of Th1 and proinflammatory cytokines, cytokines such as Interleukin-1, Interleukin-2, Interleukin-6 or Interleukin-12, keyhole limpet hemocyanin (KLH), serum albumin, bovine thyroglobulin, or soybean trypsin inhibitor, too name only a few illustrative examples.

In the present context, the term "antigen" means any substance that may be specifically bound by components of the immune system. Only antigens that are capable of eliciting (or evoking or inducing) an immune response are considered immunogenic and are called "immunogens". Exemplary non-limiting antigens are polypeptides derived from a soluble portion of proteins, hydrophobic polypeptides rendered soluble for encapsulation as well as aggregated polypeptides that are soluble as aggregates. The antigen may originate from within the body ("self-antigen") or from the external environment ("non-self").

Membrane proteins form a class of antigens that typically produce a low immune response level. Of specific interest, soluble (e.g., solubilized) membrane proteins (MPs) and membrane-associated peptides (MAPs) and fragments (i.e., portions) thereof (e.g., the antigens mentioned herein) are encapsulated by a polymersome, which may allow them to be folded in a physiologically relevant manner. This greatly boosts the immunogenicity of such antigens so that when compared to free antigens, a smaller amount of the corresponding antigen can be used to produce the same level of the immune response. Furthermore, the larger size of the polymersomes (compared to free membrane proteins) allows them to be detected by the immune system more easily.

In the present context, the term "B16 peptide" refers to any neoantigen polypeptide derived from the spontaneous C57BL/6-derived B16 melanoma model (e.g., melanoma B16-F10 mouse model). Non-limiting examples thereof include the peptides of SEQ ID NO: 9, 10 and 11.

In the present context, the term "MC38 peptide" refers to any neoantigen polypeptide derived from the colon cancer MC38 mouse model. Non-limiting examples thereof include the peptides of SEQ ID NO: 1, 2 and 3.

In the present context, the term "Influenza hemagglutinin (HA)" refers to a glycoprotein found on the surface of influenza viruses. HA has at least 18 different antigens, which are all within the scope of the present invention. These subtypes are named H1 through H18. Non-limiting examples of "Influenza hemagglutinin (HA)" subtype H1 include the polypeptides of SEQ ID NOs: 5, 6, 7 and 8.

In the present context, the term "Swine Influenza hemagglutinin (HA)" refers to a glycoprotein found on the surface of swine influenza viruses, which is a family of influenza viruses endemic in pigs. Non-limiting examples of "Swine Influenza hemagglutinin (HA)" include subtype H1 of SEQ ID NO: 6.

In the present context, the term "coronavirus" refers to a virus of the subfamily *Coronaviridae,* which is a family of enveloped, positive-sense, single stranded RNA viruses. Coronaviruses may cause diseases in mammals and birds. There are four genera within this subfamily, Alphacoronavirus, Betacoronavirus, Gammacoronavirus, and Deltacoronavirus. In humans, coronaviruses may cause respiratory tract infections that can be mild, and others that can be lethal, such as SARS, MERS, and COVID-19. Human pathogenic coronaviruses commonly belong to the genera of Alphacoronaviruses or Betacoronaviruses. Viruses that belong to genus Alphacoronavirus are e.g. PEDv, transmissible gastroenteritis virus (TGEV), Feline coronavirus (FCoV), including Feline enteric coronavirus (FECV) and Feline infectious peritonitis virus (FIPV), Canine coronavirus (CCoV), or the human-pathogenic coronaviruses Human coronavirus 229E (HCoV-229E) and Human coronavirus NL63 (HCoV-NL63). Within the genus Betacoronavirus, the subgennera Sarbecovirus and Merbecovirus are most relevant in the context of the present disclosure, which include the species SARS-CoV-1, SARS-CoV-2, and MERS-CoV. Other human-pathogenic Betacoronaviruses are Human coronavirus OC43 (HCoV-OC43) Human coronavirus HKU1 (HCoV-HKU1). An overview over human-pathogenic coronaviruses is given by Corman VM, Muth D, Niemeyer D, Drosten C., Hosts and Sources of Endemic Human Coronaviruses. Adv Virus Res. 2018;100:163-188.

In the present context, the term "SPIKE protein" relates to a glycoprotein that is present on the surface of a viral capsid or viral envelope. SPIKE proteins bind to certain receptors on the host cell and are thus important for both host specificity and viral infectivity.

In the present context, the term "PEDv S Protein" refers to SPIKE glycoprotein present on the surface of Porcine epidemic diarrhea virus (PEDV), which is a family of coronavirus in pigs. Non-limiting examples of soluble "PEDv S Protein" as may be used in the present invention include the entire soluble fragment consisting of the S1 and S2 region having the amino acid sequence of SEQ ID NO: 12, the soluble fragment of the S1 region of SEQ ID NO: 13, or the soluble fragment of the S2 region of SEQ ID NO: 14, of the Porcine Epidemic Diarrhea virus (PEDv) Spike protein (S Protein) (UniProtKB Accession number: V5TA78). It is of course also possible to use shorter fragments of the entire soluble fragment of the S1 and the S2 region or of either of the S1 or S2 regions alone (cf. Fig. 12 in this respect) It is of course also possible to use in polymersomes of the present invention a fragment that contains part of the S1 and part of the S2, say for example, amino acids 500 to 939 of the deposited sequence of the Spike protein. It is also noted here that a polymersome of the present invention may have encapsulated one or more different soluble fragments of the Spike protein, for example, the S1 region, the S2 region and/or the entire S1 and S2 region. In illustrative embodiments of a polymersomes of the invention, it has encapsulated therein one type of soluble fragments (for example, only the S1 region), two different types of soluble fragments (for example, the S1 and S2 region), three different types of soluble fragments (the S1 region, the S2 region and the entire soluble fragment of S1 and S2 of SEQ ID NO: 12 (amino acid residues 19 to 1327)) or even four different types of fragments (for example, the S1 region, the S2 region, the entire soluble fragment of S1 and S2 of SEQ ID NO: 12 (amino acid residues 19 to 1327) and as fourth type, the above- mentioned fragment that contains part of the S1 and part of the S2, say for example, amino acids 500 to 939 of the Spike protein sequence). It is also noted here that a polymersome of the present invention having encapsulated one or more different soluble fragments of the Spike protein are used in one preferred embodiment as oral vaccine against the Porcine Epidemic Diarrhea virus.

In the present context, the term "MERS-CoV S Protein" or "MERS-CoV SPIKE Protein" refers to SPIKE glycoprotein present on the surface of Middle East respiratory syndrome-related coronavirus (MERS-CoV), which is a human-pathogenic coronavirus. A MERS-CoV Spike protein of the disclosure has the sequence set forth in UniProtKB Accession number: K0BRG7 version 40 of 26 February 2020 (GenBank Accession No. AFS88936, version AFS88936.1) or SEQ ID NO: 42. A non-limiting example of soluble "MERS-CoV S Protein" as may be used in the present invention includes the entire soluble fragment of the S1 and S2 region of the the MERS-CoV Spike protein (S Protein), which may correspond to positions 1 to 1297 of the MERS-CoV Spike protein or has the amino acid sequence set forth in SEQ ID NO: 43. A non-limiting example of soluble "MERS-CoV S Protein" as may be used in the present invention also includes the S1 region, which corresponds to positions 18 to 725 of the MERS-CoV Spike protein (S Protein) or has the amino acid sequence of SEQ ID NO: 44. A non-limiting example of soluble "MERS-CoV S Protein" as may be used in the present invention also includes the soluble fragment of the S2 region, which may correspond to positions 726 to 1296 of the MERS-CoV Spike protein (S Protein) or has the amino acid sequence of SEQ ID NO: 45. It is of course also possible to use shorter fragments of the entire soluble fragment of the S1 and the S2 region or of either of the S1 or S2 regions alone, for example a fragment may include a Receptor Binding Domain (RBD), which corresponds to positions 377-588 of the MERS-CoV Spike protein or has the amino acid sequence of SEQ ID NO: 46. It is also noted here that a polymersome of the present invention may have encapsulated one or more different soluble fragments of the Spike protein, for example, the S1 region, the S2 region or the soluble fragment thereof, the entire soluble fragment of the S1 and S2 regions, and/or an RBD. In illustrative embodiments of a polymersomes of the invention, it has encapsulated therein one type of soluble fragments (for example, only the entire soluble fragment of the S1 and S2 regions), two different types of soluble fragments (for example, the entire soluble fragment of the S1 and S2 regions and either S1 region or soluble fragment of the S2 region), three different types of soluble fragments (the S1 region, a soluble fragment of the S2 region and the entire soluble fragment of S1 and S2 of SEQ ID NO: 42 (amino acid residues 1 to 1297) or even four different types of fragments (for example, the S1 region, a soluble fragment of the S2 region, the entire soluble fragment of S1 and S2 of SEQ ID NO: 42 (amino acid residues 1 to 1297) and as fourth type, an the RBD). In a preferred embodiment, a polymersome of the invention has encapsulated therein a soluble fragment that comprises, essentially consists of, or consists of the S1 region corresponding to amino acid residues 18 to 725 of the full-length MERS-CoV SPIKE Protein. In a preferred embodiment, a polymersome of the invention has encapsulated therein a soluble fragment that comprises, essentially consists of, or consists of the soluble fragment of the S2 region corresponding to amino acid residues 726 to 1296 of the full length MERS-CoV SPIKE Protein. In a preferred embodiment, a polymersome of the invention has encapsulated therein a soluble fragment that comprises, essentially consists of, or consists of the S1 and the S2 region corresponding to amino acid residues 1 to 1297 of the full length MERS-CoV SPIKE Protein. In a preferred embodiment, a polymersome of the invention has encapsulated therein a fragment that comprises, essentially consists of, or consists of the S1 and the S2 region corresponding to amino acid residues 1 to 1327 of the full length MERS-CoV SPIKE Protein. In this context, "essentially consist of" means that the N terminal and/or C terminal endpoints of the fragment may vary to a limited extent, such as up to 25 amino acid positions, such as up to 20 amino acid positions, such as up to 15 amino acid positions, up to 10 amino acid positions, up to 5 amino acid positions, up to 4 amino acid positions, up to 3 amino acid positions, up to 2 amino acid positions, or up to 1 amino acid position. As an illustrative example, a fragment that essentially consists of amino acids 726 to 1296 of the full length MERS-CoV SPIKE Protein may consists of positions 716 to 1296, 736 to 1296, 726 to 1286, or 726 to 1306, 716 to 1286, 736 to 1286, 736 to 1306, or 716 to 1306 of the full length MERS-CoV SPIKE Protein.

A MERS-CoV Spike protein of the disclosure may also comprise variants of the sequences mentioned above, which include natural variants of other isolates of the MERS-CoV as well as artificial modification, which can be introduced into the sequence of the MERS-CoV S Protein. As an illustrative example, mutations can be introduced to change the formation of the expressed protein. For this purpose, the furin cleavage site located from position 754 to 757 of SEQ ID NO: 42 may be mutated. Reduction in post expression cleavage may be achieved by reducing the basic nature of this amino acid sequence. For example, the residues Arginine 754 and/or 757 may be mutated to less basic amino acids, such as Glycine (position numbering corresponding to the amino acid sequence set forth in SEQ ID NO: 42), or other less basic amino acids. A furin cleavage site having the native sequence of RSVR (SEQ ID NO: 58) may thus be mutated to the sequence of GSVG (SEQ ID NO: 59).Further modifications may include the addition of a trimerization domain, preferably to the C-terminus of the protein, which may help increasing the native fold of the S1 and/or S2 domains. Such trimerization domains can include a foldon domain (e.g. SEQ ID NO: 54), a GCN4 based trimerization domain (such as SEQ ID NO: 55 or 56), or other motifs that are well known to the person skilled in the art. Further, secretion leader sequences may be added to the N terminus of proteins which may improve production and/or downstream processing, such as isolation and purification. An illustrative example for such a leader sequence is the honey bee melittin leader sequence (SEQ ID NO: 57). Further useful leader sequences are well known to the person skilled in the art. Accordingly, a soluble fragment of a spike protein of the present disclosure also includes highly identical variants of particular sequences of soluble fragments of a spike protein that are explicitly or implicitly disclosed herein. Such as variants having at least about 95%, at least about 96%, at least about 97%, at least about 98%, or at least about 99% sequence identity to a soluble fragment of a spike protein of the disclosure, in particular a soluble fragment of a MERS-CoV S protein of the disclosure. As an illustrative example, a soluble fragment of a S fragment of the disclosure may comprise, essentially consists of or consists of a sequence that has at least about 95%, at least about 96%, at least about 97%, at least about 98%, or at least about 99% sequence identity to a sequence selected from the group consisting of SEQ ID NO: 43-46.

Alternatively or additionally, a polymersome of the present disclosure may have encapsulated one or more nucleic acids, such as mRNA, self-amplifying mRNA, DNA encoding one or more MERS-CoV Spike protein or a soluble fragment thereof according to the disclosure.

It is also noted here that a polymersome of the present invention having encapsulated one or more different soluble fragments of the MERS-CoV Spike protein and/or nucleic acids encoding the same or a full-length MERS-CoV Spike protein are used in one preferred embodiment as vaccine against a human disease, in particular an infection by a human-pathogenic coronavirus, in particular Middle East respiratory syndrome (MERS). Thus, a polymersome of the present invention having encapsulated one or more different soluble fragments of the MERS-CoV Spike protein and/or nucleic acids encoding the same or a full-length MERS-CoV Spike protein may be used in the treatment, including prevention, of fever, cough, expectoration, shortness of breath, pneumonia, and/or acute respiratory distress syndrome (ARDS).

In one preferred embodiment, the polymersome having encapsulated one or more different soluble fragments of the MERS-CoV Spike protein and/or nucleic acids encoding the same or a full-length MERS-CoV Spike protein is administered intramuscularly. In one preferred embodiment, the polymersome having encapsulated one or more different soluble fragments of the MERS-CoV Spike protein and/or nucleic acids encoding the same or a full-length MERS-CoV Spike protein is administered intranasally. In one preferred embodiment, the polymersome having encapsulated one or more different soluble fragments of the MERS-CoV Spike protein and/or nucleic acids encoding the same or a full-length MERS-CoV Spike protein is administered by inhalation.

In the present context, the term "SARS-CoV-2 S Protein" or "SARS-CoV-2 SPIKE Protein" refers to SPIKE glycoprotein present on the surface of severe acute respiratory syndrome coronavirus 2 (SARS-CoV-2), which is a human-pathogenic coronavirus. A SARS-CoV-2 Spike protein of the disclosure has the sequence set forth in UniProtKB Accession number: P0DTC2 version 1 of 22 April 2020 (GenBank Accession Number MN908947, version MN908947.3) or SEQ ID NO: 19. A non-limiting example of soluble "SARS-CoV-2 S Protein" as may be used in the present invention includes the entire soluble fragment consisting of the S1 and S2 region of the the SARS-CoV-2 Spike protein (S Protein), which corresponds to positions 16 to 1213 or 14 to 1204 or 19 to 1204 of the SARS-CoV-2 Spike protein or has the amino acid sequence set forth in SEQ ID NO: 34 or SEQ ID NO: 35 or SEQ ID NO: 65. A non-limiting example of soluble "SARS-CoV-2 S Protein" as may be used in the present invention also includes the S1 region, which corresponds to positions 16 to 685 of the SARS-CoV-2 Spike protein (S Protein) or has the amino acid sequence of SEQ ID NO: 37. A non-limiting example of soluble "SARS-CoV-2 S Protein" as may be used in the present invention also includes the S2 region, which corresponds to positions 686 to 1213 or 646 to 1204 of the SARS-CoV-2 Spike protein (S Protein) or has the amino acid sequence of SEQ ID NO: 38 or 39. It is of course also possible to use shorter fragments of the entire soluble fragment of the S1 and the S2 region or of either of the S1 or S2 regions alone, for example the amino acid sequence of 318-524 of SARS-CoV-2 protein as the Receptor Binding domains (SEQ ID NO: 41, cf. Fig. 23 A in this respect). As an illustrative example, a shorter fragment of S2 region may comprise, essentially consist, or consist of amino acids corresponding to positions 686 to 1204 of SEQ ID NO: 19. In an illustrative example a soluble fragment of a Spike protein may comprise, essentially consist, or consist of amino acids corresponding to positions 646 to 1204 of SEQ ID NO: 19. In an illustrative example, a soluble fragment of a Spike protein may comprise, essentially consist or consist of the sequence set forth in any one of SEQ ID NO: 34-36 and 65. It is also noted here that a polymersome of the present invention may have encapsulated one or more different soluble fragments of the Spike protein, for example, the S1 region or a fragment thereof, the S2 region or a fragment thereof and/or the entire S1 and S2 region or a fragment thereof comprising parts of the S1 region and parts of the S2 region. In illustrative embodiments of a polymersomes of the invention, it has encapsulated therein one type of soluble fragments (for example, only the S1 region or a fragment thereof), two different types of soluble fragments (for example, the S1 and S2 region or fragments of the S1 and/or the S2 region), three different types of soluble fragments (the S1 region or fragment thereof, the S2 region or fragment thereof and the entire soluble fragment of S1 and S2 of SEQ ID NO: 19 or even four different types of fragments (for example, the S1 region or fragment thereof, the S2 region or fragment thereof, the entire soluble fragment of S1 and S2 of SEQ ID NO: 19 or a fragment thereof comprising parts of the S1 region and parts of the S2 region, and as fourth type, the above- mentioned fragment that contains part of the S1 and part of the S2, say for example, amino acids 14 to 1204 of the Spike protein sequence).

Several variants of the SARS-CoV-2 S Protein are known in the art, such as GeneBank Accession No. QII57278.1 (SEQ ID NO: 20), GeneBank Accession No. YP_009724390.1 (SEQ ID NO: 21), GeneBank Accession No. QIO04367.1(SEQ ID NO: 22), GeneBank Accession No. QHU79173.2 (SEQ ID NO: 23), GeneBank Accession No. QII87830.1 (SEQ ID NO: 24), GeneBank Accession No. QIA98583.1 (SEQ ID NO: 25), GeneBank Accession No. QIA20044.1 (SEQ ID NO: 26), GeneBank Accession No. QIK50427.1 (SEQ ID NO: 27), GeneBank Accession No. QHR84449.1 (SEQ ID NO: 28), GeneBank Accession No. QIQ08810.1 (SEQ ID NO: 29), GeneBank Accession No. QIJ96493.1 (SEQ ID NO: 30), GeneBank Accession No. QIC53204.1 (SEQ ID NO: 31), GeneBank Accession No. QHZ00379.1 (SEQ ID NO: 32), and GeneBank Accession No. QHS34546.1 (SEQ ID NO: 33). Compared to SEQ ID NO: 19, mutations at sequence positions corresponding to positions 28, 49, 74, 145, 157, 181, 221, 307, 408, 528, 614, 655, 797, 930 can be found in these variants. Further modifications can be introduced into the sequence of the SARS-CoV-2 S Protein. As an illustrative example, mutations can be introduced to change the formation of the expressed protein. For this purpose, the furin cleavage site located from positions 679 to 685 of SEQ ID NO: 19 may be mutated. Reduction in post expression cleavage may be achieved by reducing the basic nature of this amino acid sequence. For example, the residues Pro 681, Arg 682, and/or Arg 683 may be mutated to less basic amino acids, such as Pro 681 -> Asn, Arg 682 -> Gln, and/or Arg 683 -> Ser (position numbering corresponding to the amino acid sequence set forth in SEQ ID NO: 19), or other less basic amino acids. A furin cleavage site having the native sequence of NSPRRAR (SEQ ID NO: 52) may thus be mutated to the sequence of NSNQSAR (SEQ ID NO: 53). An illustrative example for a soluble fragment of a SARS-CoV-2 spike protein having a mutated furin cleavage site is shown in SEQ ID NO: 65. An illustrative example for a SARS-CoV-2 spike protein having a mutated furin cleavage site is shown in SEQ ID NO: 66. Further modifications may include the addition of a trimerization domain, preferably to the C- terminus of the protein, which may help increasing the native fold of the S1 and/or S2 domains. Such trimerization domains can include a foldon domain (GYIPEAPRDG QAYVRKDGEW VLLSTFL, SEQ ID NO: 54, as e.g. described in Güthe et al.,J. Mol. Biol. (2004) 337, 905-915), a GCN4 based trimerization domain including a immune-silenced variant thereof (such as GGGTGGGGTG RMKQIEDKIEE ILSKIYHIEN EIARIKKLIG ERGGR, SEQ ID NO: 55, or GGGTGGNGTG RMKQIEDKIE NITSKIYNITN EIARIKKLIG NRTGGR, SEQ ID NO: 56, as described in Sliepen et al. J. Biol. Chem. (2015) 290(12):7436-7442), or other motifs that are well known to the person skilled in the art. Further, secretion leader sequences may be added to the N terminus of proteins which may improve production and/or downstream processing, such as isolation and purification. An illustrative example for such a leader sequence is the honey bee melittin leader sequence (MKFLVNVALV FMVVYISYIY A, SEQ ID NO: 57). Further useful leader sequences are well known to the person skilled in the art. Accordingly, a soluble fragment of a spike protein of the present disclosure also includes highly identical variants of particular sequences of soluble fragments of a spike protein that are explicitly or implicitly disclosed herein. Such as variants having at least about 95%, at least about 96%, at least about 97%, at least about 98%, or at least about 99% sequence identity to a soluble fragment of a spike protein of the disclosure, in particular a soluble fragment of a SARS CoV-2 S protein of the disclosure. As an illustrative example, a soluble fragment of a S fragment of the disclosure may comprise, essentially consists of or consists of a sequence that has at least about 95%, at least about 96%, at least about 97%, at least about 98%, or at least about 99% sequence identity to a sequence selected from the group consisting of: a sequence corresponding to positions 16 to 1213, 16 to 685, 686 to 1213, 686 to 1204, 646 to 1204, or 14 to 1204 of SEQ ID NO: 19 (the SARS-CoV-2 Spike protein). As another illustrative example, a soluble fragment of a S fragment of the disclosure may have at least about 95%, at least about 96%, at least about 97%, at least about 98%, or at least about 99% sequence identity to a sequence selected from the group consisting of: SEQ ID NO: 34-41 and 65.

In a preferred embodiment, a polymersome of the invention has encapsulated therein a soluble fragment that comprises, essentially consists of, or consists of the S1 region corresponding to amino acid residues 16 to 685 of the full length SARS-CoV-2 SPIKE Protein set forth in SEQ ID NO: 19 or has the amino acid sequence of SEQ ID NO: 37. In a preferred embodiment, a polymersome of the invention has encapsulated therein a soluble fragment that comprises, essentially consists of, or consists of the S2 region corresponding to amino acid residues 686 to 1213 of the full length SARS-CoV-2 SPIKE Protein set forth in SEQ ID NO: 19 or has the amino acid sequence of SEQ ID NO: 38. In a preferred embodiment, a polymersome of the invention has encapsulated therein a soluble fragment that comprises, essentially consists of, or consists of the S1 and the S2 region corresponding to amino acid residues 16 to 1213 of the full length SARS-CoV-2 SPIKE Protein set forth in SEQ ID NO: 19 or has the amino acid sequence of SEQ ID NO: 34. In a preferred embodiment, a polymersome of the invention has encapsulated therein a soluble fragment that comprises, essentially consists of, or consists of amino acids corresponding to amino acid residues 686 to 1204 of the full length SARS-CoV-2 SPIKE Protein set forth in SEQ ID NO: 19. In a preferred embodiment, a polymersome of the invention has encapsulated therein a soluble fragment that comprises, essentially consists of, or consists of amino acids corresponding to amino acid residues 646 to 1204 of the full length SARS-CoV-2 SPIKE Protein set forth in SEQ ID NO: 19 or has the amino acid sequence of SEQ ID NO: 39. In a preferred embodiment, a polymersome of the invention has encapsulated therein a soluble fragment that comprises, essentially consists of, or consists of amino acids corresponding to amino acid residues 14 to 1204 of the full length SARS-CoV-2 SPIKE Protein set forth in SEQ ID NO: 19 or has the amino acid sequence of SEQ ID NO: 35. In a preferred embodiment, a polymersome of the invention has encapsulated therein a soluble fragment that comprises, essentially consists of, or consists of amino acids corresponding to amino acid residues 19 to 1204 of the full length SARS-CoV-2 SPIKE Protein set forth in SEQ ID NO: 19 or has the amino acid sequence of SEQ ID NO: 65. In a preferred embodiment, a polymersome of the invention has encapsulated therein a soluble fragment that comprises, essentially consists of, or consists of a sequence that has at least about 95%, at least about 96%, at least about 97%, at least about 98%, or at least about 99% sequence identity to a sequence selected from the group consisting of: a sequence corresponding to positions 16 to 1213, 16 to 685, 686 to 1213, 686 to 1204, 646 to 1204, 14 to 1204, or 19 to 1204 of SEQ ID NO: 19 (the SARS-CoV-2 Spike protein). In a preferred embodiment, a polymersome of the invention has encapsulated therein a soluble fragment that comprises, essentially consists of, or consists of a sequence that has at least about 95%, at least about 96%, at least about 97%, at least about 98%, or at least about 99% sequence identity to a sequence selected from the group consisting of: SEQ ID NO: 36, 40 and/or 65. In this context, "essentially consist of" means that the N terminal and/or C terminal endpoints of the fragment may vary to a limited extent, such as up to 25 amino acid positions, such as up to 20 amino acid positions, such as up to 15 amino acid positions, up to 10 amino acid positions, up to 5 amino acid positions, up to 4 amino acid positions, up to 3 amino acid positions, up to 2 amino acid positions, or up to 1 amino acid position. As an illustrative example, a fragment that essentially consists of amino acids 646 to 1204 of the full length SARS-CoV-2 SPIKE Protein may consists of positions 641 to 1204, 651 to 1204, 646 to 1209, or 646 to 1199, 641 to 1209, or 651 to 1199 of the full length SARS-CoV-2 SPIKE Protein.

Alternatively or additionally, a polymersome of the present disclosure may have encapsulated one or more nucleic acids, such as mRNA, self-amplifying mRNA, DNA encoding one or more SARS-CoV-2 Spike protein or a soluble fragment thereof according to the disclosure.

It is also noted here that a polymersome of the present invention having encapsulated one or more different soluble fragments of the SARS-CoV-2 Spike protein and/or nucleic acids encoding the same are used in one preferred embodiment as vaccine against a human disease, in particular an infection by a human-pathogenic coronavirus, Coronavirus disease 2019 (COVID-19). Thus, a polymersome of the present invention having encapsulated one or more different soluble fragments of the SARS-CoV-2 Spike protein and/or nucleic acids encoding the same may be used in the treatment, including prevention, of fever, cough, shortness of breath, pneumonia, organ failure, acute respiratory distress syndrome (ARDS), fatigue, muscle pain, diarrhea, sore throat, loss of smell and/or abdominal pain.

In one preferred embodiment, the polymersome having encapsulated one or more different soluble fragments of the SARS-CoV-2 Spike protein and/or nucleic acids encoding the same or a full-length SARS-CoV-2 Spike protein is administered intramuscularly. In one preferred embodiment, the polymersome having encapsulated one or more different soluble fragments of the SARS-CoV-2 Spike protein and/or nucleic acids encoding the same or a full-length SARS-CoV-2 Spike protein is administered intranasally. In one preferred embodiment, the polymersome having encapsulated one or more different soluble fragments of the SARS-CoV-2 Spike protein and/or nucleic acids encoding the same or a full-length SARS-CoV-2 Spike protein is administered by inhalation.

In the present context, the term "SARS-CoV-1 S Protein" or "SARS-CoV-1 Spike protein" refers to Spike glycoprotein present on the surface of Severe acute respiratory syndrome coronavirus (SARS-CoV or SARS-CoV-1), which is a human-pathogenic coronavirus. A SARS-CoV-1 Spike protein of the disclosure has the sequence set forth in UniProtKB Accession number: P59594 version 134 of 11 December 2019 or SEQ ID NO: 48. A non-limiting example of soluble "SARS-CoV-1 S Protein" as may be used in the present invention includes the entire soluble fragment of the S1 and S2 region of the the SARS-CoV-1 Spike protein (S Protein), which may correspond to positions 14 to 1195 of the SARS-CoV-1 Spike protein or has the amino acid sequence set forth in SEQ ID NO: 48. A non-limiting example of soluble "SARS-CoV-1 S Protein" as may be used in the present invention also includes the S1 region, which corresponds to positions 14 to 667 of the SARS-CoV-1 Spike protein (S Protein) or has the amino acid sequence of SEQ ID NO: 49. A non-limiting example of soluble "SARS-CoV-1 S Protein" as may be used in the present invention also includes the soluble fragment of the S2 region, which may correspond to positions 668 to 1198 of the SARS-CoV-1 Spike protein (S Protein) or has the amino acid sequence of SEQ ID NO: 50. It is of course also possible to use shorter fragments of the entire soluble fragment of the S1 and the S2 region or of either of the S1 or S2 regions alone, for example a fragment may include a Receptor Binding Domain (RBD), which corresponds to positions 306-527 of the SARS-CoV-1 Spike protein or has the amino acid sequence of SEQ ID NO: 51. It is also noted here that a polymersome of the present invention may have encapsulated one or more different soluble fragments of the Spike protein, for example, the S1 region, the S2 region or the soluble fragment thereof, the entire soluble fragment of the S1 and S2 regions, and/or an RBD. In illustrative embodiments of a polymersomes of the invention, it has encapsulated therein one type of soluble fragments (for example, only the entire soluble fragment of the S1 and S2 regions), two different types of soluble fragments (for example, the entire soluble fragment of the S1 and S2 regions and either S1 region or a soluble fragment of the S2 region), three different types of soluble fragments (the S1 region, a soluble fragment of the S2 region and the entire soluble fragment of S1 and S2 of SEQ ID NO: 47 (amino acid residues 14 to 1195)) or even four different types of fragments (for example, the S1 region, a soluble fragment of the S2 region, the entire soluble fragment of S1 and S2 of SEQ ID NO: 47 (amino acid residues 14 to 1195) and as fourth type, an RBD). In a preferred embodiment, a polymersome of the invention has encapsulated therein a soluble fragment that comprises, essentially consists of, or consists of the S1 region corresponding to amino acid residues 14 to 667 of the full-length SARS-CoV-1 Spike protein. In a preferred embodiment, a polymersome of the invention has encapsulated therein a soluble fragment that comprises, essentially consists of, or consists of the soluble fragment of the S2 region corresponding to amino acid residues 668 to 1195 of the full length SARS-CoV-1 Spike protein. In a preferred embodiment, a polymersome of the invention has encapsulated therein a soluble fragment that comprises, essentially consists of, or consists of the S1 and the S2 region corresponding to amino acid residues 14 to 1195 of the full length SARS-CoV-1 Spike protein. In a preferred embodiment, a polymersome of the invention has encapsulated therein a fragment that comprises, essentially consists of, or consists of the S1 and the S2 region corresponding to amino acid residues 14 to 1255 of the full length SARS-CoV-1 Spike protein. In this context, "essentially consist of" means that the N terminal and/or C terminal endpoints of the fragment may vary to a limited extent, such as up to 25 amino acid positions, such as up to 20 amino acid positions, such as up to 15 amino acid positions, up to 10 amino acid positions, up to 5 amino acid positions, up to 4 amino acid positions, up to 3 amino acid positions, up to 2 amino acid positions, or up to 1 amino acid position.

A SARS-CoV-1 Spike protein of the disclosure may also comprise variants of the sequences mentioned above, which include natural variants of other isolates of SARS-CoV-1 as well as artificial modification(s), which can be introduced into the sequence of the SARS-CoV-1 S Protein. As an illustrative example, mutations can be introduced to change the formation of the expressed protein. For this purpose, the furin cleavage site located from position 761 to 767 of SEQ ID NO: 47 may be mutated. Reduction in post expression cleavage may be achieved by reducing the basic nature of this amino acid sequence. For example, the residues Arg 764 and/or Arg 767 may be mutated to less basic amino acids, such as Gly (position numbering corresponding to the amino acid sequence set forth in SEQ ID NO: 47), or other less basic amino acids. A furin cleavage site having the native sequence of EQDRNTR (SEQ ID NO: 60) may thus be mutated to the sequence of EQDGNTG (SEQ ID NO: 61).Further modifications may include the addition of a trimerization domain, preferably to the C- terminus of the protein, which may help increasing the native fold of the S1 and/or S2 domains. Such trimerization domains can include a foldon domain (e.g. SEQ ID NO: 54), a GCN4 based trimerization domain (such as SEQ ID NO: 55 or 56), or other motifs that are well known to the person skilled in the art. Further, secretion leader sequences may be added to the N terminus of proteins which may improve production and/or downstream processing, such as isolation and purification. An illustrative example for such a leader sequence is the honey bee melittin leader sequence (SEQ ID NO: 57). Further useful leader sequences are well known to the person skilled in the art. Accordingly, a soluble fragment of a spike protein of the present disclosure also includes highly identical variants of particular sequences of soluble fragments of a spike protein that are explicitly or implicitly disclosed herein. Such as variants having at least about 95%, at least about 96%, at least about 97%, at least about 98%, or at least about 99% sequence identity to a soluble fragment of a spike protein of the disclosure, in particular a soluble fragment of a SARS-CoV-1 S protein of the disclosure. As an illustrative example, a soluble fragment of a S fragment of the disclosure may comprise, essentially consists of or consists of a sequence that has at least about 95%, at least about 96%, at least about 97%, at least about 98%, or at least about 99% sequence identity to a sequence selected from the group consisting of SEQ ID NO: 48-51.

Alternatively or additionally, a polymersome of the present disclosure may have encapsulated one or more nucleic acids, such as mRNA, self-amplifying mRNA, DNA encoding one or more SARS-CoV-1 Spike protein or a soluble fragment thereof according to the disclosure.

It is also noted here that a polymersome of the present invention having encapsulated one or more different soluble fragments of the SARS-CoV-1 Spike protein and/or nucleic acids encoding the same or a full-length SARS-CoV-1 Spike protein are used in one preferred embodiment as vaccine against a human disease, in particular an infection by a human-pathogenic coronavirus, in particular Severe acute respiratory syndrome (SARS). Thus, a polymersome of the present invention having encapsulated one or more different soluble fragments of the SARS-CoV-1 Spike protein and/or nucleic acids encoding the same or a full-length SARS-CoV-1 Spike protein may be used in the treatment, including prevention, of fever, muscle pain, lethargy, cough, sore throat, shortness of breath, pneumonia, and/or acute respiratory distress syndrome (ARDS).

In one preferred embodiment, the polymersome having encapsulated one or more different soluble fragments of the SARS-CoV-1 Spike protein and/or nucleic acids encoding the same or a full-length SARS-CoV-1 Spike protein is administered intramuscularly. In one preferred embodiment, the polymersome having encapsulated one or more different soluble fragments of the SARS-CoV-1 Spike protein and/or nucleic acids encoding the same or a full-length SARS-CoV-1 Spike protein is administered intranasally. In one preferred embodiment, the polymersome having encapsulated one or more different soluble fragments of the SARS-CoV-1 Spike protein and/or nucleic acids encoding the same or a full-length SARS-CoV-1 Spike protein is administered by inhalation.

In the present context, the term "oxidation-stable" refers to a measure of polymersomes (or the corresponding polymers or membranes) resistance to oxidation, for example, using the method described by Scott et al., 2012, In this method a polymersome with an encapsulated antigen is incubated in a 0.5% solution of hydrogen peroxide and the amount of free (released) antigen can be quantified with UV/fluorescence HPLC. Polymersomes which release a substantial or all of the encapsulated antigen under these oxidizing conditions are considered to be oxidation sensitive. Another method of determining whether a block-copolymer and thus the resulting polymersome is oxidation stable or oxidation-sensitive is described in column 16 of US Patent 8,323,696. According to this method, polymers with functional groups that are oxidation-sensitive will be chemically altered by mild oxidizing agents, with a test for the same being enhanced solubility to 10 % hydrogen peroxide for 20 h in vitro. As, for example, poly(propylene sulfide) (PPS) is an oxidation-sensitive polymer (see, for example, Scott et al 2012, supra and US 8,323,696) PPS can serve as a reference to determine whether a polymer of interest and the respective polymersome of interest is oxidation-sensitive or oxidation stable, If, for example, the same or a higher amount of antigen, or about 90% or more of the amount, or about 80% or more, or about 70% or more, or about 60 % or more is released from polymersomes of interest as it is from a PPS polymersome that has encapsulated therein the same antigen, then the polymersome is considered oxidation sensitive. If about only 0.5% or less, or about only 1.0 % or less, or about 2 % or less, or about 5 % of less, or about 10% or less, or about 20 % or less, or about 30 % or less, or about 40 % or less or about 50 % or less of antigen is released from polymersomes of interest as it is from a PPS polymersome that has encapsulated therein the same antigen, then the polymersome is considered oxidation-stable. Thus, in line with this, PPS polymersomes as described in US Patent 8,323,696 or. PPS-bl-PEG polymersomes, e.g., made from poly(propylene sulfide) (PPS) and poly(ethylene glycol) (PEG) as components as described in Stano et al, are not oxidation-stable polymersomes within the meaning of the present invention. Similarly, PPS30-PEG17 polymersomes are not oxidation-stable polymersomes within the meaning of the present invention. Other non-limiting examples of measuring oxidation stability include measurement of stability in the presence of serum components (e.g., mammalian serum, e.g., human serum components) or stability inside an endosome, for example.

In the present context, the term "reduction-stable" refers to a measure of polymersome resistance to reduction in a reducing environment.

In the present context, the term "serum" refers to blood plasma from which the clotting proteins have been removed.

In the present context, the term "oxidation-independent release" refers to a release of the polymersome content without or essentially without oxidation of the polymers forming the polymersomes.

The term "polypeptide" is equally used herein with the term "protein". Proteins (including fragments thereof, preferably biologically active fragments, and peptides, usually having less than 30 amino acids) comprise one or more amino acids coupled to each other via a covalent peptide bond (resulting in a chain of amino acids). The term "polypeptide" as used herein describes a group of molecules, which, for example, consist of more than 30 amino acids. Polypeptides may further form multimers such as dimers, trimers and higher oligomers, i.e. consisting of more than one polypeptide molecule. Polypeptide molecules forming such dimers, trimers etc. may be identical or non-identical. The corresponding higher order structures of such multimers are, consequently, termed homo- or heterodimers, homo- or heterotrimers etc. An example for a heteromultimer is an antibody molecule, which, in its naturally occurring form, consists of two identical light polypeptide chains and two identical heavy polypeptide chains. The terms "polypeptide" and "protein" also refer to naturally modified polypeptides/proteins wherein the modification is effected e.g. by post-translational modifications like glycosylation, acetylation, phosphorylation and the like. Such modifications are well known in the art.

In the present context, the term "carbohydrates" refers to compounds such as aldoses and ketoses having the stoichiometric formula Cn(H₂O)n (e.g., hence "hydrates of carbon"). The generic term "carbohydrate" includes, but is not limited to, monosaccharides, oligosaccharides and polysaccharides as well as substances derived from monosaccharides by reduction of the carbonyl group (alditols), by oxidation of one or more terminal groups to carboxylic acids, or by replacement of one or more hydroxy group(s) by a hydrogen atom, an amino group, thiol group or similar groups. It also includes derivatives of these compounds.

In the present context, the term "polynucleotide" (also "nucleic acid", which can be used interchangeably with the term "polynucleotide") refers to macromolecules made up of nucleotide units which e.g., can be hydrolysable into certain pyrimidine or purine bases (usually adenine, cytosine, guanine, thymine, uracil), d-ribose or 2-deoxy-d-ribose and phosphoric acid. Non-limiting examples of "polynucleotide" include DNA molecules (e.g. cDNA or genomic DNA), RNA (mRNA), combinations thereof or hybrid molecules comprised of DNA and RNA. The nucleic acids can be double- or single-stranded and may contain double- and single-stranded fragments at the same time. Most preferred are double stranded DNA molecules and mRNA molecules.

In the present context, the term "antisense oligonucleotide" refers to a nucleic acid polymer, at least a portion of which is complementary to a nucleic acid which is present in a normal cell or in an affected cell. Exemplary "antisense oligonucleotide" include antisense RNA, siRNA, RNAi.

In the present context, the term "CD8(+) T cell-mediated immune response" refers to the immune response mediated by cytotoxic T cells (also known as TC, cytotoxic T lymphocyte, CTL, T-killer cells, cytolytic T cells, CD8(+) T-cells or killer T cells). Example of cytotoxic T cells include, but are not limited to antigen-specific effector CD8(+) T cells. In order for the T-cell receptors (TCR) to bind to the class I MHC molecule, the former must be accompanied by a glycoprotein called CD8, which binds to the constant portion of the class I MHC molecule. Therefore, these T cells are called CD8(+) T cells. Once activated, the TC cell undergoes "clonal expansion" with the help of the cytokine Interleukin-2 (IL-2), which is a growth and differentiation factor for T cells. This increases the number of cells specific for the target antigen that can then travel throughout the body in search of antigen-positive somatic cells.

In the present context, the term "clonal expansion of antigen-specific CD8(+) T cells" refers to an increase in the number of CD8(+) T cells specific for the target antigen.

In the present context, the term "cellular immune response" refers to an immune response that does not involve antibodies, but rather involves the activation of phagocytes, antigen-specific cytotoxic T-lymphocytes, and the release of various cytokines in response to an antigen.

In the present context, the term "cytotoxic phenotype of antigen-specific CD8(+) T cells" refers to the set of observable characteristics of antigen-specific CD8(+) T cells related to their cytotoxic function.

In the present context, the term "lymph node-resident macrophages" refers to macrophages, which are large white blood cell that is an integral part of our immune system that use the process of phagocytosis to engulf particles and then digest them, present in lymph nodes that are small, bean-shaped glands throughout the body.

In the present context, the term "humoral immune response" refers to an immune response mediated by macromolecules found in extracellular fluids such as secreted antibodies, complement proteins, and certain antimicrobial peptides. Its aspects involving antibodies are often called antibody-mediated immunity.

In the present context, the term "B cells", also known as B lymphocytes, are a type of white blood cell of the lymphocyte subtype. They function in the humoral immunity component of the adaptive immune system by secreting antibodies.

An "antibody" when used herein is a protein comprising one or more polypeptides (comprising one or more binding domains, preferably antigen binding domains) substantially or partially encoded by immunoglobulin genes or fragments of immunoglobulin genes. The term "immunoglobulin" (Ig) is used interchangeably with "antibody" herein. The recognized immunoglobulin genes include the kappa, lambda, alpha, gamma, delta, epsilon and mu constant region genes, as well as myriad immunoglobulin variable region genes. In particular, an "antibody" when used herein, is typically tetrameric glycosylated proteins composed of two light (L) chains of approximately 25 kDa each and two heavy (H) chains of approximately 50 kDa each. Two types of light chain, termed lambda and kappa, may be found in antibodies. Depending on the amino acid sequence of the constant domain of heavy chains, immunoglobulins can be assigned to five major classes: A, D, E, G, and M, and several of these may be further divided into subclasses (isotypes), e.g., IgG1, IgG2, IgG3, IgG4, IgA1, and IgA2, with IgG being preferred in the context of the present invention. An antibody relating to the present invention is also envisaged which has an IgE constant domain or portion thereof that is bound by the Fc epsilon receptor I. An IgM antibody consists of 5 of the basic heterotetramer unit along with an additional polypeptide called a J chain, and contains 10 antigen binding sites, while IgA antibodies comprise from 2-5 of the basic 4-chain units which can polymerize to form polyvalent assemblages in combination with the J chain. In the case of IgGs, the 4-chain unit is generally about 150,000 daltons. Each light chain includes an N-terminal variable (V) domain (VL) and a constant (C) domain (CL). Each heavy chain includes an N-terminal V domain (VH), three or four C domains (CHs), and a hinge region. The constant domains are not involved directly in binding an antibody to an antigen, but can exhibit various effector functions, such as participation of the antibody dependent cellular cytotoxicity (ADCC). If an antibody should exert ADCC, it is preferably of the IgG1 subtype, while the IgG4 subtype would not have the capability to exertADCC.

The term "antibody" also includes, but is not limited to, but encompasses monoclonal, monospecific, poly- or multi-specific antibodies such as bispecific antibodies, humanized, camelized, human, single-chain, chimeric, synthetic, recombinant, hybrid, mutated, grafted, and in vitro generated antibodies, with chimeric or humanized antibodies being preferred. The term "humanized antibody" is commonly defined for an antibody in which the specificity encoding CDRs of HC and LC have been transferred to an appropriate human variable frameworks ("CDR grafting"). The term "antibody" also includes scFvs, single chain antibodies, diabodies or tetrabodies, domain antibodies (dAbs) and nanobodies. In terms of the present invention, the term "antibody" shall also comprise bi-, tri- or multimeric or bi-, tri- or multifunctional antibodies having several antigen binding sites.

Furthermore, the term "antibody" as employed in the invention also relates to derivatives of the antibodies (including fragments) described herein. A "derivative" of an antibody comprises an amino acid sequence which has been altered by the introduction of amino acid residue substitutions, deletions or additions. Additionally, a derivative encompasses antibodies which have been modified by a covalent attachment of a molecule of any type to the antibody or protein. Examples of such molecules include sugars, PEG, hydroxyl-, ethoxy-, carboxy- or amine-groups but are not limited to these. In effect the covalent modifications of the antibodies lead to the glycosylation, pegylation, acetylation, phosphorylation, amidation, without being limited to these.

The antibody relating to the present invention is preferably an "isolated" antibody. "Isolated" when used to describe antibodies disclosed herein, means an antibody that has been identified, separated and/or recovered from a component of its production environment. Preferably, the isolated antibody is free of association with all other components from its production environment. Contaminant components of its production environment, such as that resulting from recombinant transfected cells, are materials that would typically interfere with diagnostic or therapeutic uses for the polypeptide, and may include enzymes, hormones, and other proteinaceous or non-proteinaceous solutes. In preferred embodiments, the antibody will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Ordinarily, however, an isolated antibody will be prepared by at least one purification step.

The term "essentially non-immunogenic" means that the block copolymer or amphiphilic polymer of the present invention does not elicit an adaptive immune response, i.e., in comparison to an encapsulated immunogen, the block copolymer or amphiphilic polymer shows an immune response of less than 30%, preferably 20%, more preferably 10%, particularly preferably less than 9, 8, 7, 6 or 5%.

The term "essentially non-antigenic" means that the block copolymer or amphiphilic polymer of the present invention does not bind specifically with a group of certain products that have adaptive immunity (e.g., T cell receptors or antibodies), i.e., in comparison to an encapsulated antigen the block copolymer or amphiphilic polymer shows binding of less than 30%, preferably 20%, more preferably 10%, particularly preferably less than 9, 8, 7, 6 or 5%.

Typically, binding is considered specific when the binding affinity is higher than 10⁻⁶M. Preferably, binding is considered specific when binding affinity is about 10⁻¹¹ to 10⁻⁸ M (KD), preferably of about 10⁻¹¹ to 10⁻⁹ M. If necessary, nonspecific binding can be reduced without substantially affecting specific binding by varying the binding conditions.

The term "amino acid" or "amino acid residue" typically refers to an amino acid having its art recognized definition such as an amino acid selected from the group consisting of: alanine (Ala or A); arginine (Arg or R); asparagine (Asn or N); aspartic acid (Asp or D); cysteine (Cys or C); glutamine (Gln or Q); glutamic acid (Glu or E); glycine (Gly or G); histidine (His or H); isoleucine (He or I): leucine (Leu or L); lysine (Lys or K); methionine (Met or M); phenylalanine (Phe or F); pro line (Pro or P); serine (Ser or S); threonine (Thr or T); tryptophan (Trp or W); tyrosine (Tyr or Y); and valine (Val or V), although modified, synthetic, or rare amino acids may be used as desired. Generally, amino acids can be grouped as having a nonpolar side chain (e.g., Ala, Cys, He, Leu, Met, Phe, Pro, Val); a negatively charged side chain (e.g., Asp, Glu); a positively charged sidechain (e.g., Arg, His, Lys); or an uncharged polar side chain (e.g., Asn, Cys, Gln, Gly, His, Met, Phe, Ser, Thr, Trp, and Tyr).

"Effector cells", preferably human effector cells are leukocytes which express one or more FcRs and perform effector functions. Preferably, the cells express at least FcyRm and perform ADCC effector function. Examples of human leukocytes which mediate ADCC include peripheral blood mononuclear cells (PBMC), natural killer (NK) cells, monocytes, cytotoxic T cells and neutrophils. The effector cells may be isolated from a native source, e.g., blood.

The term "immunizing" refers to the step or steps of administering one or more antigens to a human non-human animal so that antibodies can be raised in the animal.

Specifically, the non-human animal is preferably immunized at least two, more preferably three times with said polypeptide (antigen), optionally in admixture with an adjuvant. An "adjuvant" is a nonspecific stimulant of the immune response. The adjuvant may be in the form of a composition comprising either or both of the following components: (a) a substance designed to form a deposit protecting the antigen (s) from rapid catabolism (e.g. mineral oil, alum, aluminium hydroxide, liposome or surfactant (e.g. pluronic polyol) and (b) a substance that nonspecifically stimulates the immune response of the immunized host animal (e.g. by increasing lymphokine levels therein).

As used herein, "cancer" refers a broad group of diseases characterized by the uncontrolled growth of abnormal cells in the body. Unregulated cell division may result in the formation of malignant tumors or cells that invade neighboring tissues and may metastasize to distant parts of the body through the lymphatic system or bloodstream.

Non-limiting examples of cancers include squamous cell carcinoma, small-cell lung cancer, non- small cell lung cancer, squamous non-small cell lung cancer (NSCLC), non NSCLC, glioma, gastrointestinal cancer, renal cancer (e.g. clear cell carcinoma), ovarian cancer, liver cancer, colorectal cancer, endometrial cancer, kidney cancer (e.g., renal cell carcinoma (RCC)), prostate cancer (e.g. hormone refractory prostate adenocarcinoma), thyroid cancer, neuroblastoma, pancreatic cancer, glioblastoma (glioblastoma multiforme), cervical cancer, stomach cancer, bladder cancer, hepatoma, breast cancer, colon carcinoma, and head and neck cancer (or carcinoma), gastric cancer, germ cell tumor, pediatric sarcoma, sinonasal natural killer, melanoma (e.g., metastatic malignant melanoma, such as cutaneous or intraocular malignant melanoma), bone cancer, skin cancer, uterine cancer, cancer of the anal region, testicular cancer, carcinoma of the fallopian tubes, carcinoma of the endometrium, carcinoma of the cervix, carcinoma of the vagina, carcinoma of the vulva, cancer of the esophagus, cancer of the small intestine, cancer of the endocrine system, cancer of the parathyroid gland, cancer of the adrenal gland, sarcoma of soft tissue, cancer of the urethra, cancer of the penis, solid tumors of childhood, cancer of the ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally-induced cancers including those induced by asbestos, virus-related cancers (e.g., human papilloma virus (HPV)- related tumor), and hematologic malignancies derived from either of the two major blood cell lineages, i.e., the myeloid cell line (which produces granulocytes, erythrocytes, thrombocytes, macrophages and mast cells) or lymphoid cell line (which produces B, T, NK and plasma cells), such as all types of luekemias, lymphomas, and myelomas, e.g., acute, chronic, lymphocytic and/or myelogenous leukemias, such as acute leukemia (ALL), acute myelogenous leukemia (AML), chronic lymphocytic leukemia (CLL), and chronic myelogenous leukemia (CML), undifferentiated AML (MO), myeloblastic leukemia (MI), myeloblastic leukemia (M2; with cell maturation), promyelocytic leukemia (M3 or M3 variant [M3V]), myelomonocytic leukemia (M4 or M4 variant with eosinophilia [M4E]), monocytic leukemia (M5), erythroleukemia (M6), megakaryoblastic leukemia (M7), isolated granulocytic sarcoma, and chloroma; lymphomas, such as Hodgkin' s lymphoma (HL), non-Hodgkin' s lymphoma (NHL), B-cell lymphomas, T-cell lymphomas, lymphoplasmacytoid lymphoma, monocytoid B-cell lymphoma, mucosa-associated lymphoid tissue (MALT) lymphoma, anaplastic (e.g., Ki 1+) large-cell lymphoma, adult T-cell lymphoma/leukemia, mantle cell lymphoma, angio immunoblastic T-cell lymphoma, angiocentric lymphoma, intestinal T-cell lymphoma, primary mediastinal B-cell lymphoma, precursor T-lymphoblastic lymphoma, T-lymphoblastic; and lymphoma/leukaemia (T-Lbly/T-ALL), peripheral T- cell lymphoma, lymphoblastic lymphoma, post-transplantation, lymphoproliferative disorder, true histiocytic lymphoma, primary central nervous system lymphoma, primary effusion lymphoma, lymphoblastic lymphoma (LBL), hematopoietic tumors of lymphoid lineage, acute lymphoblastic leukemia, diffuse large B-cell lymphoma, Burkitt's lymphoma, follicular lymphoma, diffuse histiocytic lymphoma (DHL), immunoblastic large cell lymphoma, precursor B -lymphoblastic lymphoma, cutaneous T-cell lymphoma (CTLC) (also called mycosis fungoides or Sezary syndrome), and lymphoplasmacytoid lymphoma (LPL) with Waldenstrom's macroglobulinemia; myelomas, such as IgG myeloma, light chain myeloma, nonsecretory myeloma, smoldering myeloma (also called indolent myeloma), solitary, plasmocytoma, and multiple myelomas, chronic lymphocytic leukemia (CLL), hairy cell lymphoma; hematopoietic tumors of myeloid lineage, tumors of mesenchymal origin, including fibrosarcoma and rhabdomyoscarcoma; seminoma, teratocarcinoma, tumors of the central and peripheral nervous, including astrocytoma, schwannomas; tumors of mesenchymal origin, including fibrosarcoma, rhabdomyoscaroma, and osteosarcoma; and other tumors, including melanoma, xeroderma pigmentosum, keratoacanthoma, seminoma, thyroid follicular cancer and teratocarcinoma, hematopoietic tumors of lymphoid lineage, for example T-cell and B-cell tumors, including but not limited to T-cell disorders such as T-prolymphocytic leukemia (T-PLL), including of the small cell and cerebriform cell type; large granular lymphocyte leukemia (LGL) preferably of the T-cell type; a/d T-NHL hepatosplenic lymphoma; peripheral/post-thymic T cell lymphoma (pleomorphic and immunoblastic subtypes); angiocentric (nasal) T-cell lymphoma; cancer of the head or neck, renal cancer, rectal cancer, cancer of the thyroid gland; acute myeloid lymphoma, as well as any combinations of said cancers. The methods described herein may also be used for treatment of metastatic cancers, refractory cancers (e.g., cancers refractory to previous immunotherapy, e.g., with a blocking CTLA-4 or PD-1 or PD-L1 antibody), and recurrent cancers.

The term "subject" is intended to include living organisms. Examples of subjects include mammals, e.g., humans, dogs, cows, horses, pigs, sheep, goats, cats, mice, rabbits, rats, and transgenic non-human animals. The subject (animal) can however be a non-mammalian animal such as a bird or a fish. In some preferred embodiments of the invention, the subject is a human, while in other some other preferred embodiments, the subject might be a farm animal, wherein the farm animal can be either a mammal or a non-mammalian animal. Examples of such non-mammalian animals are birds (e.g. poultry such as chicken, duck, goose or turkey), fishes (for example, fishes cultivated in aquaculture such as salmon, trout, or tilapia) or crustacean (such as shrimps or prawns). Examples of mammalian (life stock) animals includes goats; sheep; cows; horses; pigs; or donkeys. Other mammals include cats, dogs, mice and rabbits, for example. In illustrative embodiments the polymersomes of the present invention are used for the vaccination or immunization of the above-mentioned farm animals, both mammalian farm animals and non-mammalian farm animals (a bird, a fish, a crustacean) against virus infections (cf. the Example section in this regard). Accordingly, in such cases, polymersomes of the invention may have encapsulated therein soluble viral full length proteins or soluble fragments of viral full-length proteins.

When used for vaccinations of both humans and non-humans animals, polymersomes or compositions comprising polymersomes of the invention may be administered orally to the respective subject (cf. also the Example Section) dissolved only in a suitable (pharmaceutically acceptable) buffer such as phosphate-buffered saline (PBS) or 0.9 % saline solution (an isotonic solution of 0.90% w/v of NaCl, with an osmolality of 308 mOsm/L). The polymersomes may further be mixed with adjuvants. If administered orally, the adjuvant may help protecting the polymersomes against the acidic environment in the stomach. Such adjuvants may be water-miscible or capable of forming a water-oil emulsion, such as oil in water emulsion or water in oil emulsion. Illustrative examples of such an adjuvant are an oil in water emulsion, a water in oil emulsion, monophosphoryl lipid A, and/or trehalose dicorynomycolate, wherein the oil preferably comprises, essentially consists of or consists of mineral oil, simethicone, Span 80, squalene, and combinations thereof. Further illustrative examples are monophosphoryl lipid A (e.g. from Salmonella Minnesota), trehalose dicorynomycolate, or a mixture thereof, which may be in form of an oil (such as squalene) in water emulsion. Said emulsion may comprise an emulsifier (such as polysorbate, such as polysorbate 80). Alternatively, the polymersomes can be modified, for example, by a coating with natural polymers or can be formulated in particles of natural polymers such as alginate or chitosan or of synthetic polymers such as as poly(d,l-lactide-co-glycolide) (PLG), poly(d,I-lactic-coglycolic acid)(PLGA), poly(g-glutamicacid) (g-PGA) [31,32] or poly(ethylene glycol) (PEG). These particles can either be particles in the micrometer range ("macrobeads") or nanoparticles, or nanoparticles incorporated into macobeads all of which are well known in the art. See, for example. Hari et al, "Chitosan/calcium-alginate beads for oral delivery of insulin", Applied Polymer Science, Volume 59, Issue11, 14 March 1996, 1795-1801, the review of Sosnik "Alginate Particles as Platform for Drug Delivery by the Oral Route: State-of-the-Art" ISRN Pharmaceutics Volume 2014, Article ID 926157, Machado et al, Encapsulation of DNA in Macroscopic and Nanosized Calcium Alginate Gel Particles", Langmuir 2013, 29, 15926-15935, International Patent Application WO 2015/110656, the review "Nanoparticle vaccines" of Liang Zhao et al. Vaccine 32 (2014) 327-337) or Li et al "Chitosan-Alginate Nanoparticles as a Novel Drug Delivery System for Nifedipine" Int J Biomed Sci vol. 4 no. 3 September 2008, 221-228. In illustrative embodiments of these polymersomes and oral formulations, the polymersomes that are used for vaccination have encapsulated therein a viral antigen that comprises a soluble portion of Influenza hemagglutinin, Swine Influenza hemagglutinin, Foot and Mouth Disease (FMD) virus protein such as the VP1, VP2 or VP3 coat protein (the VP1 coat protein contains the main antigenic determinants of the FMD virion, and hence changes in its sequence should be responsible for the high antigenic variability of the virus), Ovalbumin (OVA), a SPIKE protein, such as the Porcine epidemic diarrhea (PED) virus SPIKE protein, a SPIKE protein of a human-pathogenic coronavirus, such as MERS-CoV SPIKE protein, SARS-CoV-2 SPIKE protein, or SARS-CoV-1 SPIKE protein. As evident from the use of polymersomes comprising a soluble portion of the influenza hemagglutinin or a Foot and Mouth Disease (FMD) virus protein such as the VP1, VP2 or VP3 coat protein, the viral disease can affect any animal including birds and mammals, wherein a mammal can also be a human.

The term "effective dose" or "effective dosage" is defined as an amount sufficient to achieve or at least partially achieve the desired effect. The term "therapeutically effective dose" is defined as an amount sufficient to cure or at least partially arrest the disease and its complications in a patient already suffering from the disease. Amounts effective for this use will depend upon the severity of the infection and the general state of the subject's own immune system. The term "patient" includes human and other mammalian subjects that receive either prophylactic or therapeutic treatment.

The appropriate dosage, or therapeutically effective amount, of the antibody or antigen binding portion thereof will depend on the condition to be treated, the severity of the condition, prior therapy, and the patient's clinical history and response to the therapeutic agent. The proper dose can be adjusted according to the judgment of the attending physician such that it can be administered to the patient one time or over a series of administrations. The pharmaceutical composition can be administered as a sole therapeutic or in combination with additional therapies as needed.

If the pharmaceutical composition has been lyophilized, the lyophilized material is first reconstituted in an appropriate liquid prior to administration. The lyophilized material may be reconstituted in, e.g., bacteriostatic water for injection (BWFI), physiological saline, phosphate buffered saline (PBS), or the same formulation the protein had been in prior to lyophilization.

Pharmaceutical compositions for injection may be presented in unit dosage form, e.g., in ampoules or in multi-dose containers, with an added preservative. In addition, a number of recent drug delivery approaches have been developed and the pharmaceutical compositions of the present invention are suitable for administration using these new methods, e. g., Inject-ease, Genject, injector pens such as Genen, and needleless devices such as MediJector and BioJector. The present pharmaceutical composition can also be adapted for yet to be discovered administration methods. See also Langer, 1990, Science, 249: 1527-1533.

The pharmaceutical composition may be prepared for intranasal or inhaled administration, e.g. local administration to the respiratory tract and/or the lung. Means and devides for inhaled administration of a substance are known to the skilled person and are for example disclosed in WO 94/017784A and Elphick et al. (2015) Expert Opin Drug Deliv, 12, 1375-87. Such means and devices include nebulizers, metered dose inhalers, powder inhalers, and nasal sprays. Other means and devices suitable for directing inhaled administration of a drug or vaccine are also known in the art. A preferred route of local administration to the respiratory tract and/or the lung is via aerosol inhalation. An overview about pulmonary drug delivery, i.e. either via inhalation of aerosols (which can also be used in intranasal administration) or intratracheal instillation is given by Patton, J.S., et al. (2004) Proc. Amer. Thoracic Soc., 1, 338-344, for example. Nebulizers are useful in producing aerosols from solutions, while metered dose inhalers, dry powder inhalers, etc. are effective in generating small particle aerosols. The pharmaceutical composition may thus be formulated in form of an aerosol (mixture), a spray, a mist, or a powder.

A pharmaceutical composition against mucosal pathogens such as respiratory coronaviruses like SARS-CoV-2, MERS, or SARS-CoV1 should confer sustained, protective immunity at both system and mucosal levels. A pharmaceutical composition of the disclosure may thus be preferably prepared for mucosal administration, such as inhaled or intranasal administration. As shown in Example 14, intranasal administration of a coronavirus vaccine is not only capable of eliciting a mucosal but also a systemic immune response. A pharmaceutical composition of the disclosure may also be preferably prepared for systemic administration, such as intramuscular administration.

A nebulizer is a drug delivery device used to administer medication in the form of a mist inhaled into the lungs. Different types of nebulizers are known to the skilled person and include jet nebulizers, ultrasonic wave nebulizers, vibrating mesh technology, and soft mist inhalers. Some nebulizers provide a continuous flow of nebulized solution, i.e. they will provide continuous nebulization over a long period of time, regardless of whether the subject inhales from it or not, while others are breath-actuated, i.e. the subject only gets some dose when they inhale from it. A vaccine of the present invention, in particular a vaccine for a human-pathogenic coronavirus infection, such as MERS, COVID-19, or SARS, may be, confectioned for the use in a nebulizer, comprised in a nebulizer or administered by using a nebulizer.

A metered-dose inhaler (MDI) is a device that delivers a specific amount of medication to the lungs, in the form of a short burst of liquid aerosolized medicine. Such a metered-dose inhaler commonly consists of three major components; a canister which comprises the formulation to be administered, a metering valve, which allows a metered quantity of the formulation to be dispensed with each actuation, and an actuator (or mouthpiece) which allows the patient to operate the device and directs the liquid aerosol into the patient's lungs. A vaccine of the present invention, in particular a vaccine for a human-pathogenic coronavirus infection, such as MERS, COVID-19, or SARS, may be, confectioned for the use in a MDI, comprised in a MDI, in particular a canister for an MDI, or administered by using a MDI.

A dry-powder inhaler (DPI) is a device that delivers medication to the lungs in the form of a dry powder. Dry powder inhalers are an alternative to the aerosol-based inhalers, such as metered-dose inhalers. The medication is commonly held either in a capsule for manual loading or a proprietary blister pack located inside the inhaler. A vaccine of the present invention, in particular a vaccine for a human-pathogenic coronavirus infection, such as MERS, COVID-19, or SARS, may be, confectioned for the use in a DPI, comprised in a DPI, in particular a capsule or a blister pack for an MDI, or administered by using a MDI.

A nasal spray can be used for nasal administration, by which a drug is insufflated through the nose. A vaccine of the present invention, in particular a vaccine for a human-pathogenic coronavirus infection, such as MERS, COVID-19, or SARS may be, confectioned as a nasal spray, comprised in a nasal spray bottle, or administered as a nasal spray.

The pharmaceutical composition can also be formulated as a depot preparation. Such long acting formulations may be administered by implantation (for example subcutaneously, into the ligament or tendon, subsynovially or intramuscularly), by subsynovial injection or by intramuscular injection. Thus, for example, the formulations may be modified with suitable polymeric or hydrophobic materials (for example as a emulsion in an acceptable oil) or ion exchange resins, or as sparingly soluble derivatives, for example, as a sparingly soluble salt.

The pharmaceutical compositions may also be in a variety of conventional depot forms employed for administration to provide reactive compositions. These include, for example, solid, semi-solid and liquid dosage forms, such as liquid solutions or suspensions, slurries, gels, creams, balms, emulsions, lotions, powders, sprays, foams, pastes, ointments, salves, balms and drops.

The pharmaceutical compositions may, if desired, be presented in a vial, pack or dispenser device which may contain one or more unit dosage forms containing the active ingredient. In one embodiment, the dispenser device can comprise a syringe having a single dose of the liquid formulation ready for injection. The syringe can be accompanied by instructions for administration.

The pharmaceutical composition may further comprise additional pharmaceutically acceptable components. Other pharmaceutically acceptable carriers, excipients, or stabilizers, such as those described in Remington's Pharmaceutical Sciences 16th edition, Osol, A. Ed. (1980) may also be included in a protein formulation described herein, provided that they do not adversely affect the desired characteristics of the formulation. As used herein, "pharmaceutically acceptable carrier" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, asparagine, 2-phenylalanine, , and threonine; sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as glutathione, thioctic acid, sodium thioglycolate, thioglycerol, [alpha]-monothioglycerol, and sodium thio sulfate; low molecular weight proteins, such as human serum albumin, bovine serum albumin, gelatin, or other immunoglobulins; and hydrophilic polymers, such as polyvinylpyrrolidone.

The formulations described herein are useful as pharmaceutical compositions in the treatment and/or prevention of the pathological medical condition as described herein in a patient in need thereof. The term "treatment" refers to both therapeutic treatment and prophylactic or preventative measures. Treatment includes the application or administration of the formulation to the body, an isolated tissue, or cell from a patient who has a disease/disorder, a symptom of a disease/disorder, or a predisposition toward a disease/disorder, with the purpose to cure, heal, alleviate, relieve, alter, remedy, ameliorate, improve, or affect the disease, the symptom of the disease, or the predisposition toward the disease.

As used herein, the term "treating" and "treatment" refers to administering to a subject a therapeutically effective amount of a pharmaceutical composition according to the invention. A "therapeutically effective amount" refers to an amount of the pharmaceutical composition or the antibody which is sufficient to treat or ameliorate a disease or disorder, to delay the onset of a disease or to provide any therapeutic benefit in the treatment or management of a disease.

As used herein, the term "prophylaxis" refers to the use of an agent for the prevention of the onset of a disease or disorder. A "prophylactically effective amount" defines an amount of the active component or pharmaceutical agent sufficient to prevent the onset or recurrence of a disease.

As used herein, the terms "disorder" and "disease" are used interchangeably to refer to a condition in a subject. In particular, the term "cancer" is used interchangeably with the term "tumor".

As used herein the term "CpG oligonucleotide" may refer to any synthetic or naturally occurring oligodeoxynucleotides (ODNs) containing unmethylated CpG motifs (e.g., as described by Bode et al., CpG DNA as a vaccine adjuvant. Expert Rev Vaccines. 2011 April ; 10(4): 499-511). Thus, any suitable CpG oligonucleotide may be used in the present invention, e.g., in combination with a suitable animal (e.g., as described by Rankin et al., 2001 (Antisense and nucleic acid drug development 11:333-340 (2001), especially in Table1 and 2 therein, as well as in Figure 32 herein). The CpG oligonucleotide may, for example, belong to any of the three major classes of (stimulatory) CpG ODNs that have been identified based on structural characteristics and activity on human peripheral blood mononuclear cells (PBMCs), in particular B cells and plasmacytoid dendritic cells (pDCs). These three classes are Class A (e.g., PS-PO (phosphorothioated-phosphodiester) backbone; also known as Type D), Class B (e.g., PS (phosphorothioated) backbone; also known as Type K) and Class C (e.g., PS (phosphorothioated) backbone). CpG-A ODNs are usually characterized by a PO (phosphodiester) central CpG-containing palindromic motif and a PS-modified (i.e., phosphorothioated-modified) 3' poly-G string, while CpG-B ODNs contain a full PS backbone with one or more CpG dinucleotides. CpG-C ODNs combine features of both classes A and B CpG oligonucleotides. Exemplary CpG ODNs of the present invention are further depicted in Figure 31 herein (derived and modified from https://www.invivogen.com/cpg-odns-classes). Preferred CpG-A ODNs of the present invention are capable of predominantly inducing IFN-α production from plasmacytoid dendritic cells (pDCs) over stimulating TLR9-dependent NF-κB signalling and pro-inflammatory cytokine (e.g. IL-6) production. Preferred CpG-B ODNs of the present invention are capable of predominantly activating B cells and TLR9-dependent NF-κB signalling over stimulating IFN-α secretion. Preferred CpG-C ODNs of the present invention are capable of: (i) predominantly inducing IFN-α production from plasmacytoid dendritic cells (pDCs) over stimulating TLR9-dependent NF-κB signalling and pro-inflammatory cytokine (e.g. IL-6) production; and (ii) predominantly activating B cells and TLR9-dependent NF-κB signalling over stimulating IFN-α secretion.

The kit will typically comprise the container described above and one or more other containers comprising materials desirable from a commercial and user standpoint, including buffers, diluents, filters, needles, syringes, and package inserts with instructions for use.

In the present context, the term "liposome" refers to a spherical vesicle having at least one lipid bilayer.

In the present context, the term "endosome" refers to a membrane-bound compartment (i.e., a vacuole) inside eukaryotic cells to which materials ingested by endocytosis are delivered.

In the present context, the term "late-endosome" refers to a pre-lysosomal endocytic organelle differentiated from early endosomes by lower lumenal pH and different protein composition. Late endosomes are more spherical than early endosomes and are mostly juxtanuclear, being concentrated near the microtubule organizing center.

In the present context, the term "T helper cells" (also called TH cells or "effector CD4(+) T cells") refers to T lymphocytes that assist other white blood cells in immunologic processes, including maturation of B cells into plasma cells and memory B cells, and activation of cytotoxic T cells and macrophages. These cells are also known as "CD4(+) T cells" because they express the CD4 glycoprotein on their surfaces. Helper T cells become activated when they are presented with e.g., peptide antigens, by MHC class II molecules, which are expressed on the surface of antigen-presenting cells (APCs).

As used herein, the term "self-antigen" refers to any molecule or chemical group of an organism which acts as an antigen in inducing antibody formation in another organism but to which the healthy immune system of the parent organism is tolerant.

As used herein, the term "% identity" refers to the percentage of identical amino acid residues at the corresponding position within the sequence when comparing two amino acid sequences with an optimal sequence alignment as exemplified by the ClustalW or X techniques as available from www.clustal.org, or equivalent techniques. Accordingly, both sequences (reference sequence and sequence of interest) are aligned, identical amino acid residues between both sequences are identified and the total number of identical amino acids is divided by the total number of amino acids (amino acid length). The result of this division is a percent value, i.e. percent identity value/degree.

An immunization method of the present invention can be carried out using a either a full length soluble encapsulated antigen (e.g., protein) or fragment of the protein in a synthetic environment that allows its proper folding, and therefore the probability of isolating antibodies capable of detecting corresponding antigens (e.g., a membrane protein) *in vivo* would be higher. Moreover, the immunization and antibody generation can be carried out without any prior knowledge of the membrane protein structure, which may otherwise be necessary when using a peptide-based immunization approach.

Further, when compared to other techniques, the method of the present invention allows for a rapid and cost-effective production of membrane protein encapsulated in an oxidation-stable membrane environment.

In some aspects, the present invention relates to a method for eliciting an immune response to an antigen (e.g., an immunogen) in a subject. The method may include administering to the subject a composition including a polymersome of the present invention having a membrane (e.g., circumferential) of an amphiphilic polymer. The composition further includes a soluble antigen encapsulated by the membrane of the amphiphilic polymer of the polymersome of the present invention. The immunogen may be a membrane-associated protein. In some further aspects, the polymersome of the present invention comprises a lipid polymer. The administration may be carried out in any suitable fashion, for example, by oral administration, topical administration, local administration to the respiratory tract, local administration to the lung, inhaled administration, intranasal administration, or injection.

The frequency of the administration (e.g. oral administration or injection) may be determined and adjusted by a person skilled in the art, dependent on the level of response desired. For example, weekly or bi-weekly administration (e.g. orally or by injection) of polymersomes of the present invention may be given to the subject, which may include a mammalian animal. The immune response can be measured by quantifying the blood concentration level of antibodies (titres) in the mammalian animal against the initial amount of antigen encapsulated by the polymersome of the present invention (cf., the Example Section).

The structure of the polymersomes may include amphiphilic block copolymers self-assembled into a vesicular format and encapsulating various antigens (e.g., soluble proteins, etc.), that are encapsulated by methods of solvent re-hydration, direct dispersion or by spontaneous self-assembly (e.g., Example 1 as described herein).

In the present context, the term "soluble antigen" as used herein means an antigen capable of being dissolved or liquefied. As an illustrative example, soluble antigen may consist of amino acids of the extracellular and/or intracellular region of a membrane protein. It can, however also comprise amino acids from the extracellular and/or intracellular region of a membrane protein and further one or more amino acids belonging to the transmembrane region of the membrane protein, as long as the antigen is still capable of being dissolved or liquefied. As an illustrative example, the soluble fragment of the MERS-CoV Spike protein of SEQ ID NO: 43 is a soluble antigen within the meaning of the present disclosure, while it comprises one amino acid (position 1297), which belongs to the transmembrane region. It is however envisioned that a soluble antigen preferably lacks at least a portion of a transmembrane region or the entire transmembrane region. The term "soluble antigen" includes antigens that were "solubilized", i.e., rendered soluble or more soluble, especially in water, by the action of a detergent or other agent. Exemplary non-limiting soluble antigens of the present invention include: polypeptides derived from a non-soluble portion of proteins, hydrophobic polypeptides rendered soluble for encapsulation as well as aggregated polypeptides that are soluble as aggregates.

In some aspects, the antigens (e.g., membrane proteins) of the present invention are solubilized with the aid of detergents, surfactants, temperature change or pH change. The vesicular structure provided by the amphiphilic block copolymers allows the antigens (e.g., membrane protein) to be folded in a physiologically correct and functional manner, allowing the immune system of the target mammalian animal to detect said antigens, thereby producing a strong immune response.

In some aspects, the injection of the composition of the present invention may include intraperitoneal, subcutaneous, or intravenous, intramuscular injection, or non-invasive administration. In some other aspects, the injection of the composition of the present invention may include intradermal injection.

In some other aspects, the immune response level may be further heightened or boosted by including an adjuvant in the composition including the polymersome of the present invention. The adjuvant may be encapsulated adjuvant or non-encapsulated adjuvant. The adjuvant may be in mixture with a polymersome or combination of the invention. The adjuvant may be soluble in water or may be in form of a water-oil emulsion. In such aspects, the polymersome and the adjuvant can be administered simultaneously to the subject.

In some aspects, a block copolymer or an amphiphilic polymer of the polymersome of the present invention is neither immunostimulant nor adjuvant.

In some other aspects, a block copolymer or an amphiphilic polymer of the polymersome of the present invention is immunostimulant and/or adjuvant.

In some further aspects, a polymersome of the present invention is immunogenic.

In some further aspects, a polymersome of the present invention is non-immunogenic.

In some aspects, the adjuvant may be administered separately from the administration of the composition of the present invention including the polymersome of the present invention. The adjuvant may be administered before, simultaneously, or after the administration of the composition including the polymersome encapsulating an antigen of the present invention. For example, the adjuvant may be injected to the subject after injecting the composition including the polymersome encapsulating an antigen of the present invention. In some aspects, the adjuvant can be encapsulated together with the antigen in the polymersomes. In other preferred aspects the adjuvant is encapsulated in separate polymersomes, meaning the adjuvant in encapsulated separately from the antigen, so the antigen is encapsulated in a first kind of polymersome and the adjuvant is encapsulated in a second kind of polymersome. It is noted here that the adjuvant and the polymersome can be encapsulated in polymersomes that are formed from the same amphiphilic polymer. See Examples 7 to 9 or 14 or 18 of the present application in which the respective antigen and CpG oligodeoxynucleotide (for example, CpG ODN1826: 5'-tccatgacgttcctgacgtt-3', SEQ ID NO: 18 or CpG ODN 2007: 5'- TCGTCGTTGTCGTTTTGTCGTT -3', SEQ ID NO: 63) as illustrative adjuvant are both encapsulated in BD21 polymersomes. Alternatively, the amphiphilic polymer that is used for encapsulation of the antigen can be different from the amphiphilic polymersome that is used for encapsulation of the adjuvant. As a purely illustrative example, the antigen may be encapsulated in BD21 polymersomes while the adjuvant may be encapsulated in PDMS₁₂-PEO₄₆ or PDMS₄₇PEO₃₆ polymersomes.

Any known adjuvant can be used in the present invention and the person skilled in the art will readily recognize and appreciate that the types of adjuvant to be injected may depend on the types of antigen to be used for eliciting an immune response. The adjuvant may be an antigen of bacterial, viral, or fungi origin. The adjuvant may be a nucleic acid such as CpG oligodeoxynucleotides (also known as "CpG ODN" or herein also referred to as "CpG"), CpG molecules are natural oligonucleotides from bacteria that contain unmethylated CpG dinucleotides, in particular sequence contexts (CpG motifs). These CpG motifs are present at a 20-fold greater frequency in bacterial DNA compared to mammalian DNA. CpG ODNs are recognized by Toll-like receptor 9 (TLR9) leading to strong immunostimulatory effects. and are widely commercially available. Illustrative examples of commercially available CpG ODN include ODN 2006, a 24mer having the sequence TCGTCGTTTTGTCGTTTTGTCGTT (SEQ ID NO: 62, commercially available from Miltenyi Biotech under catalogue number 130-100-106), ODN 2007, a 22mer having the sequence 5'- TCGTCGTTGTCGTTTTGTCGTT -3' (SEQ ID NO: 63), ODN 1826 mentioned earlier, a 20mer having the sequence 5'-TCCATGACGTTCCTGACGTT-3' (SEQ ID NO: 18), or ODN 2216, a 20mer having the sequence 5'-GGGGGACGA:TCGTCGGGGGG-3' (SEQ ID NO: 64), with the latter three all being available from InvivoGen. Being natural DNA molecules, the bases are linked together through a phosphodiester bond (PO₄). This bond however is susceptible to degradation from nucleases. When used as an adjuvant without any protective elements, the half-life of nature CpG molecules in the body is extremely short. In order to avoid this short half-life, phosphodiester bonds may be replaced with phosphorothioate bonds by changing one of the oxygen atom to a sulphur atom. This substitution prevents degradation by nucleases and extends the half-life of modified CpG. For example, the CpG molecules ODN 2006, ODN 2007 or ODN 1826 are offered with a complete phosphorothioate backbone form to render them nuclease resistant. Alternatively, CpG are encapsulated in cationic liposomes to avoid the degradation from nucleases. Other than CpG, many other widely used Toll like receptor agonists such as polyinosinic:polycytidylic acid (Poly (I:C)) (TLR3), Lipopolysaccharide (LPS) (TLR4), Monophosphryl lipid (MPL) (TLR5) can be used as one or more adjuvants in the present invention. Furthermore. components derived from bacterial and mycobacterial cell wall such as components present in Sigma Adjuvant System or Freund's adjuvants, or a protein such as Keyhole limpet hemocyanin (KLH) are further illustrative examples of adjuvants that can be also used in the present invention. Further illustrative examples of suitable adjuvants that can be used in the present invention include Sigma Adjuvant System (SAS) or simethicone or alpha-tocopherol. Other antigen-adjuvant pairs are also suitable for use in the methods of the present invention.

In this context, the term "adjuvant" as used herein is not limited to a pharmacological or immunological agent that modifies the effect of other agents (as, for example the adjuvants described above do) but means "any substance that stimulates the actions of the immune system". Thus, a checkpoint inhibitor that stimulates the actions of the immune system is also encompassed within the meaning of the term adjuvant as used herein. For example, PD-L1 that is present on a cell surface binds to PD1 on an immune cell surface, which inhibits immune cell activity. Accordingly, for example, antibodies that bind to either PD-1 or PD-L1 and block the interaction of PD1 with PD-L1 are "such positive checkpoint inhibitor" since they may allow T-cells to attack the tumor.

In some aspects, a membrane protein used as antigen in the present invention may comprise a fragment or a extracellular domain of a transmembrane protein. The antigen may also be a (full length) transmembrane protein, G protein-coupled receptor, neurotransmitter receptor, kinase, porin, ABC transporter, ion transporter, acetylcholine receptor and cell adhesion receptor. The membrane proteins may also be fused to or coupled with a tag or may be tag-free. If the membrane proteins are tagged, then the tag may, for example, be selected from well-known affinity tags such as VSV, His-tag, Strep-tag^{®}, Flag-tag, Intein-tag or GST-tag or a partner of a high affinity binding pair such as biotin or avidin or from a label such as a fluorescent label, an enzyme label, NMR label or isotope label.

In some aspects, the membrane proteins of fragments (or portions) thereof may be presented prior to encapsulation, or encapsulated simultaneously with the production of the protein through a cell-free expression system. The cell-free expression system may be an *in vitro* transcription and translation system.

The cell-free expression system may also be an eukaryotic cell-free expression system such as the TNT system based on rabbit reticulocytes, wheat germ extract or insect extract, a prokaryotic cell-free expression system or an archaic cell-free expression system.

An antigen or fragment (or portion) thereof of the disclosure may be produced in vivo. The antigen or fragment (or portion) thereof can for example be produced in a bacterial or eukaryotic host organism and then isolated from this host organism or its culture. It is also possible to produce antigen or fragment (or portion) thereof in vitro, for example by use of an in vitro translation system. A preferred expression system is the Baculovirus expression system. The utilization of the Baculovirus protein expression system is often overlooked as it is seen as being slow and expensive. However, one of the major advantages of the Baculovirus system is that the cell lines can be produced and maintained independent of the virus. This allows for rapid production of new subunit antigens without having to gain regulatory approval for new cell lines a useful tool given the rapid change in the sequence of virus's like MERS-CoV and SARS-CoV-1. Moreover, Baculovirus system produces antigens with novel glycosylation profiles compared to mammalian systems that have been shown to enhance the immune response. For example, both the full soluble (S1-S2) domains of the spike proteins for SARS-CoV-1 and MERS-CoV can been expressed in Sf9 cells. These proteins once immunised into Balb/c mice and show high virus neutralisation titres whether given alone, with alum of Matrix M1 adjuvants and this neutralisation may last for at least 45 days. The antigen of the disclosure is thus preferably produced using a eukaryotic host cell, preferably an insect cell, such as a Sf9 cell, or preferably using a Baculovirus expression system.

As mentioned above, the polymersomes may be formed of amphiphilic diblock or tri-block copolymers. In various aspects, the amphiphilic polymer may include at least one monomer unit of a carboxylic acid, an amide, an amine, an alkylene, a dialkylsiloxane, an ether or an alkylene sulphide.

In some aspects, the amphiphilic polymer may be a polyether block selected from the group consisting of an oligo(oxyethylene) block, a poly(oxyethylene) block, an oligo(oxypropylene) block, a poly(oxypropylene) block, an oligo(oxybutylene) block and a poly(oxybutylene) block. Further examples of blocks that may be included in the polymer include, but are not limited to, poly(acrylic acid), poly(methyl acrylate), polystyrene, poly(butadiene), poly(2-methyloxazoline), poly(dimethyl siloxane), poly(e-caprolactone), poly(propylene sulphide), poly(N-isopropylacrylamide), poly(2-vinylpyridine), poly(2-(diethylamino)ethyl methacrylate), poly(2-diisopropylamino)ethylmethacrylate), poly(2-methacryloyloxy)ethylphosphorylcholine, poly (isoprene), poly (isobutylene), poly (ethylene-co-butylene) and poly(lactic acid). Examples of a suitable amphiphilic polymer include, but are not limited to, poly(ethyl ethylene)-b-poly(ethylene oxide) (PEE-b-PEO), poly(butadiene)-b-poly(ethylene oxide) (PBD-b-PEO), poly(styrene)-b-poly(acrylic acid) (PS-PAA), poly (dimethylsiloxane)-poly(ethylene oxide (herein called PDMS-PEO) also known as poly(dimethylsiloxane-b-ethylene oxide), poly(dimethyl siloxane)-poly(acrylic acid) (PDMS-PAA), poly(2-methyloxazo1ine)-b-poly(dimethylsiloxane)-b-poly(2-methyloxazoline) (PMOXA-bPDMS-bPMOXA) including for example, triblock copolymers such as PMOXA₂₀-PDMS₅₄-PMOXA₂₀ (ABA) employed by May et al., 2013, poly(2-methyloxazoline)-b-poly(dimethylsiloxane)-b-poly(ethylene oxide) (PMOXA-b-PDMS-b-PEO), poly(ethylene oxide)-b-poly(propylene sulfide)-b-poly(ethylene oxide) (PEO-b-PPS-b-PEO) and a poly(ethylene oxide)-poly(butylene oxide) block copolymer. A block copolymer can be further specified by the average block length of the respective blocks included in a copolymer. Thus, PB_{M}PEO_{N} indicates the presence of polybutadiene blocks (PB) with a length of M and polyethyleneoxide (PEO) blocks with a length of N. M and N are independently selected integers, which may for example be selected in the range from about 6 to about 60. Thus, PB₃₅PEQ₁₈ indicates the presence of polybutadiene blocks with an average length of 35 and of polyethyleneoxide blocks with an average length of 18. In certain aspects, the PB-PEO diblock copolymer comprises 5-50 blocks PB and 5-50 blocks PEO. Likewise, PB₁₀PEO₂₄ indicates the presence of polybutadiene blocks with an average length of 10 and of polyethyleneoxide blocks with an average length of 24. Illustrative examples of suitable PB-PEO diblock copolymers that can be used in the present invention include the diblock copolymers PBD₂₁-PEO₁₄ (that is also commercially available) and [PBD]₂₁-[PEO]₁₂, (cf,, WO2014/077781A1 and Nallani et al., 2011), As a further example E₀Bₚ indicates the presence of ethylene oxide blocks (E) with a length of 0 and butadiene blocks (B) with a length of P. Thus, O and P are independently selected integers, e.g. in the range from about 10 to about 120. Thus, E₁₆E₂₂ indicates the presence of ethylene oxide blocks with an average length of 16 and of butadiene blocks with an average length of 22.

Turning to another preferred block copolymer that is used to form polymersome of the invention, poly(dimethylsiloxane-b-ethyleneoxide) (PDMS-PEO), it is noted that both linear and comb-type PDMS-PEO can be used herein (cf. Gaspard et al, "Mechanical Characterization of Hybrid Vesicles Based on Linear Poly(Dimethylsiloxane-b-Ethylene Oxide) and Poly(Butadiene-b-Ethylene Oxide) Block Copolymers" Sensors 2016, 16(3), 390 which describes polymersomes formed from PDMS-PEO).

The structure of linear PDMS-PEO is shown in the following as formula (I) 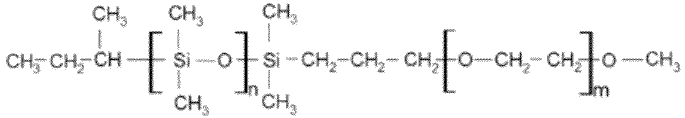 while the structure of comb-type PDMS-PEO is shown in the following formula (II): 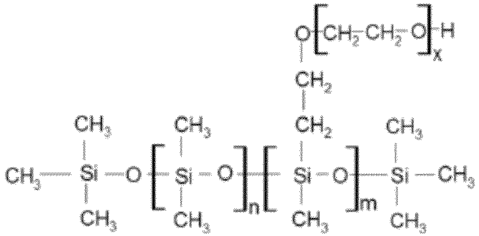 In line with the structural formula (I), the terminology PDMSₙ-PEOₘ indicates the presence of polydimethylsiloxane (PDMS) blocks with a length of n and polyethyleneoxide (PEO) blocks with a length of m. m and n are independently selected integers, each of which may, for example, be selected in the range from about 5 or about 6 to about 100, from about 5 to about 60 or from about 6 to about 60 or from about 5 to 50. For example, linear PDMS-PEO such as PDMS₁₂-PEO₄₆, or PDMS₄₇PEO₃₆ are commercially available from Polymer Source Inc., Dorval (Montreal) Quebec, Canada. Accordingly, the PDMS-PEO block copolymer may comprise 5-100 blocks PDMS and 5-100 blocks PEO, 6-100 blocks PDMS and 6-100 blocks PEO, 5-100 blocks PDMS and 5-60 blocks PEO, or 5-60 blocks PDMS and 5-60 blocks PEO.

In accordance with the above, the present invention relates in one aspect to the composition for use in a method of eliciting an immune response in a subject, comprising administering to the subject a polymersome formed from PDMS-PEO carrying an antigen. The antigen can be associated/physically linked with the PDMS-PEO polymersome in any suitable way. For example, the PDMS-PEO polymersome may have a soluble antigen encapsulated therein as described in the present invention. Alternatively or in addition, the polymersome may have an antigen integrated/incorporated into the circumferential membrane of the polymersome as described in WO2014/077781A1. In this case, antigen is a membrane protein that is integrated with its (one or more) transmembrane domain into the circumferential membrane of the PDMS-PEO-polymersome. The integration can be achieved as described in WO2014/077781A1 or Nallani et al, "Proteopolymersomes: in vitro production of a membrane protein in polymersome membranes", Biointerphases, 1 December 2011, page 153. In case, the antigen is encapsulated in the PDMS-PEO polymersome, it may be a soluble antigen selected from the group consisting of a polypeptide, a carbohydrate, a polynucleotide and combinations thereof. Also disclosed, but not claimed, is a method for production of such encapsulated antigens in a polymersome formed from PDMS-PEO as well as to polymersomes produced by said method.

Disclosed, but not claimed, are compositions comprising PDMS-PEO polymersomes carrying an antigen. Also, in these compositions, the antigen can be associated/physically linked with the PDMS-PEO polymersome in any suitable way. For example, the PDMS-PEO polymersome may have a soluble antigen encapsulated therein. Alternatively, or in addition, the polymersome may have an antigen integrated/incorporated into the circumferential membrane of the polymersome as described in WO2014/077781A1. The present invention also relates to vaccines comprising such PDMS-PEO polymersomes carrying an antigen, methods of eliciting an immune response or methods for treatment, amelioration, prophylaxis or diagnostics of cancers, autoimmune or infectious diseases, such methods comprising providing PDMS-PEO polymersomes carrying an antigen to subject in need thereof.

Disclosed, but not claimed, is the *in vitro* and *in vivo* use of a PDMS-PEO polymersomes carrying (or transporting) an antigen in a manner suitable for eliciting an immune response. The antigen can either be encapsulated in the PDMS-PEO polymersome or, for example, incorporated into the circumferential membrane of the polymersome as described in WO2014/077781A1.

Another preferred block copolymer is poly(dimethyl siloxane)-poly(acrylic acid) (PDMS-PAA). The PDMS-PAA may be PDMS_{M}-PAA_{N} which indicates the presence of poly(dimethyl siloxane) (PDMS) blocks with a length of M and poly(acrylic acid) (PAA) blocks with a length of N. M and N are independently selected integers, which may for example be selected in the range from about 5 to about 100 and represent the average length of the blocks. The PDMS-PAA preferably comprises 5-100 blocks PDMS and 5-100 blocks PAA. Preferably, the PDMS-PAA comprises 5-50, preferably 10-40 blocks of PDMS and/or 5-30, preferably 5-25, preferably 5-20 blocks of PAA. The PDMS-PAA is preferably selected from the group consisting of PDMS₃₀-PAA₁₄, PDMS₁₅-PAA₇, or PDMS₃₄-PAA₁₆.

In certain aspects, the polymersome of the present invention may contain one or more compartments (or otherwise termed "multicompartments). Compartmentalization of the vesicular structure of polymersome allows for the coexistence of complex reaction pathways in living cell and helps to provide a spatial and temporal separation of many activities inside a cell. Accordingly, more than one type of antigens may be encapsulated by the polymersome. The different antigens may have the same or different isoforms. Each compartment may also be formed of a same or a different amphiphilic polymer. In various aspects, two or more different antigens are integrated into the circumferential membrane of the amphiphilic polymer. Each compartment may encapsulate at least one of peptide, protein, and nucleic acid. The peptide, protein, polynucleotide or carbohydrate may be immunogenic.

Further details of suitable multicompartmentalized polymersomes can be found in WO 20121018306.

The polymersomes may also be free-standing or immobilized on a surface, such as those described in WO 2010/1123462.

In the case where the polymersome carrier contains more than one compartment, the compartments may comprise an outer block copolymer vesicle and at least one inner block copolymer vesicle, wherein the at least one inner block copolymer vesicle is encapsulated inside the outer block copolymer vesicle. In some aspects, each of the block copolymer of the outer vesicle and the inner vesicle includes a polyether block such as a poly(oxyethylene) block, a poly(oxypropylene) block, and a poly(oxybutylene) block. Further examples of blocks-that may be included in the copolymer include, but are not limited to, poly(acrylic acid), poly(methyl acrylate), polystyrene, poly(butadiene), poly(2-methyloxazoline), poly(dimethyl siloxane), poly(L-isocyanoalanine(2-thiophen-3-yl-ethyl)amide), poly(e-caprolactone), poly(propylene sulphide), poly(N-isopropylacrylamide), poly(2-vinylpyridine), poly(2-(diethylamino)ethyl methacrylate), poly(2-(diisopropylamino)ethylmethacrylate), poly(2-(methacryloyloxy)ethylphosphorylcholine) and poly(lactic acid). Examples of suitable outer vesicles and inner vesicles include, but are not limited to, poly(ethyl ethylene)-b-poly(ethylene oxide) (PEE-b-PEO), poly(butadiene)-b-poly( ethylene oxide) (PBD-b-PEO), poly(styrene)-b-poly(acrylic acid) (PS-b-PAA), poly(ethylene oxide)-poly(caprolactone) (PEO-b-PCL), poly(ethylene oxide)-poly(lactic acid) (PEO-b-PLA), poly(isoprene)-poly(ethylene oxide) (PI-b-PEO), poly(2-vinylpyridine)-poly(ethylene oxide) (P2VP-b-PEO), poly(ethylene oxide)-poly(N-isopropylacrylamide) (PEO-b-PNIPAm), poly(ethylene glycol)-poly(propylene sulfide) (PEG-b-PPS), poly(dimethyl siloxane)-poly(acrylic acid) (PDMS-PAA), poly (methylphenylsilane)-poly(ethylene oxide) (PMPS-b-PEO-b-PMPS-b-PEO-b-PMPS), poly(2-methyloxazoline)-b-poly-(dimethylsiloxane)-b-poly(2-methyloxazoline) (PMOXA-b-PDMS-b-PMOXA), poly(2-methyloxazoline)-b-poly(dimethylsiloxane)-b-poly(ethylene oxide) (PMOXA-b-PDMS-b-PEO), poly[styrene-b-poly(L-isocyanoalanine(2-thiophen-3-yl-ethyl)amide)] (PS-b-PIAT), poly(ethylene oxide)-b-poly(propylene sulfide)-b-poly(ethylene oxide) (PEO-b-PPS-b-PEO) and a poly(ethylene oxide)-poly(butylene oxide) (PEO-b-PBO) block copolymer. A block copolymer can be further specified by the average number of the respective blocks included in a copolymer. Thus PS_{M}-PIAT_{N} indicates the presence of polystyrene blocks (PS) with M repeating units and poly(L-isocyanoalanine(2-thiophen-3-yl-ethyl)amide) (PIAT) blocks with N repeating units. Thus, M and N are independently selected integers, which may for example be selected in the range from about 5 to about 95. Thus, PS₄₀-PIAT₅₀ indicates the presence of PS blocks with an average of 40 repeating units and of PIAT blocks with an average of 50 repeating units.

In some aspects, the polymersome of the disclosure includes a lipid, which is preferably in mixture with the block copolymer or amphiphilic polymer. The content of the lipid is typically low as compared to the amount of block copolymer or amphiphilic polymer. Typically, the lipid will be up to about 50%, up to about 45%, up to about 40%, up to about 35%, up to about 30%, up to about 20%, up to about 15%, up to about 10%, up to about 5%, up to about 2%, up to about 1%, up to 0.5%, up to about 0.2%, up to about 0.1% of the components that form the polymersome membrane (percentages are given by weight). Addition of a lipid may enhance encapsulation efficiency. The lipid may be a synthetic lipid, a natural lipid, a lipid mixture, or a combination of synthetic and natural lipids. Non-limiting examples for a lipid are phospholipids, such as a phosphatidylcholine, such as POPC, lecithin, cephalin, or phosphatidylinositol, or lipid mixture comprising phospholipids such as soy phospholipids such as asolectin. Further non-limiting examples of a lipid include cholesterol, cholesterol sulfate, 1,2-Dioleoyl-3-trimethylammonium propane (DOTAP). The lipid is preferably non-antigenic. In some aspects, the polymersome of the disclosure includes less than about 20%, less than about 15%, less than about 10%, less than about 5%, less than about 2%, less than about 1%, less than about 0.5%, less than about 0.2%, less than about 0.1% or is essentially free of a saponin (percentages are given by weight).

Disclosed, but not claimed, is a method for production of an encapsulated antigen in polymersome, said method comprising: i) dissolving an amphiphilic polymer of the present invention in chloroform, preferably said amphiphilic polymer is polybutadiene-polyethylene oxide (BD); ii) drying said dissolved amphiphilic polymer to form a polymer film; iii) adding a solubilized antigen to said dried amphiphilic polymer film from step ii), wherein said antigen is selected from the group consisting of:
(a) a polypeptide; preferably said polypeptide is an antigen;
(b) a carbohydrate; (c) a combination of a) and/or b) and/or c); iv) rehydrating said polymer film from step iii) to form polymer vesicles; v) optionally, filtering polymer vesicles from step iv) to purify polymer vesicles monodisperse vesicles; and/or vi) optionally, isolating said polymer vesicles from step iv) or v) from the non-encapsulated antigen.

Disclosed, but not claimed, are other methods for production of an encapsulated antigen in polymersome including methods based on mixing a non-aqueous solution of polymers in aqueous solution of antigens, sonication of corresponding mixed solutions of polymers and antigens, or extrusion of corresponding mixed solutions of polymers and antigens. Exemplary methods include those described in Rameez et al, Langmuir 2009, and in Neil et al Langmuir 2009, 25(16), 9025-9029.

In some aspects, the invention relates to a composition for use in a method of eliciting an immune response in a subject by sole administration of one or more adjuvants, wherein said one or more adjuvants are associated with one or more populations of polymersomes.

According to the claims, sole administration is an administration characterized in that no antigen is administered to said subject in combination with said one or more adjuvants.

In some aspects, sole administration of the present invention is one or more of the following: (i) a prophylactic sole administration (e.g., for anti-viral and/or immunomodulatory prophylaxis); (ii) a therapeutic sole administration (e.g., for anti-viral and/or immunomodulatory treatment); (iii) a sole administration for reducing stress level in a subject (e.g. reducing stress level during shipping, transportation of cattle and/or mixing of cattle with other animals; (iv) any combination of (i)-(iii).

In some aspects, one or more adjuvants of the present invention are independently associated with the same or different populations of polymersomes of the present invention.

In some aspects, one or more adjuvants of the present invention are independently associated with said one or more populations of polymersomes by one or more of the following means: (i) encapsulating said one or more adjuvants within said one or more populations of polymersomes; (ii) integrating and/or embedding said one or more adjuvants into the circumferential membranes of said one or more populations of polymersomes; (iii) conjugating said one or more adjuvants to the exterior surfaces of said one or more populations of polymersomes via covalent bonds; (iv) conjugating said one or more adjuvants to the exterior surfaces of said one or more populations of polymersomes via a non-covalent bond; and/or (v) any combination of (i)-(iv).

In some aspects, one or more adjuvants are independently selected from the group consisting of: (i) CpG oligodeoxynucleotides as described herein (or CpG ODN), (ii) non-antigenic components derived from bacterial and mycobacterial cell walls and proteins.

In some aspects, polymersomes are oxidation-stable polymersomes.

In some aspects, an immune response comprises stimulating production and/or secretion of one or more cytokines, preferably said immune response is an innate immune response.

In some aspects, the immune response comprises stimulating production and/or secretion of one or more cytokines comprising stimulating production and/or secretion of interleukin-6 (IL-6), preferably said production and/or secretion of interleukin-6 (IL-6) is predominant over production and/or secretion of interleukin-12 (IL-12), further preferably said production and/or secretion of interleukin-6 (IL-6) is free from production and/or secretion of interleukin-12 (IL-12).

In some aspects, the route of said sole administration is selected from the group consisting of: oral administration, intranasal administration, administration to a mucosal surface, inhalation, intradermal administration, intraperitoneal administration, subcutaneous administration, intravenous administration and intramuscular administration.

In some aspects, subject is selected from the group consisting of: (i) a mammalian animal, preferably said mammalian animal is a human, cat, dog, cattle, goat, sheep, cow or pig; and (ii) a non-mammalian animal.

In some aspects, the compositions for use in a method of the present invention is a method of treatment and/or prophylactic method against a disease selected from the group consisting of: cancer (e.g., sarcoma, fibrosarcoma), Atopic Dermatitis, African swine fever, Avian influenza, Bovine spongiform encephalopathy, Brucellosis, Cattle Fever Tick, Chronic wasting disease, Classical swine fever, Contagious equine metritis, Equine herpesvirus, Equine infectious anemia, Equine piroplasmosis, Equine viral arteritis, Foot and mouth disease, Johnes disease, Mycoplasma ovipneumoniae, Porcine Epidemic Diarrhea Virus, Pseudorabies, Rabbit Hemorrhagic Disease Virus, Schmallenberg Virus, Scrapie, Spring viremia carp, Influenza A virus in swine, Tuberculosis, Vesicular stomatitis, West Nile virus, stress-related diseases (e.g., pasteurellosis, Mannheimia haemolytica, and coccidiosis), viral disease (e.g., Feline Calicivirus, Coronavirus, Herpesvirus, Canine parvovirus, Swine post-weaning Diarrhea), anti-viral treatment or prophylaxis, immune disease, immunomodulatory treatment treatment or prophylaxis, Feline Calicivirus, Coronavirus, Herpesvirus, Canine parvovirus, Swine post-weaning Diarrhea, Upper respiratory disease complex for cattle, horses and/or kittens.

Compared to existing uptake and cross-presentation vehicles and methods based thereon the polymersomes of present invention *inter alia* offer the following advantages that are also aspects of the present invention:
- The polymersomes are very efficient in uptake and cross-presentation to the immune system;
- The immune response comprises a CD8⁽⁺⁾ T cell-mediated immune response;
- The polymersomes are oxidation-stable;
- The humoral response is stronger compared to that produced by free antigen-based techniques with or without adjuvants;
- The immune response induced by polymersomes of the present invention could still be even further boosted using adjuvants;
- The polymers of polymersomes of the present invention are inherently robust and can be tailored or functionalized to increase their circulation time in the body;
- The polymersomes of the present invention are stable in the presence of serum components;
- The polymers of polymersomes are inexpensive and quick to synthesize;
- The amount of an antigen required to elicit an immune response by the methods of the present invention using polymersomes of the present invention is less compared to free antigen-based techniques with or without adjuvants.
- The composition for use in a method of eliciting an immune response in a subject by sole administration of one or more adjuvants of the present invention allows for improved eliciting an immune response in the absence of antigens (e.g., for anti-viral and/or immunomodulatory prophylaxis and/or treatment).
- ACM encapsulation enhanced the biological function of CpG.
- ACM-CpG exhibited superior adjuvant activity compared to free CpG.
- ACM-CpG induced broader cytokine profile than free CpG.
- ACM-CpG encapsulated in polymersomes of the present invention consisting of BD21 (85 mole %) and DOTAP (15 mole %) when administered at a single dosage from about 7.5 µg to about 12.5 µg CpG per kg produced a significant immune response comprising therapeutic and/or prophylactic modality against highly infection agents (e.g., bacteria and/or viruses, e.g., Calcici virus and/or FHV (Feline herpesvirus)) in cats and dogs (e.g., see Examples 23-24 herein).

### Examples of the invention

In order that the invention may be readily understood and put into practical effect, some aspects of the invention are described by way of the following non-limiting examples.

### Materials and Methods

### Example 1: Encapsulation of Ovalbumin, adjuvants, peptides, soluble HA, PEDv SPIKE protein and eGFP DNA in polymersomes

A 100 mg/ml stock of Polybutadiene-Polyethylene oxide (herein referred to as "BD21") is dissolved in chloroform. 100 µL of the 100 mg/ml BD21 stock is then deposited into a borosilicate (12x75 mm) culture tube and slowly dried under a stream of nitrogen gas to form a thin polymer film. The film was further dried under vacuum for 6 hours in a desiccator. A 1 mL solution of 1-5 mg/ml solubilized Ovalbumin (OVA) protein in 1x PBS buffer was then added to the culture tube. The mixture was stirred at 600 rpm, 4 °C for at least 18 hours to rehydrate the film and to allow the formation of polymer vesicles. The turbid suspension was extruded through a 200-nm pore size Whatman Nucleopore membrane with an extruder (Avanti 1 mL liposome extruder, 21 strokes) to obtain monodisperse vesicles [e.g., Fu et al., 2011, Lim. S.K, et al., 2017]. The protein containing BD21 polymer vesicles were purified from the non-encapsulated proteins by dialyzing the mixture against 1 L of 1x PBS using a dialysis membrane (300kDa MWCO, cellulose ester membrane).

The final vesicle mixture was analysed for non-encapsulated protein using size-exclusion chromatography. Fractions of the vesicle peak from SEC were used to quantify the amount of protein encapsulation via SDS-PAGE. Vesicle size and mono-dispersity was characterized by dynamic light scattering instrument (Malvern, United Kingdom) (100x dilution with 1x PBS). For quantification of OVA encapsulated in polymersomes, samples were pre-treated with 20% DMSO followed by sample buffer, after which they were loaded on to the SDS-PAGE analysis.

For peptides encapsulation (exemplified by MC 38 neo-antigen peptides of SEQ ID NO: 1, 2 and 3), a similar protocol was followed. Peptides concentration was 0.5-0.3 mg/ml dissolved in PBS for encapsulation. After dialysis, an amount of encapsulated peptides was determined using Phenylalanine fluorescence (ex 270nm/ em 310nm) using a Cary Eclipse Spectrophotometer (Agilent). Encapsulation of all 3 peptides was performed individually and concentration was determined to be 20-30ug/ml for all peptides. An equivalent volume of each of 3 encapsulated peptides was mixed together just before injection into mice.

For Trp2 peptide encapsulation, co-solvent or nanoprecipitation method was followed. 0.4 mg of Trp2 173-196 peptide (QPQIANCSVYDFFVWLHYYSVRDT, SEQ ID NO: 9) was diluted in 1 ml of buffer containing 10 mM Borate buffer, 125 mM NaCl, 10% Glycerol, pH 8.5. 4.25 µmol of BD21/0.75 µmol of Dioleoyl-3-trimethylammonium propane (DOTAP lipid) mixture dissolved in THF was added slowly to the solution while vortexing vigorously for 4-5 h. Extrusion and dialysis was performed as above with slight modification in the dialysis step. Briefly, vesicles were then filtered through a 0.22 µm filter (PES membrane, Millipore) and subjected to dialysis over 48 h with 3 buffer exchanges. Concentration of encapsulated Trp2 was determined by HPLC and the final concentration of Trp2 is 160 µg/ml.

For adjuvant CpG encapsulation (using the class B CpG-Oligodeoxynucleotide of SEQ ID NO: 18, available from InvivoGen), 4.25 µmol of BD21/0.75 µmol of Dioleoyl-3-trimethylammonium propane (DOTAP lipid) mixture was dissolved in chloroform. The resulting mixture was then deposited into a borosilicate (12x75 mm) culture tube and slowly dried under a stream of nitrogen gas to form a thin polymer film. The film was further dried under vacuum for 6 hours in a desiccator. 100 µg of the CpG dissolved in 10 mM Borate buffer, 125 mM NaCl, 10% Glycerol. The samples were extruded was then dialyzed over 48 h with 3 buffer exchanges. CpG quantified by generating a standard curve using known amount of CpG using SYBR-Safe dye. ACM samples were ruptured and incubated for 30 min at RT and transferred to a black plate for quantification (Ex500 nm: Em 530 nm). Routinely, the encapsulated CpG concentration was around 70-90 µg/ml.

For HA encapsulation, a similar protocol was followed. Recombinant HA (H1N1/A/Puerto Rico/8/1934 strain) at a concentration of 10 ug/ml was dissolved in PBS for encapsulation. After dialysis, an amount of encapsulated peptides was determined by western blot. HA concentration after encapsulation was determined to be around 1ug/ml. 100ul were injected in mice.

For PEDv SPIKE protein encapsulation in BD21 polymersomes, a similar protocol was followed as described above. PEDv SPIKE protein (different constructs, SEQ ID Nos: 12-14) were expressed using Baculovirus expression system. Proteins isolated from the insect cells were added for encapsulation. Whereas, for encapsulation of PEDv SPIKE protein in polymersomes made of poly (dimethyl siloxane)-poly(ethylene oxide (PDMS₄₆-PEO₃₇ obtained from Polymer Source, Quebec, Canada), or a mixture of block copolymers and lipids such as PDMS₄₆-PEO₃₇ (/DSPE-PEG, PLA-PEG/POPC, PLA-PEG/Asolectin, a different protocol was followed in order to show the generality of the methods. Polymer and or polymer lipid mixture were dissolved in ethanol or any water miscible solvent and added dropwise to a protein solution to self-assemble and the proteins are encapsulated into polymersomes during self-assembly. Non-encapsulated proteins were removed by dialysis with PBS. After dialysis, amount of each polymersome sample encapsulated proteins was determined by densitometry. The concentration of proteins after encapsulation was determined to be around 1 µg/ml for each of these polymersome formulations. Polymersomes were encapsulated either with soluble SPIKE protein (SEQ 12) or S1 region of SPIKE protein (SEQ 13) and S2 region of SPIKE protein (SEQ 14). 100-200µl of polymersomes (either only with soluble SPIKE protein or with mixture of polymersomes with S1 and S2 region of SPIKE proteins) were injected in mice and 1 ml of such polymersomes was orally administered to pigs.

For eGFR DNA encapsulation, a similar protocol as OVA encapsulation was followed. Briefly, block co-polymers such as poly(butadiene)-poly(ethyleneoxide) (BD21), poly(butadiene)-poly(ethyleneoxide) modified with functional groups (e.g., NH₂, COOH) at the end of poly (ethylene oxide) chain (BD21-NH₂), mixture of block copolymers and lipids such as PLA-PEG/POPC, PLA-PEG/Asolectin, Dimethylaminoethane-carbamoyl (DC)-Cholesterol, 1,2-dioleoyl-3-trimethylammonium-propane (DOTAP) were dissolved in chloroform and transferred to a glass tube and slowly dried under a stream of nitrogen gas to form a thin film. The film was further dried under vacuum for 6 hours in a desiccator. 1 µg of eGFP DNA was added to the film and rehydrated overnight. Afterwards, the samples were extruded with 0.2 um polycarbonate filter and dialyzed in HEPES Buffer.

### Example 2: Transfection of eGFP DNA encapsulated polymersomes with HEK293T Cells

HEK293T cells were seeded with a density of 50,000 cells/well into a 48-well plate. For transfections (the Lipofectamine 2000 transfection), 1,000 µL of Opti-MEM I (Invitrogen), 2 µL of Lipofectamine 2000 (Invitrogen), and 1 µg of SF-GFP PC DNA (or polymersomes formulation containing 1 µg of SF-GFP PC DNA) were mixed. The transfection complexes were formed during 20 min incubation at RT. For transfection, the lipofectamine complex was added to the cells and incubated for 24 hr to 72 hr at 37C and 5% CO2. The efficiency of transfection was measured by GFP fluorescence, Ex 485 nm, Em 520 nm). For cellular uptake fluorescence measured at Ex 530 nm Em 560 nm. For imaging, aspirated the cell media followed by washed the cells with DPBS (with Ca2+/Mg2+) and fixed with 4% p-formaldehyde. Then, the glass cover-slip was removed and flipped into a glass slide containing a drop of 20 ul mounting media with DAPI. Finally, sealed the cover-slip with nail polish and stored at 4C for future imaging. Fluorescence microscopy was used for imaging.

### Example 3: Immunization of OVA encapsulated polymersomes for antibody titers

C57bl/6 mice were immunized using free OVA with or without Sigma Adjuvant System (SAS) and OVA encapsulated ACMs (polymersomes) by doing a prime and a boost 21 days later. All immunizations were performed with a final amount of OVA: 5-10ug OVA/ injection/ mouse. Final bleeds were collected 42 days after prime. ELISA was then performed to assess titers: OVA was coated onto MaxiSorp plates (1ug/ml) overnight. Plates were blocked using 3% BSA in PBS for 1h at RT. All sera were diluted at 1:100 and incubated on plates for 1h at RT. After 3 washes with PBS + 0.05% Tween 20, secondary antibody anti-mouse IgG HRP coupled was incubated at 1:10,000 dilution for 1h, RT (room temperature). After 3 washes with PBS/ Tween 20 buffer, TMB substrate was added and reaction was stopped using 1M HCl. Optical densities were quantified at 450nm.

### Example 4: Immunization of HA encapsulated polymersomes for antibody titers

Similarly, Balb/c mice were immunized with free HA proteins (SEQ ID NO: 7), ACM encapsulated HA (polymersomes) in PBS or PBS control. All immunizations were performed with a same final amount of HA: 100ng HA/ injection/ mouse. Final bleeds were collected 42 days after prime and ELISA were performed as above using 1ug/ml HA for plate coating.

### Example 5: Immunization of MC 38 neo-antigen peptides encapsulated polymersomes for cellular response

To observe a specific CD8 T cell response after immunization we used a MC-38 syngeneic tumour model. C57bl/6 mice were inoculated with subcutaneously at the right flank with MC-38 tumour cells (3 x 10⁵) in 0.1 ml of PBS for tumour development. The inoculation day is defined as Day 0. The animals were randomized based on the bodyweights and immunizations were started at day 4 after the inoculation. Immunizations consisted of: free peptides, ACM encapsulated peptides (polymersomes) with and without co-treatment with a commercially available anti-PD-1 antibody. Peptides were: Reps1 P45A (SEQ ID NO: 1), Adpgk R304M (SEQ ID NO: 2) and Dpagt1 V213L (SEQ ID NO: 3) and were obtained from Genscript. 200 ul of peptides and peptides in ACMs were immunized subcutaneously on day 4, 11 and 18. The concentration of peptides in ACMs was determined to be 20-30 µg/ml, whereas for peptides alone 10 µg per injection per mice was used. The anti-PD1 antibody was injected intraperitoneally on day 5, 8, 12, 15, 19 and 22 at 5mg/kg dosage. Animals were checked for any effects of tumour growth and treatments on normal behaviour such as mobility, food and water consumption, body weight gain/loss (body weights will be measured 3 times per week). Tumour sizes were measured 3 times per week in two dimensions using a caliper, and the volume was expressed in mm³ using the formula: V = 0.5 *a* x *b*² where a and b are the long and short diameters of the tumour, respectively.

### Example 6: Immunization of mice and pigs with PEDv spike protein encapsulated polymersomes

Mice were immunised with ACM encapsulated PEDv spike protein (as an illustrative example of a vaccine against a coronavirus) and boosted with a second dose after 21 days, 150 ul-200 µl of polymersomes encapsulated with PEDv Spike protein were immunized. Sera was collected from the final bleed and was used for ELISA. Moreover, these sera were tested for their ability to neutralise the PEDV *strain* USA/Colorado/2013 (CO/13) through a conventional virus neutralisation. Furthermore, weaned pigs were orally vaccinated with 1 ml of polymersome encapsulated with PED SPIKE protein (after a prime on day 1 and a boost on day 14). A simple physiological solution was used for the oral vaccination.

### Example 7: Immunization of mice with OVA protein encapsulated polymersomes together with adjuvant CpG (either co-injected) or CpG encapsulated polymersomes as prophylactic B16-OVA tumor model

Mice were administered with four different OVA protein immunization protocols: 1. free OVA with free CpG co-administered, 2. OVA encapsulated by BD21 polymersomes with free CpG co-administered, 3. free OVA with CpG encapsulated by BD21 polymersomes and 4. OVA encapsulated by BD21 polymersomes (representing a first population of polymersomes as used in the present invention) co-administered with a CpG encapsulated by BD21 polymersomes (representing a second population of polymersomes as used in the present invention) as a prime and boost subcutaneously to C57BI/6 mice 7 days apart followed by inoculation of 10⁵ B16-OVA cells on the right flank on the same side as immunizations. Tumor development was monitored for 30 days.

### Example 8: Immunization of mice with OVA protein encapsulated polymersomes together with adjuvant CpG (either co-injected) or CpG encapsulated polymersomes as therapeutic B16-OVA tumor model

Mice were inoculated with 10⁵ B16-OVA cells for tumor growth and three different OVA protein formulations (1. free OVA with CpG co-administered, 2. OVA encapsulated BD21 polymersomes and free CpG co-administered, 3. OVA encapsulated BD21 polymersomes (representing a first population of polymersomes as used in the present invention) with separate CpG encapsulated polymersomes (representing a second population of polymersomes as used in the present invention) were immunized as prime and 2 boosts (on day 5, day 10 and day 14) after inoculation of B16-OVA cells. All immunization samples consist of 5-10 µg of OVA, 8 µg of CpG per mice. Tumor development was monitored for more than 20 days and in order to directly correlate the tumor response for the different OVA formulations, blood samples were collected on day 20 for dextramer staining.

### Example 9: Immunization of mice with Trp2 peptide encapsulated polymersomes together with adjuvant CpG (either co-injected) or CpG encapsulated polymersomes as therapeutic melanoma B16F10 tumor model

10⁵ B16F10 cells were first inoculated into C57BI/6 mice and followed by the different Trp2 (tyrosinase related protein-2, as an antigen) formulations for immunization. All formulations consist of 16 µg of Trp2 peptide that was injected per mice. After Vaccination, the tumor growth was monitored and in order to directly correlate the tumor response for the different Trp2 formulations, tumor samples were collected on day 17 by sacrificing animals (n=4) and the blood samples on day 21 for the animals that were monitored for tumor growth.

### Example 10: Conjugation of CpG adjuvant to ACM Polymersomes

CpG ODN can be conjugated via either 5' or 3' end with a functional group. Amine (-NH₂) and free thiol (-SH) functional ODN can be custom synthesized in either 5' or 3' terminus. Three conjugation strategies described in more detail below can all be used to effectively conjugate an adjuvant such as CpG ODN to functional polymers and surface functional ACM particles. (1) SH-ODN/ACM - Maleimide conjugation, (2) NH₂-ODN/ACM - NHS (N-hydroxysuccinimidyl ester), (3) NH₂-ODN/ACM-Aldehyde. In addition to the covalent conjugation of ODN to ACM, hydrolyzable linkers or cleavable linkers can be introduced between ODN and polymer chain. Acid cleavable linker (hydrazone, oxime), enzyme cleavable linker (dipeptide-based linkers Val-Cit-PABC and Phe-Lys) or glutathione cleavable disulfide linker can be introduced to release CpG in the Antigen Presenting Cells.

**ACM-ODN conjugation strategy using SH-ODN and Polymer-Maleimide (Polymer-MAL):** The disulfide precursor to 5' sulfhydryl ISS CpG-ODN or 3' sulfhydryl ISS CpG-ODN was treated with 700 mM tris-(2-carboxyethyl) phosphine (TCEP) solution was made in HBSE (140 mM NaCl buffered with 10 mM HEPES containing 1 mM EDTA) pH 7, and used at a five molar excess to reduce disulfide-ODN at 40°C for 2 h. Residual TCEP was removed using a PD-10 desalting column (GE Healthcare) and eluted in HBSE pH 6.5. Reduced SH-ODN was used immediately or stored at -80°C until use. Polymer-MAL was prepared beforehand using amine function polymer and NHS-PEG-MAL linker group. ACM-ODN complex can be prepared either pre-conjugating ODN to polymer then form ACM or conjugation of ODN on pre-formed ACM. For pre-conjugation of SH-ODN and polymer-MAL can be done in presence of DMF in HBSE buffer, pH 7 at 40 °C for 4 hr in dark or via water-in-oil emulsion (HBSE buffer: ether, 2:1 ratio) at 40 °C for 4 hr in dark. The organic solvents and water were removed by rotor evaporator followed by lyophilization. Dry ODN-polymer was used to form ACM upon mixing with a non-functional polymer. For pre-formed ACM-MAL was prepared using 10-20% function Polymer-MAL with 80-90% non-functional polymer via thin-film rehydration technique, rehydrated in HBSE buffer, pH 7. Reduced SH-ODN was conjugated with pre-formed ACM-MAL in HBSE buffer, pH 7 at 40 °C for 4 hr. Unconjugated SH-ODN was removed from ACM-ODN conjugates by Sepharose CL-4B size-exclusion chromatography or via dialysis.

**ACM-ODN conjugation strategy using NH2-ODN and Polymer- N-hydroxysuccinimidyl ester (Polymer-NHS):** The amine functional 5' CpG-ODN or 3' CpG-ODN was conjugated with N-hydroxysuccinimidyl ester functionalized polymer (polymer-NHS). Polymer-NHS was prepared beforehand from hydroxyl function polymer and N,N'-Disuccinimidyl carbonate in presence of DMAP under dry acetone/dioxane mixture.

ACM-ODN complex can be prepared either pre-conjugating ODN to polymer then form ACM or conjugation of ODN on pre-formed ACM. For pre-conjugation of NH2-ODN and polymer-NHS can be done in the presence of dry DMF at room temperature for 8 hr. The organic solvent was removed by lyophilization. Dry ODN-polymer was used to form ACM-ODN upon mixing with non-functionalized polymer via thin-film rehydration technique.

For pre-formed ACM-NHS was prepared using 20-30% function Polymer-NHS with 70-80% non-functional polymer via thin-film rehydration technique in phosphate buffer, pH 6.8. NH2-ODN was added to the pre-formed ACM-NHS in PB buffer, pH 6.8 at 4 °C and react overnight. Unconjugated NH2-ODN was removed from ACM-ODN conjugates by Sepharose CL-4B size-exclusion chromatography or via dialysis.

### ACM-ODN conjugation strategy using NH2-ODN and Polymer-Aldehyde (Polymer-CHO):

The amine functional 5' CpG-ODN or 3' CpG-ODN was conjugated with aldehyde functionalized polymer (polymer-CHO) to form imine bond which further reduced to stable amine bond formation by sodium cyanoborohydride (NaCNBH₄) treatment. Polymer-CHO was prepared beforehand from hydroxyl function polymer by selective oxidation of alcohol to aldehyde in the presence of Dess-Martin periodinane.

ACM-ODN complex can be prepared either pre-conjugating ODN to polymer then form ACM or conjugation of ODN on pre-formed ACM.

For pre-conjugation of NH2-ODN and polymer-CHO can be done in the presence of dry DMF at room temperature for 16 hr which give rise to imine bond formation which further reduced to an amine by NaCNBH₄. Residual NaCNBH4 was removed using a PD-10 desalting column (GE Healthcare) and eluted in water/DMF mixture. The organic solvent was removed by lyophilization. Dry ODN-Polymer was used to form ACM-ODN upon mixing with non-function polymer via thin-film rehydration technique.

For pre-formed ACM-CHO was prepared using 30-40% functional Polymer-CHO with 60-70% non-functional polymer via thin-film rehydration technique, rehydrated in 10 mM borate buffer, pH 8.2. NH2-ODN was added to pre-formed ACM-CHO in borate buffer, pH 8.2 and react overnight at room temperature for form imine bond. Further imine bond reduced to a stable amine bond upon NaCNBH₄ treatment at 4 °C overnight. Unconjugated NH2-ODN and free NaCNBH₄ were removed from ACM-ODN conjugates by Sepharose CL-4B size-exclusion chromatography or via dialysis.

### Conjugation of BD21 vesicles to Ovalbumin (OVA):

### BD₂₁ + 5% DSPE-PEG(3000)-Maleimide Vesicles formation:

100 µL of BD₂₁ (100 mg/mL) in CHCl₃ was transferred to 25 mL of single-neck RBF (round bottom flask) to which was added 80.89 µL of DSPE-PEG-Maleimide (10 mg/mL in CHCl₃). The solvent was slowly evaporated under reduced pressure at 35°C to get wide-spread thin-film and was dried in desiccator under vacuum for 6 hours. 1 mL of NaHCO₃ buffer (10 mM, 0.9% NaCl, pH 6.5) was added to the thin-film for rehydration and stirred at 25°C for 16-20 hours to form milky homogeneous solution. After rehydration for 16-20 hours, the solution was extruded with 200 nm Whatman membrane at 25°C for 21 times. The solution was transferred to dialysis bag (MWCO (weight cut-off): 300 KD) and dialyzed in NaHCO₃ buffer (10 mM, 0.9% NaCl, pH 6.5) (2 x 500 mL and 1 x 1L; first two dialysis were done for 3 hours each and the last one for 16 hours). Vesicle size and mono-dispersity was characterized by dynamic light scattering Instrument (Malvern, United Kingdom) (100x dilution with 1x PBS).

### Conjugation of BD₂₁ + DSPE-PEG(3000)-maleimide (5%) to OVA:

OVA (0.5 mg) was dissolved in 200 µL of NaHCO₃ buffer (10 mM, 0.9% NaCl, pH 6.5) to which was added 2.5 mg of TCEP-HCl (dissolved in 100 µL of same NaHCO₃ buffer) and incubated for 20 minutes. pH of the reaction was adjusted from ~2.0 to 6-7 using 1N NaOH solution (~10 µL). 350 µL of polymersomes (10 mg/mL of BD/DSPE-PEG(3000)-Maleimide 5% in 10 Mm NaHCO₃, 0.9% NaCl buffer, pH 7.0) was then added to the protein mix and pH of the reaction was adjusted again to pH 7.0 (if pH of reaction was not 7). Reaction was incubated at 24°C for 3 hours away from light. The reaction solution (~660 µL) was transferred to dialysis bag (MWCO: 1000 KD) and dialyzed in NaHCO₃ buffer (10 mM, 0.9% NaCl, pH 7.0) (3 x 1L; first two dialysis were done for 3 hours each and the last one for 16 hours). 100 µL of dialyzed solution was purified through SEC chromatography and collected in 96-well plate. The corresponding ACM peak fractions were combined and lyophilized for quantification by SDS-PAGE.

For comparison, OVA was also encapsulated in BD₂, alone. For this a film was produced as above using 100µl of a 100mg/ml BD₂₁ stock dissolved in CHCl₃. Rehydration was then performed by adding 1 mL solution of 0.5 mg/ml solubilized OVA protein in 1X PBS buffer. The mixture was stirred at 600 rpm, 4°C for at least 18 hours to allow the formation of polymer vesicles, extruded and dialyzed as above.

### Conjugation of BD₂₁ vesicles to Hemagglutinin (HA):

### Preparation of BD₂₁-CHO from BD₂₁:

To a stirred solution of BD₂₁ (100 mg) in single-neck RBF was dissolved in anhydrous CH₂Cl₂ (6 mL) and was added Dess-Martin periodinane (10 mg, 0.4 equiv) at 0°C in one-portion. Reaction was stirred at 25°C for 4 hours. Then 1:1 mixture of saturated NaHCO₃ and Na₂S₂O₃ (20 mL) was added and stirred at the same temperature for 2 hours. Organic layers was separated, and the aqueous layers was extracted with CH₂Cl₂ (20 mL) and separated the organic layer. The combined organic layers were washed with 1:1 mixture of sat. NaHCO₃ and Na₂S₂O₃ (20 mL), brine (20 mL), dried over anhydrous Na₂SO₄ and evaporated under reduced pressure to get colourless viscous oil (100 mg, quantitative). Modification yield was estimated to be around 30% by NMR.

### Conjugation of BD-CHO to HA:

10 mg of modified BD₂₁-CHO (colourless viscous oil) was dissolved in 0.5 mL of CHCl₃ in 25 mL of single-neck RBF and slowly evaporated the solvent under reduced pressure using Rotavap at 35°C for 10 minutes to get wide spread thin-film. The film was dried under vacuum in desiccator for 6 hours. The film was rehydrated in 400µl of borate buffer (borate 10 mM, 150 mM NaCl, pH 7.5) for 30 minutes before adding 0.5 mg of HA (150µl of HA was prepared by pre-equilibrating it in borate buffer by dialysis). Reaction was stirred at 25°C for 16 hours. 20 µL of NaCNBH₄ was then added to the solution (preparation: 126 mg of NaCNBH₄ was dissolved in 1 mL of Millipore water and degassed the excess H₂ gas by stirring the solution at 25°C for 30 minutes) and kept on stirring at 25°C for another 8-16 hours. The conjugated polymersomes were extruded by using 200 nm Whatman membrane at 25°C for 21 times. The reaction solution was transferred to dialysis bag (MWCO: 1000 KD) and dialysed in PBS buffer (1x, pH 7.4) (3 x 1L; first two dialysis were done for 3 hours each and the last one for 16 hours). After dialysis, 400 µL of dialysed solution was purified through SEC chromatography (Size-exclusion chromatography) and collected in a 96-well plate. The presence of coupled HA was detected using both Western Blot and ELISA assays (Enzyme-linked Immunosorbent Assay). Vesicle size and mono-dispersity was characterized by dynamic light scattering (100x dilution with 1x PBS).

For comparison, HA was also encapsulated in BD₂₁ alone. For this a film was produced as above using 100µl of a 100mg/ml BD₂₁ stock dissolved in CHCl₃. Rehydration was then performed by adding 1 mL solution containing 20µg of HA protein in 1x PBS buffer. The mixture was stirred at 600 rpm, 4°C for at least 18 hours to allow the formation of polymer vesicles, extruded and dialyzed as above.

### Quantification of coupled HA and OVA:

To detect the presence of coupled proteins several techniques were used. 100 to 300µl of dialyzed sample was loaded onto a Size Exclusion Chromatography (SEC, Akta) using a Sephacryl column. SEC fractions corresponding to the peak of ACM vesicles were pooled or used as is to either be analysed by SDS-PAGE or/and ELISA. For SDS-PAGE, 20-40µl of each fraction was mixed DMSO (20% v/v) and vortexed thoroughly before adding loading buffer. Different amounts of free BSA (Bovine serum albumin), HA or OVA was added for quantification. After migration, the gel was either stained by sliver staining (OVA) or used for a membrane transfer and immunoblotting with rabbit polyclonal antibody (HA). To further ensure that HA was coupled to the polymer, 25ul of all SEC fractions was coated into a Maxisorp 384-well plate overnight at 4°C. After blocking with 3% BSA, rabbit polyclonal anti-HA antibody was used as primary antibody followed by HRP (horseradish peroxidase) coupled anti-rabbit as secondary. TMB substrate was added and reaction was stopped using 1M HCl. Optical densities were quantified at 450nm.

### Mouse immunizations and titer determination (mAb):

C57bl/6 mice were immunized with different OVA formulations: PBS (negative control), free OVA with or without Sigma Adjuvant System (SAS), OVA encapsulated ACMs or OVA conjugated ACMs. Balb/c mice were immunized with different HA formulations: PBS (negative control), free HA, HA encapsulated ACMs or HA conjugated ACMs. Both trials were performed by doing a prime and a boost 21 days later. All immunizations were performed with a same final amount of antigen within each trial: 5-10µg OVA/ injection/ mouse or 100-200ng HA/ injection/ mouse. Final bleeds were collected 42 days after prime. ELISA was then performed to assess titers: OVA or HA were coated onto MaxiSorp plates (1µg/ml in carbonate buffer) overnight. Plates were blocked using 3% BSA in PBS for 1h at RT. All sera were diluted at 1:100 and incubated on plates for 1h at RT. After 3 washes with PBS + 0.05% Tween 20, secondary antibody anti-mouse IgG HRP coupled was incubated at 1:10,000 dilution for 1h, RT (room temperature). After 3 washes with PBS/ Tween 20 buffer, TMB substrate was added and reaction was stopped using 1M HCl. Optical densities were quantified at 450 nm.

### Example 11: ACM polymersomes coupling to OVA

Polymersomes (also called ACMs (artificial cell membranes) prepared with 5% DSPE-PEG(3000)-Maleimide were used to couple OVA through available cysteines. At least one cysteine has been shown to be accessible to solvent (Tatsumi et al., 1997). Coupling conditions were achieved in pH-controlled environment.

### Example 12: BD₂₁-CHO polymersomes coupling to HA

BD₂₁ polymer was modified as described in the methods and the aldehyde modification percentage was estimated to be around 30-40% by NMR. The aldehyde moiety added to the BD₂₁ will react with the primary amines of HA's lysine and arginine residues. After overnight coupling followed by extensive dialysis, the resulting vesicles were characterized.

### Example 13: Immunizations and sera tittering

C57bl/6 mice were immunized with the following formulations: a negative control (PBS), free OVA with or without Sigma Adjuvant System (SAS), BD₂₁ encapsulated OVA and BD₂₁ conjugated OVA. All immunizations had a same amount of 4µg of OVA per injection and per mouse. 21 days after the boost, sera were collected for tittering by ELISA.

In addition, Balb/c mice were immunized with the following formulations: a negative control (PBS), free HA, BD₂, encapsulated HA and BD₂₁ conjugated HA. Since some residual free HA was observable in the HA conjugated polymersome sample even after extensive dialysis, pooled fractions of SEC were used for immunizations. All immunizations had a same amount of 100-200ng of HA per injection and per mouse.

### Results

### Encapsulation of proteins and DNA of Example 1:

OVA encapsulated polymersomes were purified by dialysis and size exclusion column (SEC) to remove the non-encapsulated proteins and analysed by dynamic light scattering. As shown in **Figs. 2A****,** **Figs. 3** and **4****,** an elution profile of OVA encapsulated polymersomes from SEC and a monodisperse population was observed.

Dynamic light scattering (DLS) data is presented in **Fig. 2B** for various of different polymersomes having encapsulated therein OVA, PEDv SPIKE protein or eGFP DNA. They are all measuring a mean diameter of 120 nm- 180 nm using Z-average (d, nm), a preferred DLS parameter. Z-average size is the intensity weighted harmonic mean particle diameter, the values are in good agreement with earlier data [Fu et al., 2011, Lim. S.K, et al., 2017] of polymersomes.

### Encapsulated eGFP DNA and transfection data of Example 2:

eGFP DNA encapsulated polymersomes were transfected with HEK293T cells and after transfection, the uptake of ACM polymersomes were measured by fluorescence plate reader at Ex 530 nm and Em 560 nm and the transfection efficiency was measured by the GFP fluorescence (Ex 485 nm, Em 520 nm). As shown in **Fig.5****,** it is clear that polymersomes with DNA are able to penetrate into the cells and releasing the DNA to express the DNA to protein, all the polymersome formulations are taken up the cells and are able to release the DNA, whereas the ratio of the polymersomes release versus the protein expression correlates well with its stability and biodegradability **(****Fig. 5A****).** Non-biodegradable polymersomes such as BD21 aretaken up in smaller amount and the expression levels were lower comparing to the biodegradable polymersomes. Similar results were observed from the fluorescence images of cells as well **(****Fig.5B** **&** **Fig.5C****).**

### Encapsulated OVA and titers of Example 3:

OVA encapsulated polymersomes were immunized in C57bl/ 6 mice by doing a prime and a boost 21 days later. Final bleeds were used for performing the ELISA. As shown in **Fig. 6****,** it is clear that OVA encapsulated polymersomes is the only formulation able to trigger a titer in comparison to free OVA, OVA with adjuvants or control samples (PBS alone). The reason why OVA with SAS did not produce a titer may be due to the small amount of OVA used in the trial (around 5ug per injection). Hence ACM encapsulated OVA was able to trigger a B cell response toward OVA in the form of an IgG serum titer specific for Ovalbumin.

### Encapsulated HA and titers of Example 4:

HA (H1N1/A/Puerto Rico/8/1934 strain, SEQ ID NO: 7) encapsulated polymersomes were immunized in Balb/c mice by doing a prime and a boost 21 days later. Final bleeds were used for performing the ELISA. As shown in **Fig. 7****,** it is clear that HA encapsulated polymersomes is the only formulation able to trigger a titer in comparison to free HA or control samples (PBS alone). The reason why free HA did not produce a titer may be due to the small amount of HA used in the trial (around 100ng per injection). Hence ACM encapsulated HA was able to trigger a B cell response toward HA in the form of an IgG serum titer specific for HA.

### Encapsulated MC-38 neo antigen peptides and CD8 T cell response of Example 5:

In order to show that ACM encapsulated antigen are able to trigger a CD8 T cell response we used a well-defined MC-38 syngeneic mouse tumour model which relies on the delivery of known CD8 antigenic peptides. High quantities of these peptides combined with adjuvants have been shown to trigger tumour control in therapeutic mouse models (e.g., Kuai et al., 2017, Luo et al., 2017). In addition, these effects were clearly correlated to the presence of peptide-specific CD8 T cells in the mouse blood. Hence any tumour development difference between groups would be directly attributed to the presence of a peptide-specific pool of CD8 T cells. 4 days after inoculation with MC-38 cell lines, mice were immunized with either free peptides, ACM encapsulated peptides (polymersomes) with and without anti-PD1 antibody treatment as described in the section Materials and Methods herein. As shown in **Fig. 8****,** immunization with encapsulated peptides was able to trigger an inhibitory effect in tumour development compared to free peptides. This effect was dramatically potentiated whenever anti-PD1 antibody injections were added. This data demonstrated that ACM encapsulated peptides (polymersomes) were able to trigger a peptide-specific CD8 T cell response most likely via the delivery of these peptides to dendritic cells, which resulted in tumour control. This effect was increased by addition of a checkpoint inhibitor such the anti-PD1 antibody. Indeed, MC-38 has been shown to express PD-L1 molecule at their cell surface which is known to inhibit T cells killing activity inside tumours. Hence inhibition of such interaction by an antibody blocking PD1/ PD-L1 interaction is known to reveal the presence of tumour specific T cells even further. Altogether this data clearly demonstrates that ACM encapsulated antigens (polymersomes) were able to trigger an antigen specific CD8 T cell response without addition of adjuvant.

### Encapsulated PEDv spike protein and IgG, IgA and virus neutralisation response of Example 6:

Mice were immunised with ACM encapsulated PEDv spike protein and boosted with a second dose after 21 days. Sera was collected from the final bleed and was used for ELISA. As can be seen in **Fig. 8****,** antibodies that bind to SPIKE Protein coated on ELISA PLATE and the titers are of similar level to the animals vaccinated with killed virus in comparison with ACM vaccinated mice. Moreover, the sera were tested for their ability to neutralise the PEDV *strain* USA/Colorado/2013 (CO/13) through a conventional virus neutralisation experiment **(****Fig. 9****).** In here, it was observed that the virus neutralization occurs only for the sera from the mice immunized with ACM vaccine (i.e., ACM encapsulated PED Spike protein) while no neutralization was observed for the sera from the mice vaccinated with killed virus. Furthermore, different polymersomes (e.g., BD21, PDMS₄₆-PEO₃₇ (marked in Figure 10 only as "PDMS"), PDMS₄₆-PEO₃₇/DSPE-PEG, PLA-PEG/Asolectin lipids) encapsulated with full length soluble protein and polymersomes mixture containing S1 and S2 region were immunized and the sera were tested for virus neutralization **(****Fig. 10****).** From **Fig. 10****,** it is evident that the groups of mice immunized with PBS sample does not show any virus neutralization, whereas all other polymersome formulation shows varying degree of virus neutralization. Furthermore, when weaned pigs were orally vaccinated with ACM encapsulated PED SPIKE protein (after a prime on day 1 and a boost on day 14, an increase in specific IgA antibodies against the virus was observed from the faecal swabs collected and measured via ELISA (see **Fig. 11**).

### Encapsulated OVA and CpG encapsulated Polymersome formulation and its effect on tumor growth in a prophylactic B16-OVA model of Example 7

Mice were administered with different formulations as a prime and boost subcutaneously to C57BI/6 mice 7 days apart followed by inoculation of 10⁵ B16-OVA cells on the right flank on the same side as immunizations. In all groups but the PBS control group, CpG was used as an adjuvant. All mice immunized with PBS control developed tumors **(****Fig.13A and 13B****).** Mice receiving soluble OVA tend also developed tumors although there was a clear effect from the immunizations. In ACM-OVA group the development of tumors was even further delayed due the targeting effect of ACMs. Even more strikingly, the groups in which OVA and CpG were co-encapsulated in the same polymersome or encapsulated separately (i.e. in two separate BD21 polymersome populations and then co-administered) the mice never developed any tumors **(****Fig. 13B****).** The figure legends used here (and where applicable in other figures) are as follows: "PBS" = a Phosphate-Buffered Saline control, "Free OVA + CpG" = free OVA co-administered with free CpG, "ACM-OVA + CpG" = OVA encapsulated by BD21 polymersomes co-administered with free CpG, "ACM-OVA-CpG" = free OVA with CpG co-encapsulated by BD21 polymersomes and "ACM-OVA + ACM-CpG" = OVA encapsulated by BD21 polymersomes (representing a first polymersome population of the invention) co-administered together with a separate population of CpG encapsulated by BD21 polymersomes (representing a second polymersome population of the invention). These results suggest that ACM formulated antigen together with or in parallel to an adjuvant such as CpG is creating a much bigger pool of T cells which is able to efficiently kill tumor cells.

### Encapsulated OVA and CpG encapsulated Polymersome formulation and its effect on tumor growth in a therapeutic B16-OVA model of Example 8

Mice were treated as given in Example 8. In this experiment, CpG was used as an adjuvant in groups except the PBS control. Subcutaneous immunization of soluble OVA as well as ACM-OVA **(****Fig. 14A and 14B****)** were able to delay the appearance of tumor compared to PBS control group although both groups did not improve the overall mice survival. However, the groups in which free OVA co-administered with ACM-CpG (CpG encapsulated in BD21 polymersomes) and OVA encapsulated ACM (OVA encapsulated in BD21 polymersomes, representing a first polymersome population of the invention) co-administered with CpG encapsulated ACMs (representing a second polymersome population of the invention) were slow in delaying the tumor and 4 mice out of 8 remained tumor free 39 days (data not shown). These results suggest that CpG in ACMs has improved the immunogenicity of the ACM encapsulated antigen. In order to directly correlate the above data with the presence of OVA specific T cells, blood samples were collected on day 20 for dextramer staining **(****Fig. 14C****).** In correlation with the results obtained for tumor load only the groups with either ACM encapsulated OVA or ACM encapsulated OVA in combination with ACM encapsulated CpG shows significant dextramer staining. Furthermore, only for the group administered with ACM encapsulated OVA (OVA encapsulated in BD21 polymersomes) and separate ACM encapsulated CpG (CpG encapsulated in BD21 polymersomes) correlates both the tumor load and dextramer staining. The figure legends in **Fig. 14** are the same as those described for **Fig. 13****.**

### Encapsulated Trp2 and CpG encapsulated Polymersome formulation and its effect on tumor growth in a therapeutic melanoma B16F10 model of Example 9

Mice were treated with different ACM formulations in B16F10 tumor model in which tumorigenicity relies on endogenously expressed tumor peptide antigens. Therefore the peptide of SEQ ID NO: 9 that has already been described to be immunogenic in this model (tyrosinase related protein-2, Trp2) was chosen as an antigen for immunization. 10⁵ B16F10 cells were first inoculated into C57BI/6 mice and followed by the immunizations with the following different formulations: 1. PBS, 2. free Trp2 co-administered with CpG (figure legend "Free Trp2 + CpG"), 3. ACM (BD21) encapsulated Trp2 co-administered with free CpG (figure legend "ACM Trp2 + CpG"), 4. free Trp2 co-administered with ACM (BD21) encapsulated CpG (figure legend "Free Trp2 + ACM-CpG") and 5. ACM (BD21) encapsulated Trp2 co-administered with a separate population of ACM (BD21) encapsulated CpG (figure legend "ACM-Trp2 + ACM-CpG") groups were monitored for tumor growth **(****Fig. 15A****).** From all the groups, mice treated with ACM encapsulated Trp2 co-administered with CpG and mice treated with ACM encapsulated Trp2 co-administered with separate ACM encapsulated CpG showed a much stronger tumor response. This correlates well with CD8 tumor cells on day 17 in blood **(****Fig. 15B****)** and CD8 T cells infiltration in tumors **(****Fig. 15C****).**

### ACM polymersomes coupling to OVA of Example 11:

**Figure 16** shows the Dynamic Light Scattering (DLS) profile from OVA coupled polymersomes which is matching standard features of these exemplary polymersomes of the invention (average (mean) size of the population/collection of polymersomes: 152nm; pdi: 0.229).

After extensive dialysis, 100µl of sample was separated using SEC **(****Fig. 17A****)** and 48 fractions of around 180µl were collected. Pooled fractions corresponding to the peak were lyophilized and resuspended into 500 µl. 20ul was loaded onto an SDS-PAGE together with some BSA standards **(****Fig. 17B****).** A band at the size corresponding to OVA protein was detected suggesting that OVA was successfully coupled to ACMs vesicles. The amount of coupled OVA was estimated to be around 20µg/ml. Notably, the BD₂₁ coupling to OVA protein did not modify its migration properties as seen for HA (see below). This is probably due to the fact that OVA is likely to be modified only at one cysteine residue per OVA protein, all other five cysteines being either buried or engaged in a disulfide bound.

### BD₂₁-CHO polymersomes coupling to HA of Example 12

DLS showed a slightly smaller size (average size: 104 nm) and acceptable pdi (pdi: 0.191) **(****Fig. 18****).**

400µl of the final product were separated by SEC as above (see **Fig. 19****, light gray trace).** Fractions corresponding to the peak were loaded individually onto an SDS-PAGE followed by membrane transfer for immunoblotting. A band with a high molecular weight was detected and seemed to decrease in later fractions outside the peak suggesting that this band corresponds to the conjugated HA. The observed high molecular weight could be due to the numerous polymer molecules coupled to the HA increasing its molecular weight. In addition, covalently bound polymer could partially compete with the binding of SDS of the loading buffer decreasing the final charges state compared to free HA. With a lower negative charge, one would expect conjugated HA protein to migrate less which would result in an apparent higher molecular weight. The dialyzed sample (non-separated on SEC) shows residual free HA probably coming from aggregated HA that could not be dialyzed. Concentration of conjugated HA was determined to around 1µg/ml.

To ascertain that HA proteins are accessible at the surface of particles, ELISA was conducted on all collected fractions coated overnight on a Maxisorp plate able to trap BD₂₁ vesicles. HA protein was clearly detected and when the ELISA profile was superimposed on the SEC, both profiles nicely correlated **(****Fig. 20****, black trace)** confirming that HA was coupled to BD₂, and was accessible to an antibody detection.

### Immunizations and sera tittering of Example 13:

As shown in **Figure 21****,** free OVA with or without adjuvant was not able to elicit an IgG response. Interestingly, at similar dose conjugated OVA was able to trigger a lot stronger titer response than encapsulated OVA.

Balb/c mice were immunized with the following formulations: a negative control (PBS), free HA, BD₂, encapsulated HA and BD₂₁ conjugated HA. Since some residual free HA was observable in the HA conjugated polymersome sample even after extensive dialysis, pooled fractions of SEC were used for immunizations. All immunizations had a same amount of 100-200ng of HA per injection and per mouse. As shown in **Figure 22****,** free HA was not able to elicit an IgG response which was expected given the low amount of HA injected. Conjugated HA was able to trigger a slightly higher response than encapsulated HA in this case. By adding a second population of polymersomes associated with an adjuvant as illustrated in Examples 7 to 9 (for adjuvant encapsulation) or Example 10 (for adjuvant conjugation) the responses should be even higher.

### Example 14: Immunization of guinea pigs with PEDv spike protein encapsulated polymersomes by different routes of administration

The PEDv spike protein was expressed using the baculovirus system. The cell solution was clarified, and ACM polymers were added along with required additives to encapsulate the proteins of interest. The encapsulation was conducted as described in Example 1. CpG was also encapsulated as described in Example 1, using CpG ODN 2007 (5'- TCG TCG TTG TCG TTT TGT CGT T -3', SEQ ID NO: 63, commercially available from InvivoGen under catalogue number tlrl-2007).

Guinea pigs (N=4 for I.M. ;=5 for other groups) were immunised in 3 different ways, oral, nasal, and I.M. Each method was dosed with 40 µl of a 1:1 mixture of ACM encapsulated PEDv spike protein and ACM encapsulated CpG. and boosted with a second dose after 21 days. Sera was collected from the final bleed and was used for ELISA (Data not shown). Moreover, these sera were tested for their ability to neutralise the PEDV strain USA/Colorado/2013 (CO/13) through a conventional virus neutralisation **(****Fig. 25****).**

### Example 15: Immunization of mice with MERS spike protein encapsulated polymersomes

The soluble fragment of the MERS-CoV spike protein (SEQ ID NO: 43, corresponding to positions 1-1297 of UniProtKB accession no. K0BRG7) was expressed using the baculovirus system and purified. A thin film of 10 mg of BD21 polymer was formed in a 10 ml round bottom flask and exhaustively dried. 1 ml of the protein solution was added to the round bottom flask and spun on a rotary evaporator at 150 rpm for 4 hours. The sample was removed from the flask and extruded through a 400 nm filter followed by a 200 nm filter. The extruded sample containing ACM-proteins and free protein was then separated using size exclusion chromatography. The fractions corresponding to the ACM/protein fractions were collected and used for immunisation into mice. C57bl/6 mice were immunized using encapsulated ACM-MERS-CoV and control ACMs by doing a prime and a boost 21 days later. Final bleeds were collected 42 days after prime. ELISA was then performed to assess titers: MERS-CoV was coated onto Maxisorp plates (1ug/ml) overnight. Plates were blocked using 3%BSA for 1h at RT. All sera were diluted at 1:100 and incubated on plates for 1h at RT. After 3 washes with PBS + 0.05% Tween 20, secondary antibody anti-mouse HRP was incubated at 1:10,000 dilution for 1h, RT. TMB substrate was added and reaction was stopped using 1M HCl. Optical densities were quantified at 450nm **(****Fig. 26 A****).** All serum samples were tested for MERS-CoV neutralizing antibodies using plaque reduction neutralization assay (PRNT) **(****Fig. 26B****).**

### Example 16: Immunization of mice with different domains of the PED_{V} spike protein

The different spike protein domains were expressed in the baculovirus system (Baculo). The cell cultures were clarified, and the solution used for ACM formation. PEDv spike protein S1 domain, S2 domain, and a mixture of S1 and S2 domains was used for immunisation without adjuvants I.M. with a 200 µl dose into Balb/c female mice aged 6-8 weeks old (n=5).

The animals were boosted with a second dose after 21 days. Sera was collected from the final bleed and was used for ELISA. Moreover, these sera were tested for their ability to neutralise the PEDV strain USA/Colorado/2013 (CO/13) through a conventional virus neutralisation **(****Fig. 27****).**

### Example 17: Expression and purification of SPIKE Protein SARS-CoV-2 using Baculovirus expressions system

Soluble fragments of the SARS-CoV-2 spike proteins (SEQ ID NO: 36, 40 and 65) were expressed using the baculovirus system and purified from the media using traditional Ni-NTA affinity purification. A thin film of 10 mg BD21 polymer was formed in a 10 ml round bottom flask and exhaustively dried. 1 ml of the protein solution was added to the round bottom flask and spun on a rotary evaporator at 150 rpm for 4 hours. The sample was removed from the flask and extruded through a 400 nm filter followed by a 200 nm filter. The extruded sample containing ACM-proteins and free protein was then separated using size exclusion chromatography.

### Example 18: Immunization of mice with different domains of the SARS-CoV-2 spike protein

In a first study, ACM having encapsulated S1-S2 region (SEQ ID NO: 36) with or without adjuvant were employed. In case of adjuvant, ACM encapsulated SPIKE protein was mixed with 1:1 ratio of Sigma Adjuvant System (an oil in water emulsion consists of 0.5 mg Monophosphoryl Lipid A (detoxified endotoxin) from Salmonella Minnesota and 0.5 mg synthetic Trehalose Dicorynomycolate in 2% oil (squalene)-Tween 80-water. ACM having encapsulated S1-S2 region (SEQ ID NO: 36) with or without adjuvant were compared with ACM having encapsulated S2 region (SEQ ID NO: 40) with adjuvant and a PBS control.

Mice were immunized using encapsulated ACM-SARS-CoV2 and control ACMs by doing a prime and a boost 21 days later. Final bleeds were collected 35 days after prime **(****Fig. 23 B****).** ELISA was then performed to assess IgG antibody titers against SARS-CoV-2. **Fig. 23C** shows the IgG titres measured in Balb/c mice at day 35 that were immunized with the following formulations: BD21 polymersome encapsulated soluble S1 and S2 segments with co-administered adjuvant (Group 1), BD21 polymersome encapsulated soluble S1 and S2 segments (Group 2), BD21 polymersome encapsulated soluble S2 segment co-administered with adjuvant (Group 3), and PBS as negative control (Group 4). The highest IgG1 titers were observed for vaccination by BD21 polymersomes having encapsulated soluble S1 and S2 segments or by BD21 polymersomes having encapsulated soluble S2 segment co-administered with adjuvant (Group 1 and Group 3, respectively), while the immune response induced by administration of soluble S1 and S2 segments being encapsulated in BD21 polymersomes (Group 2) alone without adjuvant was lower.

In addition, SARS-CoV-2 neutralizing antibodies will be assessed using plaque reduction neutralization assay (PRNT).

In a second study, different modes of administration, i.e. IM and IN of ACM having encapsulated S1-S2 region (SEQ ID NO: 65), ACM having encapsulated S2 region (SEQ ID NO: 40), either alone or in combination with ACM encapsulated CpG were compared.

Mice were immunized using encapsulated ACM-SARS-CoV2 and control ACMs by doing a prime and a boost 14 days later. Final bleeds were collected 56 days after prime. ELISA was then performed to assess antibody titers against SARS-CoV-2. In addition, SARS-CoV-2 neutralizing antibodies will be assessed using plaque reduction neutralization assay (PRNT). Furthermore, Bronchoalveolar Lavage fluid (BALF) will be collected by washing the lung airways. BALF will be used to measure secretory IgA and neutralization antibodies. For neutralization assay, SARS-CoV-2 pseudovirus will be incubated with serially diluted sera or BALFs.

### Example 19: Immunization of mice with ACM-encapsulated SARS-CoV-2 spike protein and ACM-encapsulated CpG adjuvant

In this experiment BD21 encapsulated SARS-CoV-2 spike protein, with or without the use of CpG adjuvant was tested as vaccine. For this experiment, the full length soluble SARS-CoV-2 spike protein (SEQ ID NO: 65) which was produced as in the baculovirus/insect cell system was used. The protein was purified from the media using a combination of tangential flow filtration and Ion exchange chromatography. To determine the effect of the encapsulation on the immunogenicity of the spike protein antigen as well as CpG adjuvant, the following formulations were prepared: i) free recombinant spike protein (SEQ ID NO: 65, "fSpike); ii) BD21 polymersome-encapsulated spike protein ("ACM-Spike"); iii) a mixture of free spike protein and free CpG adjuvant ("fSpike fCpG); iv) a mixture of BD21 polymersome-encapsulated spike protein and BD21 polymersome-encapsulated CpG (ACM-Spike ACM-CpG).

Thereafter, 6-8 weeks old female C57BL/6 mice were immunized via subcutaneous route on days 0 and 14 (cf. **Fig. 24A**) with the four formulations. Blood was collected on day 28 to assess antibody titers against SARS-CoV2 spike protein. Compared to PBS negative controls, clear increases in serum IgG were observed in all immunized mice, indicating seroconversion **(****Fig. 24B****).** Between fSpike and BD21 polymersome encapsulated Spike groups, a trend of increased IgG titer was seen in the latter, suggesting the benefit of polymersome encapsulation on improving the immunogenicity of the spike protein. While co-administration of fSpike and fCpG resulted in higher IgG titer compared to fSpike alone, **Fig. 24B** shows that further improvement in terms of the magnitude as well as uniformity of the antibody response was achieved via co-administration of polymersome encapsulated spike protein (as a first population of polymersomes), and polymersome encapsulated CpG (adjuvant as a second population of polymersomes). Altogether, these data suggest that co-administration of polymersome encapsulated spike protein as antigen and polymersome encapsulated CpG as adjuvant conferred measurable benefits compared to the non-encapsulated material, delivered alone or in combination.

### Example 20: Comparing adjuvant activity of ACM polymersomes with CpG

It is known that CpG can induce cytokine production by BALB/c splenocytes *in vitro.* In this experiment adjuvant effect of ACM polymersomes with CpG were studied. The experimental procedure included:
1. Spleens harvested from three C57BL/6 mice
2. 3 million splenocytes added to each well of 96-well plate in complete RPMI.
3. The following treatment groups were used: untreated, free CpG, ACM-CpG and empty ACM, which were incubated approximately 20 h at 37°C 5% CO2.
4. Collection of culture supernatants and measurement of IL-6 and IL-12p70 which are indicators of adjuvant effect.
5. The following 96-well plate format was used for measuments (ms) carried out in triplicates (Table 1)

**Table 1. 96-well plate format used (Eq. conc means equivalent concentration)**

| fCpG | | | ACM-CpG | | | ACM only | | |
|---|---|---|---|---|---|---|---|---|
| Ms1 | Ms2 | Ms3 | Ms1 | Ms2 | Ms3 | Ms1 | Ms2 | Ms3 |
| 0µM | 0µM | 0µM | | | | | | |
| 0.125 µM | 0.125 µM | 0.125 µM | 0.125 µM | 0.125 µM | 0.125 µM | Eq. conc (0.125 µM) | | |
| 0.25 µM | 0.25 µM | 0.25 µM | 0.25 µM | 0.25 µM | 0.25 µM | Eq. conc (0.25µM) | | |
| 0.5 µM | 0.5 µM | 0.5 µM | 0.5 µM | 0.5 µM | 0.5 µM | Eq. conc (0.5µM) | | |

### Results:

As shown in Figure 28 the IL-6 production was only detected in free CpG- or ACM-CpG-treated splenocytes (C57BL/6); ACM alone did not trigger IL-6 hence suggesting it lacked adjuvant property. Surprisingly, the signal for IL-6 was very strong, whereas IL-12 was not detected even in neat culture supernatant.

### Conclusions:

From the prior art it is known that, BALB/c splenocytes respond quickly with IL-6 and IL-12 after CpG stimulation; whereas IFNγ production takes longer.

In this study, IL-6 production detected only in free CpG- or ACM-CpG-treated splenocytes (C57BL/6); ACM alone did not trigger IL-6 hence suggesting it lacked adjuvant property.

Unlike BALB/c splenocytes, C57BL/6 splenocytes did not seem to release IL-12 after stimulation.

### Example 21: ACM encapsulation enhanced the biological function of CpG and ACM-CpG exhibited superior adjuvant activity compared to free CpG

Class B CpG binds endosomal Toll-like receptor 9 (TLR9) to induce several immunological effects, including activation of dendritic cells (DCs), production of pro-inflammatory cytokines and B cell differentiation and antibody secretion. These attributes make class B CpG valuable as a vaccine adjuvant. In this study, C57BL/6 mice were subcutaneously (SC) injected with 5 µg free murine CpG 1826 or ACM-CpG 1826 to compare their relative abilities to activate classical dendritic cells (cDCs). Two days after, inguinal lymph nodes (which drain the site of injection) were harvested to assess DC activation. Mice injected with empty ACM polymersomes did not upregulate CD86 or CD80 activation marker on cDC1 (Fig. 29a, c) or cDC2 (Fig. 29b, d), when compared against PBS controls, indicating the non-immunogenic nature of ACM polymersomes. Administration of free CpG induced significant increase in CD86 and CD80 expression on cDC2, compared to PBS or empty ACM controls (Fig. 39b, d), but not cDC1 (Fig. 29a, c). In contrast, administration of ACM-CpG significantly upregulated CD86 and CD80 on cDC2 (Fig. 29b, d) and cDC1 (Fig. 29a, c). Altogether, the data demonstrated that the effect of free CpG was restricted to cDC2 whereas ACM-CpG could activate both DC subsets, indicating superior adjuvant activity. Moreover, ACM polymersomes alone were non-immunogenic and thus enhanced DC activation was due to efficient delivery of CpG to endosomal TLR9.

### Example 22: ACM-CpG induced broader cytokine profile than free CpG

The outcome of TLR9 activation by CpG depends on the class of the agonist. Class A CpG possesses a multimeric structure that enables signalling through the IRF7 pathway, which results in production of IFNα alongside IL-6. Class B CpG, which include murine CpG 1826 and human CpG 7909, is monomeric and signals via the NFκB pathway instead to produce IL-6 but not IFNα. Nevertheless, CpG-B may be restructured through aggregation within ACM polymersomes to resemble CpG-A, thereby gaining the properties of both.

In the present ex vivo experiment, peripheral blood mononuclear cells (PBMCs) from six healthy donors were stimulated with free CpG-A, free CpG-B (7909) or ACM-CpG-B at increasing concentration (0.62, 1.25, 2.5 and 5 µM). Levels of IL-6 and IFNα secreted into culture supernatant was measured by ELISA. IL-6 was detected at low to moderate quantities in all donors after incubating with CpG-A or CpG-B, with levels quickly saturating at around 1.25 µM CpG (Fig. 30a, b). Between free and ACM-CpG-B, similar dose-response profiles were observed (Fig. 30b, c). With regards to IFNα, potent production was seen with CpG-A (Fig. 30d) whereas little to none was detected with CpG-B (Fig. 30e). Interestingly, encapsulating CpG-B within ACM polymersomes conferred the ability to simulate IFNα production within the range of 0.62-1.25 µM CpG (Fig. 30f). Altogether, the data indicated that ACM-CpG-B could induce IFNα and IL-6 in human PBMCs, likely due to the aggregated nature of the CpG molecules within polymersomes. The ability to induce both cytokines was advantageous as they stimulate DCs to mature and B cells to proliferate and produce antibody, respectively.

### Example 23: Evaluating therapeutic and metaphylactic properties of CpG 2007 (SEQ ID NO: 74: 5'-tcgtcgttgtcgttttgtcgtt-3') encapsulated in polymersomes of the present invention consisting of BD21 (85 mole %) and DOTAP (15 mole %) (hereafter "ACM CpG") in treatment and prophylaxis of Feline upper respiratory infection (URI).

Feline upper respiratory infection (URI) is the common term for a respiratory infection caused by one or more viral or bacterial agents. Synonyms for this condition include feline infectious respiratory disease and feline upper respiratory disease complex (URD). The infection may be caused by one or more viral and bacterial agents capable of causing disease in cats. The most common viruses that cause upper respiratory infections in cats are feline herpesvirus type-1 (also known as feline viral rhinotracheitis or FVR) and feline calicivirus (FCV), while the most common bacteria that cause upper respiratory infections in cats are *Bordetella bronchiseptica* (*B. bronchiseptica*) and *Chlamydophila felis* (*C. felis*) (see for example at https://vcahospitals.com/know-your-pet/feline-upper-respiratory-infection).

### Safety and dosage:

This study concluded that ACM CpG when administered to kittens (weight of about 4 kg) aged 6-12 weeks, subcutaneously once daily for 5 consecutive days at a single daily dosage of 0.5 ml containing 50 µg CpG per cat (about 4 kg weight), caused no local or systemic reactions that were observable during the 5 days of administration and for another three days after last administration in twenty kittens aged 6-12 weeks.

### Basic properties of ACM CpG:

An immunostimulant properties of ACM CpGs of the present invention have been tested. CpG 2007 is a di-oligonucleotide which when it binds to TLR-9 triggers the production of several cytokines, among which interleukin-6 (IL-6), IL-12 and tumour-necrosis factor (TNF). TLR-9 activation also stimulates the dendritic cells and NK response. This allows triggering a strong non-specific immune response against various pathogens.

It has been shown that ACM-CpG's showed positive results when assessed in large animals (here cats) for respiratory disease in feedlots and, more recently, in dogs and cats by one veterinary clinic during FeURTD outbreaks in kittens and respiratory disease complex in cats. Preliminary findings showed a strong reduction of mortality and clinical signs via stimulation of an immune response.

### Aims of the study

The main aim of this study was to evaluate the curative (therapeutic) and metaphylactic (prevention of disease in probable infected subjects) properties of ACM CpG in clinical feline upper respiratory tract disease (FeURTD) in kittens under 16 weeks of age.

### Reasons for the study

FeURTD remains a common clinical entity leading to high morbidity and mortality in cat shelters of mainly unvaccinated kittens.

Intensive treatment is costly and due to financial constraints and poor response to treatment such kittens are often euthanized in shelter environments.

Morbidity during a FeURTD outbreak may approach 100% in a cat shelter during an outbreak among recent kittens taken in as well as in outbreak situations in breeding cattery settings.

### Limitations of the known treatment protocols and preventative measures in control of FeURTD:

Treatment of clinical FeURTD is costly and associated with still unacceptably high morbidity, extended treatment and high incidence of relapses. During outbreaks, no measures other than preventative inoculation protocols and hygienic measures are available to shelter care takers to prevent further morbidity of disease. Immunotherapy is not easily available and costly

### Statement of the problem to be solved by this study:

ACM-CpG's efficacy has recently been assessed in dogs and cats by one veterinary clinic during FeURTD outbreaks in kittens and respiratory disease complex in cats. Preliminary findings show a strong reduction of mortality and clinical signs.

### Hypotheses

ACM CpG is capable of ameliorating severity of clinical FeURTD in kittens already infected and manifesting clinical disease. ACM CpG is capable of reducing mortality by means of euthanazia (henceforth termed euthanasia rate). ACM CpG is capable of reducing morbidity of FeURTD in-contact kittens (metaphylaxis).

### Benefits arising from this experiment

Results from this preliminary study provide indications of whether ACM CpG has a strong potential of amelioration of disease (FeURTD) and reduction in mortality. Results from this study provide indications of whether ACM CpG has a strong potential of reduction in morbidity to FeURTD in outbreak situations.

### Objectives of this study

To determine whether ACM CpG contributes to strengthen immune response against FeURTD associated pathogens in a cat colony (such as shelters and large scale breeders) environment and that this improved immune response translates into more rapid recovery in already clinically affected FeURTD kittens. Objective clinical scoring of clinical disease and disease progression will be performed in a modified version of what was previously published by Jas et al., (2015). This will be termed the FeURTD-scoring (0-15).

To determine whether ACM CpG either reduces severity of clinical disease of in-contact kittens or prevents clinical disease altogether (metaphylaxis).

### Materials and methods

### Model system

Only normal field cases of FeURTD that were incidentally infected with street pathogens were included in the study. The experimental animals originated from both a shelter environment as well as a single large cat breeder with a FeURTD (snuffles) all originating from the Gauteng province in South Africa. The kittens from the breeder were purebred Burmese kittens whereas all other kittens were domestic shorthair kittens of mixed origin.

Kittens that appear to suffer from advanced FeURTD (have a FeURTD score of above 12) were euthanized and not be admitted to the trial. Likewise, kittens that were FeLV or FIV positive were euthanised. Kittens that are FPL positive or become FPL positive were respectively excluded or eliminated from the trial. These exclusions were considered permissible because it allows for the objective evaluation of the efficacy of ACM CpG in the absence of co-morbidities in adjunct to FeURTD.

### Experimental animals (age 4-16 weeks of age, weight about 4 kg)

### Group 1: Clinically affected FeURTD cases

Twenty-eight clinically affected kittens between the ages of 4-12 weeks were included in the study. Of these, 12/28 originated from a breeder whereas 16/28 were from welfare origin.

### Group 2: In-contact yet unaffected healthy kittens

There were 17 in contact clinically healthy kittensGroup 3: Non - exposed healthy kittens

### Identification

Kittens from the breeder were identified with a transponder whereas the kittens from the welfare were identified with name tag.

### Housing

All the experimental animals in both groups 1 and 2 were housed at the rant en dal Animal hospital in their cat isolation ward. Both groups 1 and 2 were housed in the same ward and kittens of the same owner (shelter or breeder) that were of similar age and hospitalised on the same day were all kept together in "litter context" irrespective of whether they were ill or not. No attempts were made to isolate sick or in contact cats. The capacity of the isolation ward was 20 cats.

### Experimental design

### Experimental procedures, data collection and clinical procedures and observations

### Data capture

Data capture was recorded on a hard copy paper sheet as well as excel sheets. For each kitten the age, sex, breed, weight, identification was recorded on the data capture sheet. The data capture sheet recorded all procedures, dates, results thereof and clinical observations made in a chronological fashion. This included dates of each event as they are observed or performed. Over and above clinical data collected, critical data collected is Duration of stay, Adverse effects noted, Morbidity, Euthanazia rate or mortality.

### Group 1: Clinically affected FeURTD cases

Inclusion entailed showing very typical signs of FeURTD, limited to score of ≤12 and appropriate age (4-16 weeks). All kittens within desired age range presenting with symptoms consistent with FeURTD will be clinically examined as follows and data will be recorded on data capture sheet upon inclusion. These form part of general clinical examination of any sick kitten. Duration of clinical observations is until clinical resolution of disease which is usually expected within 6-10 days after presentation or until euthanazia whichever comes first. Finaly, 28 kittens were selected.
*General clinical examination (daily) included: Appetite evaluation, Temperature, Heart rate, Appearance of mucous membranes, Weight progression, Presence of nasal discharge, Presence of ocular discharge, Presence of ocular ulcers, Presence of oral ulceration on nose, lips oral mucosa and tongue, Presence of diarrhoea, Presence of vomiting, Specific FeURTD scoring modified from (Jas et al , 2015) was used as shown in the Table below:

| Clinical signs | scores |
|---|---|
| Apathy | 1 |
| Depression | 2 |
| Euthanasia or death | 15 |
| Nasal discharge slight | 1 |
| Nasal discharge copious | 2 |
| Ocular discharge slight | 1 |
| Ocular discharge copious | 2 |

| | |
|---|---|
| Ulceration small (nose or mouth) 1-3 mm | 1 |
| Ulceration large (nose or mouth) 4 or more mm | 2 |
| coughing | 1 |
| Sneezing present | 1 |
| Paroxysmal sneeezing | 2 |
| Conjunctivitis | 1 |
| Corneal ulcer | 2 |

**Specific examinations included** FeLV and FIV testing of whole blood snap test (Bionote labs) were performed, Feacal wet preparation examination to exclude spirochaetosis, coccidiosis, verminosis, giardiasis, trichomoniasis, Clinically affected cats were tested for FCV, FHV, Chlamydophila and Bordetella by PCR in group context. This means that if 7 kittens of similar age present from one origin (either welfare or breeder) and 4/7 kittens are ill at least on of the 4 sick kittens would be tested. However, samples were collected from each sick cat by means of nasal and ocular swabs. Swabs were immediately sent by courier to molecular diagnostic services (MDS). Samples were however kept on frozen state at -20 degrees Celsius for evaluation by the Idexx reference laboratory in Germany. It was considered fair to assume that the single sample from one batch of cats would be representative of the other cats with regards to offending pathogen in clinical snuffles.

### Experimental procedures:

All kittens in group 1 were tested for FeLV and FIV at presentation. At presentation the cats were treated for external and internal parasites (Revolution^{™} for kittens). ACM CpG was administered to all kittens at the dose of 0.5 ml that contained 50 µg CpG 2007 (meaning the single dose per cat was 50 µg per 4 kg cat translating to a single dosis of about 12.5 µg/kg) injected subcutaneously once daily for 5 consecutive days commencing upon inclusion in study.

Each clinically affected kitten (group 1) was also treated using 12 mg/kg zithromycin PO for 5 days and meloxicam PO for 2 days.

Kittens that displayed weak or no appetite were forcefed twice daily and food solids and water be available at all time. No fluid therapy was administered.

### Group 2: In-contact yet unaffected healthy kittens

Seventeen kittens that were deemed healthy yet that were in direct contact with ill kittens on the same premises were selected for this trial. They were kept in "litter context" and kept in the same ward for the treatment period of 8 days. At presentation the cats were treated for external and internal parasites (Revolution^{™} for kittens). ACM CpG was administered to all kittens at the dose of 0.5 ml that contained 50 µg CpG 2007 (meaning the single dose per cat was 50 µg per 4 kg cat translating to a dosis of about 12.5 µg/kg) injected subcutaneously once daily for 5 consecutive days commencing upon inclusion in study. Kittens in group 2 that happened to fall ill were not reassigned to another group and were not treated using antibiotics or nutritional support. They were exclusively treated with ACM CpG.

### Data analysis

Data analyses and simple statistics will be performed using MS Office excel to calculate morbidities, euthanasia rates, influence of duration of stay, staging of disease severity and others.

A simple meta- analyses of historic data in the last 3 years of response to treatment, duration of hospital stay, morbidity and mortality (spontaneous death or euthanazia) of 247 FeURTD cases treated at the shelter of FORA were performed for purposes of comparison.

This historic data was available in electronic format or on hard copy and was included and used as indirect controls.

### Results

### Indirect controls mined from FORA shelter

Data mined from the records of the FORA shelter from September 2018 to August 2021 showed that 347 kittens judged to be at weaning age (around 4- 9 weeks of age) were admitted during this period and either presented ill or fell ill with typical signs of FeURTD during their stay at the shelter awaiting adoption.

### Morbidity

It was not possible to accurately ascertain the morbidity as entire in contact litters were often euthanatized as FeURTD spread in the shelter among young kittens frequently approaching 100% morbidity necessitating euthanasia of all affected and in-contact kittens. It was considered fair to assume that the morbidity frequently exceeded 80% in the vulnerable age group of 4-16 weeks old kittens. The latter figure is supported by figures cited in the literature on shelter medicine.

### Mortality

The mortality rate (spontaneous death or death by euthanasia) was 227/347 = 65.4 % in this cohort where treatment was attempted.

### Average duration of treatment (hospital stay)

Records were available for 120 kittens that were treated until recovery. It took 1411 treatment days for these 120 kittens. The mean duration of treatment for these 120 kittens was thus 1411/120 = 11.8 days mean hospital stay.

### Results of the trial in this study

### Mortality

No animal was euthanised in this trial. Two animals in group 1 died as result of FeURTD by the fifth day in treatment. This represents a mortality rate of 2/28 = 7.1 %.

### Morbidity

Three out of seventeen kittens that were in direct contact with the sick kittens and kept together with ill kittens during the treatment period fell ill during the 8 days following admission to the cat ward. This represents a 17.6 % morbidity rate.

### Mean hospital duration

For the 26/28 kittens that survived, the mean duration of hospitalisation until they recovered was 105/26 = 4.03 days.

### Offending pathogens (PCR results)

One case of calcici virus and 2 cases of FHV (Feline herpesvirus) were confirmed in this trial so far. More samples are enroute to another reference laboratory. This at least confirms that the two most common offending viral pathogens were at play in the ethiopathogenesis of the FeURTD encountered in this trial. This concurs with reviews on FeURTD in the literature.

### Discussion

In large cat colonies originating from both shelter and breeder environments, FeURTD remains a huge challenge leading to frequent outbreak of disease resulting in high morbidity and mortality. In this trial the use of ACM CpG vastly reduced mortality, morbidity and duration of hospital when compared to shelter statistics and the literature. The results in the current trial confirm that ACM CpG displayed therapeutic properties in vastly reducing expected mortality rates (7% vs. 60%) as well as vast reduction of hospital stay (4 days vs 11 days) by ameliorating severity of disease. In addition, the reduction in morbidity (17% vs. expected 80%) shows that ACM CpG proved useful for metaphylaxis of FeURTD in the absence of ancillary treatment.

### Conclusion

The result in the current study shows that ACM CpG administered for 5 consecutive days at the dose of 0.5 ml contained 50 µg CpG 2007 (meaning the single dose per cat was 50 µg per 4 kg cat translating to a dosis of about 12.5 µg/kg) vastly reduced the mortality and hospital stay for kittens affected with in clinical FeURTD and vastly reduced morbidity for kittens in contact with FeURTD. A single dose of 30 µg CpG per cat produced similar results (data not shown).

Accordingly, it was concluded that an examplary single dose (i.e. solo administration) of about 30-50 µg CpG administered in the form of ACM CpG per 4 kg cats translating to an illustrative exemplary dosage from about 7.5 to about 12.5 µg CpG in the form of ACM CpG per kg produced a significant immune response comprising therapeutic and prophylactic modality against highly infection agents (e.g., bacteria and/or viruses, e.g., Calcici virus and/or FHV (Feline herpesvirus)). Since viruses and bacteria that cause URI in cats are highly contagious, this study shows that administration of ACM CpGs according to the present invention elicits an immune response and this immune response works either therapeutically or prophylactically in of FeURTD, caused by, e.g., viruses, e.g., Calcici virus and/or FHV.

### Example 24: Dog study (carried out with polymersomes consisting of BD21 (85 mole %) and DOTAP (15 mole %) (hereafter "ACM CpG"), dosage and CpG (SEQ ID NO: 74) are exactly the same as in the cats Example 23 herein)

In this study an outbreak of Canine Parvo viral infection (acute, infectious gastrointestinal illness) in Rottweilers was investigated. It was found that there are therapeutic benefits for dogs, especially when treated early in the outbreak (i.e. early disease progression stage) with ACM CpGs of the present invention. All dogs were vaccinated with Primodog prior to receiving CpG as solo administration. The outbreak stopped quickly. However, an unteated outbreak usually is characterized with a quick spread.

Accordingly, it was concluded that an examplary single dose (i.e., solo administration as described herein) of about 12.5 µg CpG (SEQ ID NO: 74) per kg administered in the form of ACM CpG produced a significant immune response comprising at least a therapeutic modality against highly infection disease agent (e.g., Canine Parvo viral infection) in dogs, especially when treated early in the outbreak.

### References

Liu et al., Immune responses to vaccines delivered by encapsulation into and/or adsorption onto cationic lipid-PLGA hybrid nanoparticles, Journal of Controlled Release, 225 (2016): 230-239.
Hilbert et al., Biodegradable microspheres containing influenza A vaccine: immune response to mice, Vaccine 17 (1999): 1065-1073.
Miller et al., Adjuvant compositions and related methods, US9636397B2, Gerber et al., Adjuvant and vaccine compositions, US2015/0044242 A1.
Maji et al., A lipid based antigen delivery system efficiently facilitates MHC class-I Antigen Presentation in Dendritic Cells to Stimulate CD8+ T cells, Scientific Reports 6 (2016): 27206.
Moon et al., Interlayer-crosslinked multilamellar vesicles as synthetic vaccines for potent humoral and cellular responses, Nature Materials 10 (2011): 243-251.
May et al., "In Vitro Expressed GPCR Inserted in Polymersome Membranes for Ligand-Binding Studies" (2013) Angew. Chem. Int. Ed., 52, pages 749-753Kuai et al., Designer vaccine nanodiscs for personalized cancer immunotherapy, Nat Mater. (2017) 16(4):489-496.
Luo et al., A STING-activating nanovaccine for cancer immunotherapy, Nat Nanotechnol. (2017) 12(7):648-654.
Quer et al, "Polymersomes enhance the immunogenicity of influenza subunit vaccine", POLYMER CHEMISTRY, GB, (2012), vol. 2, no. 7, page 1482
Nallani et al., Method for eliciting an immune response to an immunogen, WO2014/077781A1.
Nallani et al., Proteopolymersomes: In vitro production of a membrane protein in polymersome membranes (2011), Biointerphases, 6(4), pages 153-157.
Neil et al, A Novel Method for the Encapsulation of Biomolecules into Polymersomes via Direct Hydration, Langmuir (2009), 25(16), 9025-9029.
Rameez et al. Large Scale Production of Vesicles by Hollow Fiber Extrusion: A Novel Method for Generating Polymersome Encapsulated Hemoglobin Dispersions Langmuir (2010) 26 (7), pp 5279-5285
Stano et al., Tunable T cell immunity towards a protein antigen using polymersomes vs. solid-core nanoparticles, Biomaterials 34 (2013): 4339-4346.
Fu et al., Multicompartmentalized polymersomes for selective encapsulation of biomacromolecules, Chemical Communication, 2011, 47, 2862-2864.
Lim. S.K, et al., Spontaneous formation of nanometer scale tubular vesicles in aqueous mixtures of lipid and block copolymer amphiphiles, Soft Matter 2017, 1107-1115.
Scott et al., Dendritic cell activation and T cell priming with adjuvant- and antigen-loaded oxidation-sensitive polymersomes, Biomaterials 33 (2012) 6211e6
Vasievich. E et al., Enantiospecific adjuvant activity of cationic lipid DOTAP in vancer vaccine, Cancer Immunol Immunother (2011) 60:629-638
Watson. D et al., Design considerations for liposomal vaccines: Influence of formulation parameters on antibody and cell-mediated immune responses to liposome associated antigens, Vaccine (2012), 30: 2256-2272
De Titta A, et al., Nanoparticle condjugation of CpG enhances adjuvancy for cellular immunity and memory recall at low dose, PNAS (2013), 110 (49):19902-7
Gursel. I et al., Sterically Stabilized Cationic Liposomes Improve the Uptake and Immunostimulatory Activity of CpG Oligonucleotide, Journal of Immunology 2001, 167, 3324-3328.

## Claims

1. An adjuvant associated with one or more populations of polymersomes for use in a method of eliciting an immune response in a subject by sole administration of said adjuvant, wherein said sole administration is an administration **characterized in that** no antigen is administered to said subject in combination with said adjuvant.

2. The adjuvant associated with one or more populations of polymersomes for the use according to claim 1, wherein said sole administration is one or more of the following:
(i) a prophylactic administration, preferably for anti-viral and/or immunomodulatory prophylaxis;
(ii) a therapeutic administration, preferably for anti-viral and/or immunomodulatory treatment;
(iii) an administration for reducing stress level in a subject (e.g. reducing stress level during shipping, transportation of cattle and/or mixing of cattle with other animals;
(iv) any combination of (i)-(iii).

3. The adjuvant associated with one or more populations of polymersomes for the use according to any one of claims 1-2, wherein said adjuvant is independently associated with the same or different populations of polymersomes, preferably said adjuvant is a CpG oligonucleotide of class A, B or C.

4. The adjuvant associated with one or more populations of polymersomes for the use according to any one of claims 1-3, wherein said adjuvant is: (i) a CpG oligonucleotide of class A, B or C; or (ii) a mixture comprising at least one CpG oligonucleotide of class A, B or C.

5. The adjuvant associated with one or more populations of polymersomes for the use according to claim 4, wherein said CpG oligonucleotide is selected from the group of oligonucleotides having the sequences as shown in SEQ ID NOs: 18, 62-64 and 67-77.

6. The adjuvant associated with one or more populations of polymersomes for the use according to any one of the preceding claims, wherein said adjuvant is independently associated with said one or more populations of polymersomes by one or more of the following means:
(i) encapsulating said adjuvant within said one or more populations of polymersomes;
(ii) integrating and/or embedding said adjuvant into the circumferential membranes of said one or more populations of polymersomes;
(iii) conjugating said adjuvant to the exterior surfaces of said one or more populations of polymersomes via covalent bonds;
(iv) conjugating said adjuvant to the exterior surfaces of said one or more populations of polymersomes via a non-covalent bond; and/or
(v) any combination of (i)-(iv).

7. The adjuvant associated with one or more populations of polymersomes for the use according to any one of the preceding claims, wherein said adjuvant is independently selected from the group consisting of:
(i) CpG oligodeoxynucleotides (or CpG ODN), preferably said CpG oligodeoxynucleotides selected from the group consisting of: CpG classes A, B and C, further preferably said CpG oligodeoxynucleotides selected from the group consisting of: CpG oligodeoxynucleotides having SEQ ID NOs: 18, 62-64 and 67-77;
(ii) non-antigenic components derived from bacterial and mycobacterial cell walls and proteins.

8. The adjuvant associated with one or more populations of polymersomes for the use according to any one of the preceding claims, wherein said polymersomes are oxidation-stable polymersomes.

9. The adjuvant associated with one or more populations of polymersomes for the use according to any one of the preceding claims, wherein said immune response comprises stimulating production and/or secretion of one or more cytokines, preferably said immune response is an innate immune response.

10. The adjuvant associated with one or more populations of polymersomes for the use according to any one of the preceding claims, wherein said immune response comprises stimulating production and/or secretion of one or more cytokines comprising stimulating production and/or secretion of interleukin-6 (IL-6), preferably said production and/or secretion of interleukin-6 (IL-6) is predominant over production and/or secretion of interleukin-12 (IL-12), further preferably said production and/or secretion of interleukin-6 (IL-6) is free from production and/or secretion of interleukin-12 (IL-12).

11. The adjuvant associated with one or more populations of polymersomes for the use according to any one of the preceding claims, wherein the route of said sole administration is selected from the group consisting of: oral administration, intranasal administration, administration to a mucosal surface, inhalation, topical administration, intradermal administration, intraperitoneal administration, subcutaneous administration, intravenous administration and intramuscular administration.

12. The adjuvant associated with one or more populations of polymersomes for the use according to any one of the preceding claims, wherein said subject is selected from the group consisting of:
(i) a mammalian animal, preferably said mammalian animal is a human, cat, dog, cattle, goat, sheep, cow, horse or pig; and
(ii) a non-mammalian animal.

13. The adjuvant associated with one or more populations of polymersomes for the use according to any one of the preceding claims, wherein said method is a method of treatment and/or prophylactic method against a disease selected from the group consisting of: cancer (e.g., sarcoma, fibrosarcoma), Atopic Dermatitis, African swine fever, Avian influenza, Bovine spongiform encephalopathy, Brucellosis, Cattle Fever Tick, Chronic wasting disease, Classical swine fever, Contagious equine metritis, Equine herpesvirus, Equine infectious anemia, Equine piroplasmosis, Equine viral arteritis, Foot and mouth disease, Johnes disease, Mycoplasma ovipneumoniae, Porcine Epidemic Diarrhea Virus, Pseudorabies, Rabbit Hemorrhagic Disease Virus, Schmallenberg Virus, Scrapie, Spring viremia carp, Influenza A virus in swine, Tuberculosis, Vesicular stomatitis, West Nile virus, stress-related diseases (e.g., pasteurellosis, Mannheimia haemolytica, and coccidiosis), viral disease (e.g., anti-viral treatment or prophylaxis), immune disease (e.g., immunomodulatory treatment or prophylaxis), Feline Calicivirus, Coronavirus, Herpesvirus, Canine parvovirus, Swine post-weaning Diarrhea, Upper respiratory disease complex for cattle, horses and/or kittens, Feline upper respiratory infection (URI), Feline Upper Respiratory Tract Disease (FeURTD), Feline herpesvirus (FHV), Canine parvovirus (CPV).

14. The adjuvant associated with one or more populations of polymersomes for the use according to any one of the preceding claims, wherein said polymersomes have one or more of the following properties:
i) comprising an oxidation-stable membrane;
ii) being synthetic;
iii) are free from encapsulated and associated antigens;
iv) comprising a membrane of an amphiphilic polymer;
v) comprising amphiphilic synthetic block copolymers forming a vesicle membrane;
vi) having a diameter greater than 70nm, preferably said diameter ranging from about 100nm to about 1µm, or from about 100nm to about 750nm, or from about 100nm to about 500nm, or from about 125 nm to about 250 nm, from about 140 nm to about 240 nm, from about 150 nm to about 235 nm, from about 170nm to about 230nm, or from about 220nm to about 180nm, or from about 190nm to about 210nm, most preferably said diameter is of about 200nm;
vii) having a vesicular morphology;
viii) being self-assembling; and/or
ix) being essentially non-immunogenic or essentially non-antigenic, preferably said block copolymer or amphiphilic polymer is non-immunogenic or non-antigenic.

15. The adjuvant associated with one or more populations of polymersomes for the use according to any one of the preceding claims, wherein:
(i) said one or more populations of polymersomes comprise or is formed from an amphiphilic polymer comprising or consisting of a diblock or a triblock (A-B-A or A-B-C) copolymer; and/or
(ii) said one or more populations of polymersomes comprise a lipid polymer:

16. The adjuvant associated with one or more populations of polymersomes for the use according to any one of the preceding claims, wherein:
(a) said amphiphilic polymer comprises a copolymer poly(N-vinylpyrrolidone)-b-PLA;
(b) said amphiphilic polymer is a poly(butadiene)-poly(ethylene oxide) (PB-PEO) diblock copolymer, or wherein said amphiphilic polymer is a poly (dimethylsiloxane)-poly(ethylene oxide) (PDMS-PEO) diblock copolymer, or poly (dimethyl siloxane)-poly(acrylic acid) (PDMS-PAA), wherein said PB-PEO diblock copolymer preferably comprises 5-50 blocks PB and 5-50 blocks PEO or wherein said PB-PDMS diblock copolymer preferably comprises 5-100 blocks PDMS and 5-100 blocks PEO;
(c) said amphiphilic polymer is a poly(lactide)-poly(ethylene oxide)/1-palmitoyl-2-oleoyl-sn-glycero-3-phospho-L-serine (PLA-PEO/POPC) copolymer, preferably said PLA-PEO/POPC has a ratio of 75 to 25 (e.g., 75/25) of PLA-PEO to POPC (e.g., PLA-PEO/POPC);
(d) said amphiphilic polymer is a poly(caprolactone)-poly(ethylene oxide)/1-palmitoyl-2-oleoyl-sn-glycero-3-phospho-L-serine (PCL-PEO/POPC) copolymer, preferably said PCL-PEO/POPC has a ratio of 75 to 25 (e.g., 75/25) of PCL-PEO to POPC (e.g., PCL-PEO/POPC);
(e) said amphiphilic polymer is polybutadiene-polyethylene oxide (BD); and/or
(f) said first and/or second population of polymersomes comprises diblock copolymer PBD₂₁-PEO₁₄ (BD21) and/or the triblock copolymer PMOXA₁₂-PDMS₅₅-PMOXA₁₂; and/or
(g) said first and/or second population of polymersomes comprises diblock copolymer PBD₂₁-PEO₁₄ (BD21) and DOTAP (e.g., BD21 at 85 mole % concentration and DOTAP at 15 mole % concentration).

17. The adjuvant associated with one or more populations of polymersomes for the use according to any one of the preceding claims, wherein said sole administration comprises one or more administrations of said first and/or second population of polymersomes encapsulating said adjuvant, wherein said adjuvant is a CpG oligonucleotide, wherein a single dosage of said CpG oligonucleotide is from about 7.5 µg to about 12.5 µg CpG per kg of weight of said subject.

18. An adjuvant for use in eliciting an immune response in a subject, the use comprising sole administration of said adjuvant, wherein said adjuvant is associated with one or more populations of polymersomes.

## Patentansprüche

1. Ein Adjuvans, das mit einer oder mehreren Populationen von Polymersomen assoziiert ist, zur Verwendung in einem Verfahren zur Auslösung einer Immunantwort in einem Subjekt durch alleinige Verabreichung des Adjuvans, wobei die alleinige Verabreichung **dadurch gekennzeichnet ist, dass** dem Subjekt kein Antigen in Kombination mit dem Adjuvans verabreicht wird.

2. Das Adjuvans, das mit einer oder mehreren Populationen von Polymersomen assoziiert ist, zur Verwendung gemäß Anspruch 1, wobei die alleinige Verabreichung eine oder mehrere der folgenden ist:
(i) eine prophylaktische Verabreichung, vorzugsweise zur antiviralen und/oder immunmodulatorischen Prophylaxe;
(ii) eine therapeutische Verabreichung, vorzugsweise zur antiviralen und/oder immunmodulatorischen Behandlung;
(iii) eine Verabreichung zur Verringerung des Stressniveaus bei einem Subjekt (z. B. zur Verringerung des Stressniveaus während des Versands, des Transports von Rindern und/oder der Vermischung von Rindern mit anderen Tieren);
(iv) jede Kombination aus (i)-(iii).

3. Das Adjuvans, das mit einer oder mehreren Populationen von Polymersomen assoziiert ist, zur Verwendung gemäß einem der Ansprüche 1 bis 2, wobei das Adjuvans unabhängig mit derselben oder verschiedenen Populationen von Polymersomen assoziiert ist, vorzugsweise ist das Adjuvans ein CpG-Oligonukleotid der Klasse A, B oder C.

4. Das Adjuvans, das mit einer oder mehreren Populationen von Polymersomen assoziiert ist, zur Verwendung gemäß einem der Ansprüche 1 bis 3, wobei das Adjuvans: (i) ein CpG-Oligonukleotid der Klasse A, B oder C ist; oder (ii) ein Gemisch ist, das mindestens ein CpG-Oligonukleotid der Klasse A, B oder C umfasst.

5. Das Adjuvans, das mit einer oder mehreren Populationen von Polymersomen assoziiert ist, zur Verwendung gemäß Anspruch 4, wobei das CpG-Oligonukleotid aus der Gruppe der Oligonukleotide mit den in SEQ ID Nr.: 18, 62-64 und 67-77 angegebenen Sequenzen ausgewählt ist.

6. Das Adjuvans, das mit einer oder mehreren Populationen von Polymersomen assoziiert ist, zur Verwendung gemäß einem der vorhergehenden Ansprüche, wobei das Adjuvans unabhängig durch eines oder mehrere der folgenden Mittel mit der einen oder den mehreren Populationen von Polymersomen assoziiert ist:
(i) Einkapselung des Adjuvans in die eine oder die mehreren Populationen von Polymersomen;
(ii) Integration und/oder Einbettung des Adjuvans in die Umfangsmembranen der einen oder der mehreren Populationen von Polymersomen;
(iii) Konjugation des Adjuvans an die Außenflächen der einen oder der mehreren Populationen von Polymersomen über kovalente Bindungen;
(iv) Konjugation des Adjuvans an die Außenflächen der einen oder der mehreren Populationen von Polymersomen über eine nicht-kovalente Bindung; und/oder
(v) jede Kombination aus (i)-(iv).

7. Das Adjuvans, das mit einer oder mehreren Populationen von Polymersomen assoziiert ist, zur Verwendung gemäß einem der vorhergehenden Ansprüche, wobei das Adjuvans unabhängig aus der Gruppe ausgewählt ist, die besteht aus:
(i) CpG-Oligodesoxynukleotiden (oder CpG-ODN), vorzugsweise ausgewählt aus der Gruppe bestehend aus: CpG-Klassen A, B und C, weiter vorzugsweise ausgewählt aus der Gruppe bestehend aus: CpG-Oligodesoxynukleotiden mit den SEQ ID Nr.: 18, 62-64 und 67-77;
(ii) nicht-antigenen Bestandteilen, die aus bakteriellen und mykobakteriellen Zellwänden und Proteinen abgeleitet sind.

8. Das Adjuvans, das mit einer oder mehreren Populationen von Polymersomen assoziiert ist, zur Verwendung gemäß einem der vorhergehenden Ansprüche, wobei die Polymersome oxidationsstabile Polymersome sind.

9. Das Adjuvans, das mit einer oder mehreren Populationen von Polymersomen assoziiert ist, zur Verwendung gemäß einem der vorhergehenden Ansprüche, wobei die Immunantwort die Stimulation der Produktion und/oder Sekretion von einem oder mehreren Zytokinen umfasst, vorzugsweise eine angeborene Immunantwort ist.

10. Das Adjuvans, das mit einer oder mehreren Populationen von Polymersomen assoziiert ist, zur Verwendung gemäß einem der vorhergehenden Ansprüche, wobei die Immunantwort die Stimulation der Produktion und/oder Sekretion von einem oder mehreren Zytokinen umfasst, wobei die Stimulation der Produktion und/oder Sekretion von Interleukin-6 (IL-6) umfasst ist, vorzugsweise die Produktion und/oder Sekretion von Interleukin-6 (IL-6) gegenüber der Produktion und/oder Sekretion von Interleukin-12 (IL-12) überwiegt, weiter vorzugsweise die Produktion und/oder Sekretion von Interleukin-6 (IL-6) frei von der Produktion und/oder Sekretion von Interleukin-12 (IL-12) ist.

11. Das Adjuvans, das mit einer oder mehreren Populationen von Polymersomen assoziiert ist, zur Verwendung gemäß einem der vorhergehenden Ansprüche, wobei der Verabreichungsweg der alleinigen Verabreichung aus der Gruppe ausgewählt ist, die besteht aus: oraler Verabreichung, intranasaler Verabreichung, Verabreichung an eine Schleimhautoberfläche, Inhalation, topischer Verabreichung, intradermaler Verabreichung, intraperitonealer Verabreichung, subkutaner Verabreichung, intravenöser Verabreichung und intramuskulärer Verabreichung.

12. Das Adjuvans, das mit einer oder mehreren Populationen von Polymersomen assoziiert ist, zur Verwendung gemäß einem der vorhergehenden Ansprüche, wobei das Subjekt aus der Gruppe ausgewählt ist, die besteht aus:
(i) einem Säugetier, vorzugsweise ist das Säugetier ein Mensch, eine Katze, ein Hund, ein Rind, eine Ziege, ein Schaf, eine Kuh, ein Pferd oder ein Schwein; und
(ii) einem Nicht-Säugetier.

13. Das Adjuvans, das mit einer oder mehreren Populationen von Polymersomen assoziiert ist, zur Verwendung gemäß einem der vorhergehenden Ansprüche, wobei das Verfahren ein Behandlungsverfahren und/oder ein prophylaktisches Verfahren gegen eine Krankheit ist, die aus der Gruppe ausgewählt ist, die besteht aus: Krebs (z. B. Sarkom, Fibrosarkom), atopische Dermatitis, Afrikanische Schweinepest, Aviäre Influenza, Bovine spongiforme Enzephalopathie, Brucellose, Rinderfieberzecke, Chronische Auszehrungskrankheit, Klassische Schweinepest, Ansteckende Gebärmutterentzündung beim Pferd, Equines Herpesvirus, Equine Infektiöse Anämie, Equine Piroplasmose, Equine Virusarteriitis, Maul- und Klauenseuche, Johne'sche Krankheit, Mycoplasma ovipneumoniae, Porcines Epidemisches Durchfallvirus, Pseudowut, Hämorrhagische Kaninchenseuche, Schmallenberg-Virus, Scrapie, Frühjahrvirämie der Karpfen, Influenzavirus A bei Schweinen, Tuberkulose, Vesikuläre Stomatitis, West-Nil-Virus, stressbedingte Erkrankungen (z. B. Pasteurellose, Mannheimia haemolytica und Kokzidiose), Viruserkrankungen (z. B. antivirale Behandlung oder Prophylaxe), Immunerkrankungen (z. B. immunmodulatorische Behandlung oder Prophylaxe), Feliner Calicivirus, Coronavirus, Herpesvirus, Canines Parvovirus, Nachabsetz-Durchfall beim Schwein, Atemwegserkrankungskomplex bei Rindern, Pferden und/oder Katzenwelpen, Obere Atemwegsinfektion bei Katzen (URI), Feline Erkrankung der oberen Atemwege (FeURTD), Felines Herpesvirus (FHV), Canines Parvovirus (CPV).

14. Das Adjuvans, das mit einer oder mehreren Populationen von Polymersomen assoziiert ist, zur Verwendung gemäß einem der vorhergehenden Ansprüche, wobei die Polymersome eine oder mehrere der folgenden Eigenschaften aufweisen:
(i) umfassen eine oxidationsstabile Membran;
(ii) sind synthetisch;
(iii) sind frei von eingekapselten und assoziierten Antigenen;
(iv) umfassen eine Membran aus einem amphiphilen Polymer;
(v) umfassen amphiphile synthetische Blockcopolymere, die eine Vesikelmembran bilden;
(vi) einen Durchmesser von mehr als 70 nm aufweisen, wobei der Durchmesser vorzugsweise im Bereich von etwa 100 nm bis etwa 1 µm oder von etwa 100 nm bis etwa 750 nm oder von etwa 100 nm bis etwa 500 nm oder von etwa 125 nm bis etwa 250 nm oder von etwa 140 nm bis etwa 240 nm liegt, etwa 150 nm bis etwa 235 nm, etwa 170 nm bis etwa 230 nm oder etwa 220 nm bis etwa 180 nm oder etwa 190 nm bis etwa 210 nm, wobei der Durchmesser am bevorzugtesten etwa 200 nm beträgt;
(vii) eine vesikuläre Morphologie aufweist;
(viii) selbstorganisierend ist; und/oder
(ix) im Wesentlichen nicht-immunogen oder im Wesentlichen nicht-antigen ist, wobei das Blockcopolymer oder amphiphile Polymer vorzugsweise nicht-immunogen oder nicht-antigen ist.

15. Das Adjuvans, das mit einer oder mehreren Populationen von Polymersomen assoziiert ist, zur Verwendung gemäß einem der vorhergehenden Ansprüche, wobei:
(i) die eine oder mehreren Populationen von Polymersomen ein amphiphiles Polymer umfassen oder aus einem amphiphilen Polymer gebildet sind, das ein Diblock- oder Triblock-Copolymer (A-B-A oder A-B-C) umfasst oder daraus besteht; und/oder
(ii) die eine oder mehreren Populationen von Polymersomen ein Lipidpolymer umfassen.

16. Das Adjuvans, das mit einer oder mehreren Populationen von Polymersomen assoziiert ist, zur Verwendung gemäß einem der vorhergehenden Ansprüche, wobei:
(a) das amphiphile Polymer ein Copolymer aus Poly(N-vinylpyrrolidon)-b-PLA umfasst;
(b) das amphiphile Polymer ein Poly(butadien)-poly(ethylenoxid) (PB-PEO)-Diblock-Copolymer ist, oder wobei das amphiphile Polymer ein Poly(dimethylsiloxan)-poly(ethylenoxid) (PDMS-PEO)-Diblock-Copolymer ist, oder Poly(dimethylsiloxan)-poly(acrylsäure) (PDMS-PAA), wobei das PB-PEO-Diblock-Copolymer vorzugsweise 5-50 Blöcke PB und 5-50 Blöcke PEO umfasst oder wobei das PB-PDMS-Diblock-Copolymer vorzugsweise 5-100 Blöcke PDMS und 5-100 Blöcke PEO umfasst;
(c) das amphiphile Polymer ein Poly(lactid)-poly(ethylenoxid)/1-palmitoyl-2-oleoyl-sn-glycero-3-phospho-L-serin (PLA-PEO/POPC)-Copolymer ist, wobei das PLA-PEO/POPC vorzugsweise ein Verhältnis von 75 zu 25 (z. B. 75/25) von PLA-PEO zu POPC (z. B. PLA-PEO/POPC) aufweist;
(d) das amphiphile Polymer ein Poly(caprolacton)-poly(ethylenoxid)/1-palmitoyl-2-oleoyl-sn-glycero-3-phospho-L-serin (PCL-PEO/POPC)-Copolymer ist, wobei das PCL-PEO/POPC vorzugsweise ein Verhältnis von 75 zu 25 (z. B. 75/25) von PCL-PEO zu POPC (z. B. PCL-PEO/POPC) aufweist;
(e) das amphiphile Polymer Polybutadien-Polyethylenoxid (BD) ist; und/oder
(f) die erste und/oder zweite Population von Polymersomen umfasst das Diblock-Copolymer PBD₂₁-PEO₁₄ (BD21) und/oder das Triblock-Copolymer PMOXA₁₂-PDMS₅₅-PMOXA₁₂; und/oder
(g) die erste und/oder zweite Population von Polymersomen das Diblock-Copolymer PBD₂₁-PEO₁₄ (BD21) und DOTAP (z. B. BD21 in einer Konzentration von 85 Mol-% und DOTAP in einer Konzentration von 15 Mol-%) umfasst.

17. Das Adjuvans, das mit einer oder mehreren Populationen von Polymersomen assoziiert ist, zur Verwendung gemäß einem der vorhergehenden Ansprüche, wobei die alleinige Verabreichung eine oder mehrere Verabreichungen der ersten und/oder zweiten Population von Polymersomen umfasst, die das Adjuvans einkapseln, wobei das Adjuvans ein CpG-Oligonukleotid ist, wobei eine Einzeldosis des CpG-Oligonukleotids etwa 7,5 µg bis etwa 12,5 µg CpG pro kg Körpergewicht des Probanden beträgt.

18. Ein Adjuvans zur Verwendung zur Auslösung einer Immunantwort bei einem Subjekt, wobei die Verwendung die alleinige Verabreichung des Adjuvans umfasst, wobei das Adjuvans mit einer oder mehreren Populationen von Polymersomen assoziiert ist.

## Revendications

1. Un adjuvant associé à une ou plusieurs populations de polymérosomes destiné à être utilisé dans un procédé visant à provoquer une réponse immunitaire chez un sujet par administration exclusive dudit adjuvant, dans lequel ladite administration exclusive est une administration **caractérisée en ce qu'**aucun antigène n'est administré audit sujet en association avec ledit adjuvant.

2. L'adjuvant associé à une ou plusieurs populations de polymérosomes pour l'utilisation selon la revendication 1, ladite administration exclusive étant une ou plusieurs des suivantes:
(i) une administration prophylactique, de préférence pour une prophylaxie antivirale et/ou immunomodulatrice;
(ii) une administration thérapeutique, de préférence pour un traitement antiviral et/ou immunomodulateur;
(iii) une administration visant à réduire le niveau de stress chez un sujet (par exemple, pour réduire le stress pendant l'expédition, le transport de bétail et/ou le mélange de bétail avec d'autres animaux);
(iv) toute combinaison de (i) à (iii).

3. L'adjuvant associé à une ou plusieurs populations de polymérosomes pour l'utilisation selon l'une quelconque des revendications 1 à 2, ledit adjuvant étant associé indépendamment aux mêmes ou à différentes populations de polymérosomes, de préférence ledit adjuvant étant un oligonucléotide CpG de classe A, B ou C.

4. L'adjuvant associé à une ou plusieurs populations de polymérosomes pour l'utilisation selon l'une quelconque des revendications 1 à 3, ledit adjuvant étant: (i) un oligonucléotide CpG de classe A, B ou C; ou (ii) un mélange comprenant au moins un oligonucléotide CpG de classe A, B ou C.

5. L'adjuvant associé à une ou plusieurs populations de polymérosomes pour l'utilisation selon la revendication 4, ledit oligonucléotide CpG étant choisi dans le groupe constitué par les oligonucléotides ayant les séquences indiquées dans les SEQ ID NO: 18, 62 à 64 et 67 à 77.

6. L'adjuvant associé à une ou plusieurs populations de polymérosomes pour l'utilisation selon l'une quelconque des revendications précédentes, ledit adjuvant étant associé indépendamment à ladite ou auxdites populations de polymérosomes par un ou plusieurs des moyens suivants:
(i) encapsulation dudit adjuvant dans ladite ou lesdites populations de polymérosomes;
(ii) intégration et/ou insertion dudit adjuvant dans les membranes périphériques de ladite ou desdites populations de polymérosomes;
(iii) conjugaison dudit adjuvant aux surfaces extérieures de ladite ou desdites populations de polymérosomes via des liaisons covalentes;
(iv) conjugaison dudit adjuvant aux surfaces extérieures de ladite ou desdites populations de polymérosomes via une liaison non covalente; et/ou
(v) toute combinaison des points (i) à (iv).

7. L'adjuvant associé à une ou plusieurs populations de polymérosomes pour l'utilisation selon l'une quelconque des revendications précédentes, ledit adjuvant étant sélectionné indépendamment dans le groupe constitué par:
(i) des oligodésoxynucléotides CpG (ou CpG ODN), de préférence des oligodésoxynucléotides CpG sélectionnés dans le groupe constitué par les classes CpG A, B et C, plus préférentiellement encore, des oligodésoxynucléotides CpG sélectionnés dans le groupe constitué par les SEQ ID NO: 18, 62 à 64 et 67 à 77;
(ii) des composants non antigéniques dérivés de parois cellulaires et de protéines bactériennes et mycobactériennes.

8. L'adjuvant associé à une ou plusieurs populations de polymérosomes pour l'utilisation selon l'une quelconque des revendications précédentes, lesdits polymérosomes étant des polymérosomes stables à l'oxydation.

9. L'adjuvant associé à une ou plusieurs populations de polymérosomes pour l'utilisation selon l'une quelconque des revendications précédentes, ladite réponse immunitaire comprenant la stimulation de la production et/ou de la sécrétion d'une ou plusieurs cytokines, de préférence ladite réponse immunitaire étant une réponse immunitaire innée.

10. L'adjuvant associé à une ou plusieurs populations de polymérosomes pour l'utilisation selon l'une quelconque des revendications précédentes, ladite réponse immunitaire comprenant la stimulation de la production et/ou de la sécrétion d'une ou plusieurs cytokines comprenant la stimulation de la production et/ou de la sécrétion d'interleukine-6 (IL-6), de préférence ladite production et/ou sécrétion d'interleukine-6 (IL-6) étant prédominante par rapport à la production et/ou à la sécrétion d'interleukine-12 (IL-12), plus préférentiellement encore ladite production et/ou sécrétion d'interleukine-6 (IL-6) étant exempte de production et/ou de sécrétion d'interleukine-12 (IL-12).

11. L'adjuvant associé à une ou plusieurs populations de polymérosomes pour l'utilisation selon l'une quelconque des revendications précédentes, la voie de ladite administration exclusive étant sélectionnée dans le groupe constitué par: administration orale, administration intranasale, administration sur une surface muqueuse, inhalation, administration topique, administration intradermique, administration intrapéritonéale, administration sous-cutanée, administration intraveineuse et administration intramusculaire.

12. L'adjuvant associé à une ou plusieurs populations de polymérosomes pour l'utilisation selon l'une quelconque des revendications précédentes, ledit sujet étant sélectionné dans le groupe constitué par:
(i) un animal mammifère, de préférence ledit animal mammifère étant un humain, un chat, un chien, un boeuf, une chèvre, un mouton, une vache, un cheval ou un porc; et
(ii) un animal non mammifère.

13. L'adjuvant associé à une ou plusieurs populations de polymérosomes pour l'utilisation selon l'une quelconque des revendications précédentes, ledit procédé étant un procédé de traitement et/ou un procédé prophylactique contre une maladie sélectionnée dans le groupe constitué par: cancer (par exemple, sarcome, fibrosarcome), dermatite atopique, peste porcine africaine, grippe aviaire, encéphalopathie spongiforme bovine, brucellose, tique de la fièvre bovine, maladie débilitante chronique, peste porcine classique, métrite contagieuse équine, herpèsvirus équin, anémie infectieuse équine, piroplasmose équine, artérite virale équine, fièvre aphteuse, maladie de Johne, Mycoplasma ovipneumoniae, virus de la diarrhée épidémique porcine, pseudorage, maladie hémorragique virale du lapin, virus de Schmallenberg, tremblante (scrapie), virémie printanière de la carpe, virus de la grippe A chez le porc, tuberculose, stomatite vésiculeuse, virus du Nil occidental, maladies liées au stress (par exemple, pasteurellose, Mannheimia haemolytica et coccidiose), maladies virales (par exemple, traitement ou prophylaxie antivirale), maladies immunitaires (par exemple, traitement ou prophylaxie immunomodulatrice), calicivirus félin, coronavirus, herpèsvirus, parvovirus canin, diarrhée post-sevrage porcine, complexe des maladies respiratoires supérieures chez les bovins, chevaux et/ou chatons, infection des voies respiratoires supérieures chez les félins (URI), maladie des voies respiratoires supérieures féline (FeURTD), herpèsvirus félin (FHV), parvovirus canin (CPV).

14. L'adjuvant associé à une ou plusieurs populations de polymérosomes pour l'utilisation selon l'une quelconque des revendications précédentes, lesdits polymérosomes présentant une ou plusieurs des propriétés suivantes:
(i) comprenant une membrane stable à l'oxydation;
(ii) étant synthétiques;
(iii) étant exempts d'antigènes encapsulés et associés;
(iv) comprenant une membrane d'un polymère amphiphile;
(v) comprenant des copolymères synthétiques amphiphiles formant une membrane vésiculaire;
(vi) ayant un diamètre supérieur à 70 nm, de préférence ledit diamètre étant compris entre environ 100 nm et environ 1 µm, ou entre environ 100 nm et environ 750 nm, ou entre environ 100 nm et environ 500 nm, ou entre environ 125 nm et environ 250 nm, entre environ 140 nm et environ 240 nm, d'environ 150 nm à environ 235 nm, d'environ 170 nm à environ 230 nm, ou d'environ 220 nm à environ 180 nm, ou d'environ 190 nm à environ 210 nm, ledit diamètre étant de préférence d'environ 200 nm;
(vii) ayant une morphologie vésiculaire;
(viii) étant auto-assemblable; et/ou
(ix) étant essentiellement non immunogène ou essentiellement non antigénique, de préférence ledit copolymère séquencé ou polymère amphiphile étant non immunogène ou non antigénique.

15. L'adjuvant associé à une ou plusieurs populations de polymérosomes destiné à être utilisé selon l'une quelconque des revendications précédentes, dans lequel:
(i) ladite ou lesdites populations de polymérosomes comprennent ou sont formées à partir d'un polymère amphiphile comprenant ou consistant en un copolymère dibloc ou tribloc (A-B-A ou A-B-C); et/ou
(ii) ladite ou lesdites populations de polymérosomes comprennent un polymère lipidique.

16. L'adjuvant associé à une ou plusieurs populations de polymérosomes pour l'utilisation selon l'une quelconque des revendications précédentes, dans lequel:
(a) ledit polymère amphiphile comprend un copolymère poly(N-vinylpyrrolidone)-b-PLA;
(b) ledit polymère amphiphile est un copolymère dibloc poly(butadiène)-poly(oxyde d'éthylène) (PB-PEO), ou dans lequel ledit polymère amphiphile est un copolymère dibloc poly(diméthylsiloxane)-poly(oxyde d'éthylène) (PDMS-PEO), ou poly(diméthylsiloxane)-poly(acide acrylique) (PDMS-PAA), dans lequel ledit copolymère dibloc PB-PEO comprend de préférence 5 à 50 blocs PB et 5 à 50 blocs PEO ou dans lequel ledit copolymère dibloc PB-PDMS comprend de préférence 5 à 100 blocs PDMS et 5 à 100 blocs PEO;
(c) ledit polymère amphiphile est un copolymère poly(lactide)-poly(oxyde d'éthylène)/1-palmitoyl-2-oléoyl-sn-glycéro-3-phospho-L-sérine (PLA-PEO/POPC), de préférence ledit PLA-PEO/POPC a un rapport de 75 à 25 (par exemple, 75/25) de PLA-PEO par rapport au POPC (par exemple, PLA-PEO/POPC);
(d) ledit polymère amphiphile est un copolymère poly(caprolactone)-poly(oxyde d'éthylène)/1-palmitoyl-2-oléoyl-sn-glycéro-3-phospho-L-sérine (PCL-PEO/POPC), de préférence ledit PCL-PEO/POPC ayant un rapport de 75 à 25 (par exemple, 75/25) de PCL-PEO par rapport à POPC (par exemple, PCL-PEO/POPC) ;
(e) ledit polymère amphiphile est du polybutadiène-polyéthylène oxyde (BD); et/ou
(f) ladite première et/ou deuxième population de polymérosomes comprend le copolymère dibloc PBD₂₁-PEO₁₄ (BD21) et/ou le copolymère tribloc PMOXA₁₂-PDMS₅₅-PMOXA₁₂; et/ou
(g) ladite première et/ou deuxième population de polymérosomes comprend le copolymère dibloc PBD₂₁-PEO₁₄ (BD21) et le DOTAP (par exemple, BD21 à une concentration de 85 % en moles et DOTAP à une concentration de 15 % en moles).

17. L'adjuvant associé à une ou plusieurs populations de polymérosomes pour l'utilisation selon l'une quelconque des revendications précédentes, dans lequel ladite administration unique comprend une ou plusieurs administrations de ladite première et/ou deuxième population de polymérosomes encapsulant ledit adjuvant, dans lequel ledit adjuvant est un oligonucléotide CpG, dans lequel une dose unique dudit oligonucléotide CpG est comprise entre environ 7,5 µg à environ 12,5 µg de CpG par kg de poids dudit sujet.

18. Un adjuvant destiné à être utilisé pour induire une réponse immunitaire chez un sujet, ladite utilisation comprenant l'administration exclusive dudit adjuvant, ledit adjuvant étant associé à une ou plusieurs populations de polymérosomes.
